(19) Europäisches Patentamt
European Patent Office
Office européen des brevets

(11) Veröffentlichungsnummer: **0 163 319**
**B1**

(12) # EUROPÄISCHE PATENTSCHRIFT

(45) Veröffentlichungstag der Patentschrift: **02.08.89**

(21) Anmeldenummer: **85106737.1**

(22) Anmeldetag: **31.05.85**

(51) Int. Cl.⁴: **C 07 D 207/08,** A 61 K 31/40, C 07 D 487/10 // (C07D487/10, 209:00, 209:00)

(54) **Neue 3-Sulfinatomethyl- und 3-Sulfonatomethyl-4-sulfomethyl-pyrrolidiniumbetaine und deren Salze sowie Verfahren zu deren Herstellung.**

(30) Priorität: **01.06.84 DD 263725**
**01.06.84 DD 263726**
**01.06.84 DD 263727**
**01.06.84 DD 263720**
**01.06.84 DD 263721**
**01.06.84 DD 263722**
**26.04.85 DD 275642**

(43) Veröffentlichungstag der Anmeldung: **04.12.85 Patentblatt 85/49**

(45) Bekanntmachung des Hinweises auf die Patenterteilung: **02.08.89 Patentblatt 89/31**

(84) Benannte Vertragsstaaten: **AT BE CH DE FR GB IT LI NL SE**

(56) Entgegenhaltungen:
**EP-A-0 095 241**
**GB-A-2 080 293**

(73) Patentinhaber: **Akademie der Wissenschaften der DDR, Otto- Nuschke- Strasse 22/23, DDR- 1086 Berlin (DD)**

(72) Erfinder: **Ballschuh, Detlef, Dr. Dipl.- Chem., Graudenzer Strasse 17, DDR- 1034 Berlin (DD)**
Erfinder: **Seibt, Horst, Dr. Dipl.- Chem., Eugen-Schönhaar Strasse 15, DDR- 1055 Berlin (DD)**
Erfinder: **Ohme, Roland, Dr. Dipl.- Chem., Waldstrasse 6, DDR- 1180 Berlin (DD)**
Erfinder: **Rusche, Jochen, Dr. Dipl.- Chem., Hans-Loch- Strasse 263, DDR- 1136 Berlin (DD)**
Erfinder: **Gründemann, Egon, Dr. Dipl.- Chem., Waldstrasse 4, DDR- 1180 Berlin (DD)**
Erfinder: **Krause, Elke, Dipl.- Chem., Bruno-Leuschner- Strasse 47, DDR- 1142 Berlin (DD)**

(74) Vertreter: **Patentanwälte Beetz sen. - Beetz jun. Timpe - Siegfried - Schmitt- Fumian, Steinsdorfstrasse 10, D-8000 München 22 (DE)**

EP 0 163 319 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue 3-substituierte 4-Sulfonatomethyl-pyrrolidiniumbetaine und ihre Salze, Verfahren zu deren Herstellung sowie ihre Verwendung, insbesondere in Tensidzusammensetzungen.

Es sind Sulfobetaine bekannt, bei denen das Kation nicht Bestandteil eines Ringsystems ist. Diese Sulfobetaine werden bevorzugt durch Alkylierung von tertiären Aminen mit Derivaten von Hydroxyalkansulfonsäuren hergestellt (Parris, Weil, Linfield, J. Amer. Oil Chemists Soc. 53 (1976) 97; DD-A-139 719). In erster Linie dient jedoch Propansulton zur Einführung des Sulfopropylrestes (DE-A2-2 431 031, 2 409 412). So gelangten beispielsweise Smith und Linfield (J. Amer. Oil Chemists Soc. 55 (1978) 741) durch Einwirkung von 2 mol Propansulton auf Hydroxyethylaminoverbindungen zu Sulfobetainen mit einer zusätzlichen Sulfogruppe, die sich durch ein gutes Kalkseifendispergiervermögen auszeichnen. Der gravierende Nachteil dieses Verfahrens besteht neben der Vielstufigkeit vor allem darin, daß Propansulton zu den gefährlichsten Cancerogenen gehört (Z. Krebsforschung, 75 (1970) 69; Registry of Toxic Effects of Chemical Substances, National Institute for Occupational Safety and Health, Maryland, USA, 1975 edition, 826) und demzufolge für technische Synthesen nur unter besonders aufwendigen Gesundheitsschutzmaßnahmen eingesetzt werden kann.

Von Linfield und Mitarbeitern wird ferner vorgeschlagen, an Trialkylallylammoniumsalze Hydrogensulfit anzulagern (J. Amer. Oil Chemists Soc. 53 (1976) 60; 55 (1978) 87). Der Mangel dieses Verfahrens besteht einerseits im Arbeiten unter Luftausschluß sowie unter Druck, und andererseits entstehen bei langen Reaktionszeiten sehr häufig uneinheitliche Reaktionsprodukte.

Die Anlagerung von gepufferter Hydrogensulfitlösung an Diallylammoniumsalze, wie Diethanoldiallylammoniumchlorid, ist in DE-B-1 173 906 beschrieben. Danach werden ausschließlich offenkettige Doppeladditionsprodukte - Sulfobetainsulfonate - erhalten.

DE-A1-2 331 515 beschreibt ein Verfahren zur radikalischen Addition von Hydrogensulfit an unsubstituierte Olefine, wobei Übergangsmetalle der 1., 7. und 8. Nebengruppe des Periodensystems als Katalysatoren verwendet werden. Die für dieses Verfahren vorgeschlagenen Olefine sind unsubstituiert und besitzen isolierte Doppelbindungen.

Die Herstellung von Sulfobetainen unter Vermeidung cancerogener Alkylantien gelingt nach DD-A-154 444 und DD-A-200 739, indem Di- oder Triallylammoniumverbindungen durch eine homogen katalytisch verlaufende radikalische Hydrogensulfitaddition umgesetzt werden. So erhält man durch Umsetzung von Diallylammoniumsalzen mit Hydrogensulfit in stöchiometrischen Mengen 3-Sulfonatomethyl-pyrrolidiniumbetaine im pH-Bereich von 2 bis 9, bevorzugt im pH-Bereich von 4 bis 8, wobei als Puffersubstanz für die Aufrechterhaltung des pH-Wertes bis zu 0,6 mol neutrales Sulfit zugegeben werden kann. (vgl. auch GB-A-2 080 293). Nach diesen Verfahren sind Sulfobetaine mit maximal nur einer zusätzlichen Sulfopropylgruppe im Molekül zugänglich.

Der vorliegenden Erfindung liegt die Aufgabe zugrunde, durch Einführung eines oder mehrerer zusätzlicher hydrophiler oder hydrophiler und reaktiver Säurereste in ein Sulfomethylpyrrolidiniumbetain ohne zwangsweise Einführung kohlenstoffhaltiger Substituenten einen neuen Typ von Sulfobetainsulfinaten/-sulfonaten zu schaffen, wobei diese Verbindungen in einfacher Weise auf Basis wohlfeiler technischer Ausgangsprodukte herstellbar sein sollen, und ihre Verwendung in Tensidzusammensetzungen anzugeben.

Diese Aufgabe wird anspruchsgemäß gelöst.

Die neuen 3-Sulfinatomethyl- oder 3-Sulfonatomethyl-4-sulfonatomethyl-pyrrolidiniumbetaine bezitzen die Formeln I a und I b,

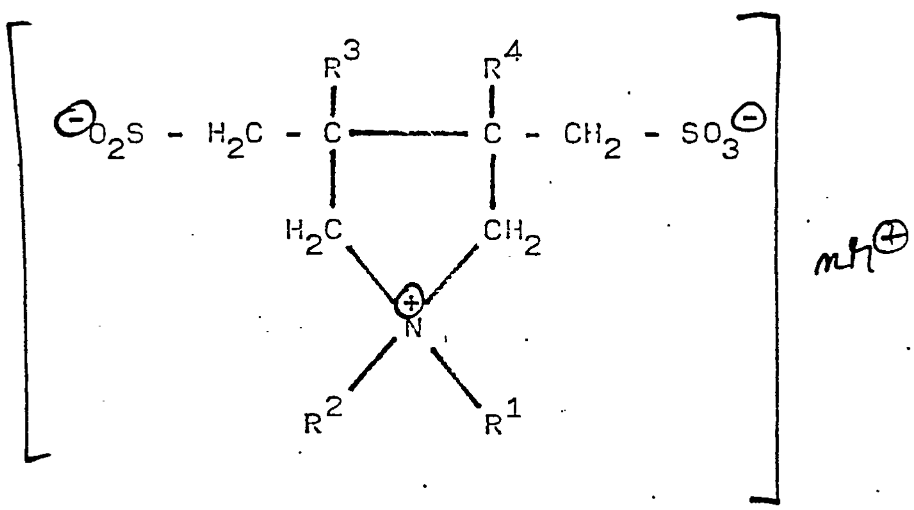

(I a),

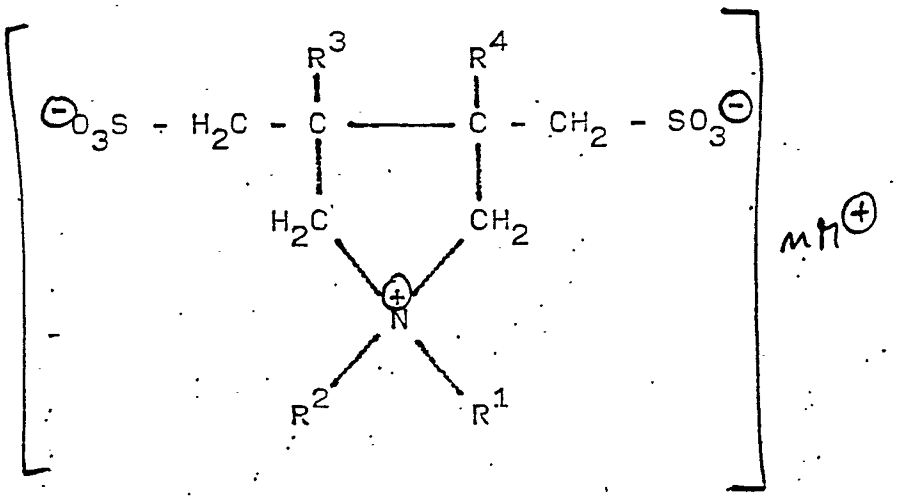
(I b),

worin bedeuten:

$R^1$ Wasserstoff, geradkettiges oder verzweigtes $C_{1-22}$-Alkyl, obei in der Kette -NH-CO- oder -CO-NH- enthalten sein können,
Benzyl,
$-CH_2-CH_2-CH_2-SO_3^{\ominus}$,
$-CH_2-CH_2-CH_2-SO_3^{\ominus}$ oder
$\quad | $
$\quad SO_2^{\ominus}$
$-CH_2-CH-CH_2-SO_3^{\ominus}$ darstellen,
$\quad | $
$\quad SO_3^{\ominus}$

$R^2$ unabhängig von $R^1$ Wasserstoff, geradkettiges oder verzweigtes $C_{1-22}$-Alkyl, wobei in der Kette -NH-CO- oder -CO-NH- enthalten sein können, oder gemeinsam mit $R^1$ eine am $N^{\ominus}$ - Atom des Pyrrolidiniumrings gebundene Tetramethylengruppe, die in den Positionen 2 und 3 durch eine Sulfinatomethyl- und eine Sulfonatomethylgruppe substituiert ist,

$R^3$, $R^4$ Wasserstoff oder Methyl,

$M^{\ominus}$ gleiche oder verschiedene Kationen aus der Gruppe $H^{\ominus}$, $NA^{\ominus}$, $K^{\ominus}$ und $NH_4^{\ominus}$ und

n 1, 2 oder 3.

Das erfindungsgemäße Verfahren zur Herstellung der 3-Sulfinatomethyl- oder 3-Sulfonatomethyl-4-sulfonatomethyl-pyrrolidiniumbetaine der Formeln Ia und Ib ist gekennzeichnet durch Umsetzung von

- Diallylammoniumsalzen der Formel II,

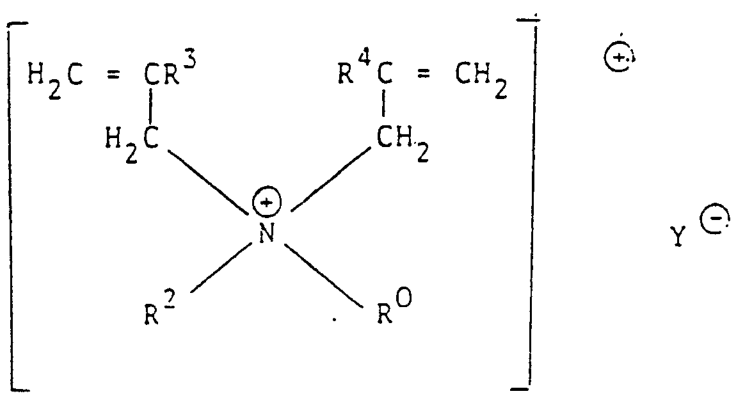
(II),

worin darstellen:

$R^2$, $R^3$ und $R^4$ dasselbe wie oben definiert,

$R^0$ unabhängig von $R^2$ dieselben Bedeutungen wie $R^2$ oder gemeinsam mit $R^2$ eine am $N^{\ominus}$-Atom des Pyrrolidiniumrings gebundene Tetramethylengruppe, die in den Positionen 2 und 3 durch eine

Sulfinatomethyl- und eine Sulfonatomethylgruppe substituiert ist, und

$Y^{\ominus}$ Chlorid, Bromid, Methosulfat oder Sulfat,

- Triallylammoniumsalzen der Formel III,

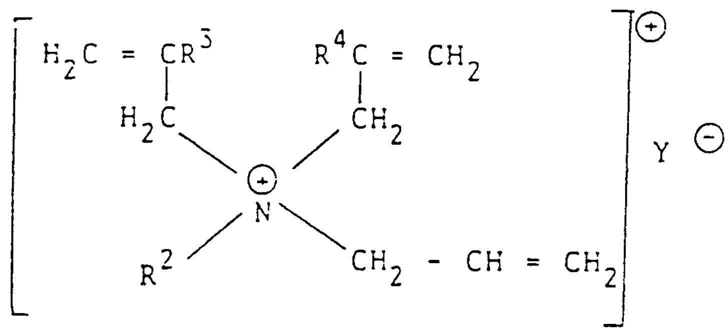

(III)

mit $R^2$, $R^3$, $R^4$ und $Y^{\ominus}$ wie oben oder

- entsprechenden Tetraallylammoniumsalzen mit äquimolaren Mengen eines Hydrogensulfits in Anwesenheit von Peroxodisulfaten allein oder im Gemisch mit unter Chlor, chlorabgebenden Stoffen, Chlorat, Bromat, Wasserstoffperoxid und Luft ausgewählten weiteren Oxidationsmitteln im pH-Bereich von 1,5 bis 6.

Hierbei entstehen die neuen 3-Sulfinatomethyl-4-sulfonatomethyl-pyrrolidiniumbetaine der Formel Ia dadurch, daß Diallylverbindungen der Formel II mit der zweifachen molaren Menge des Hydrogensulfits in Gegenwart einer katalytischen Menge des Peroxodisulfats bei pH-Werten von 2 bis 4 umgesetzt werden. Diese Reaktion wird im folgenden als Sulfocyclosulfinierung bezeichnet. Sie läuft außerordentlich schnell ab - der quantitative Umsatz wird in Minuten oder sogar Sekunden erreicht -, und man erhält das Endprodukt in großer Reinheit, wenn im genannten pH-Bereich zu den vereinigten Lösungen von Diallylammoniumsalz II und Hydrogensulfit auf einmal eine katalytische Menge eines Peroxodisulfats zugegeben wird. Als Hydrogensulfite werden vorzugsweise die Alkali- oder Ammoniumsalze eingesetzt.

Die Reaktion verläuft nach folgender Gleichung:

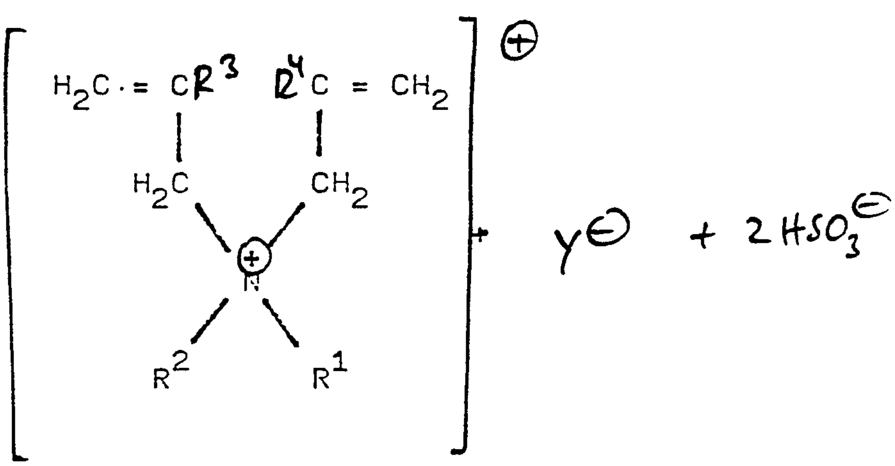

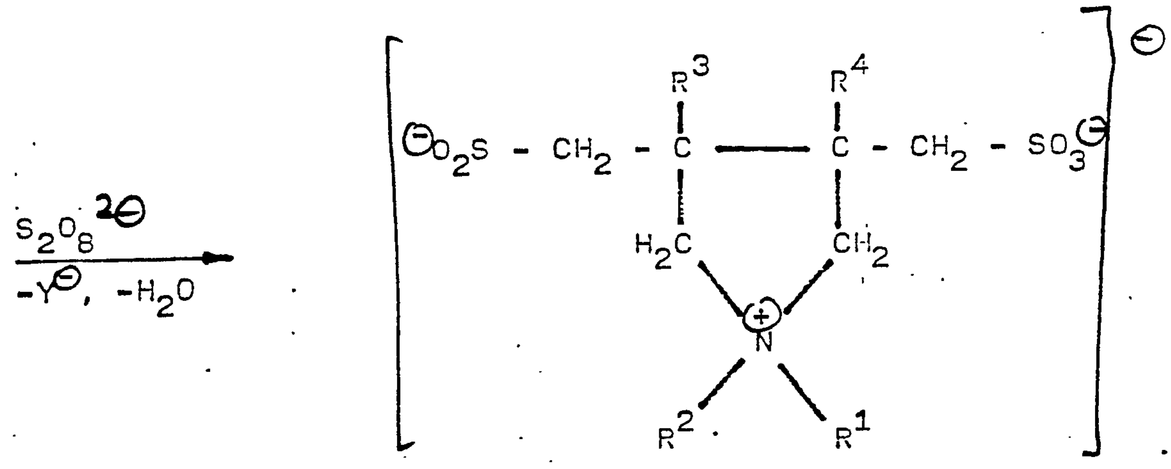

Die Bildung der Sulfobetain-sulfinate Ia war ferner auch deshalb überraschend, weil bekanntermaßen Sulfite durch Persulfate sehr leicht in Sulfate übergeführt werden und deshalb auch nach Angaben in DE-A1-2 313 539 (Seite 4) ihre Kombination als ungeeignetes Initiatorsystem gelten mußte.

Die beim Verfahren der DE-A1-2 331 515 eingesetzten unsubstituierten Olefine mit isolierten Doppelbindungen unterscheiden sich somit von den beim oben erläuterten erfindungsgemäßen Verfahren eingesetzten Verbindungen in ihrer Elektronenkonfiguration und ihrer Reaktivität.

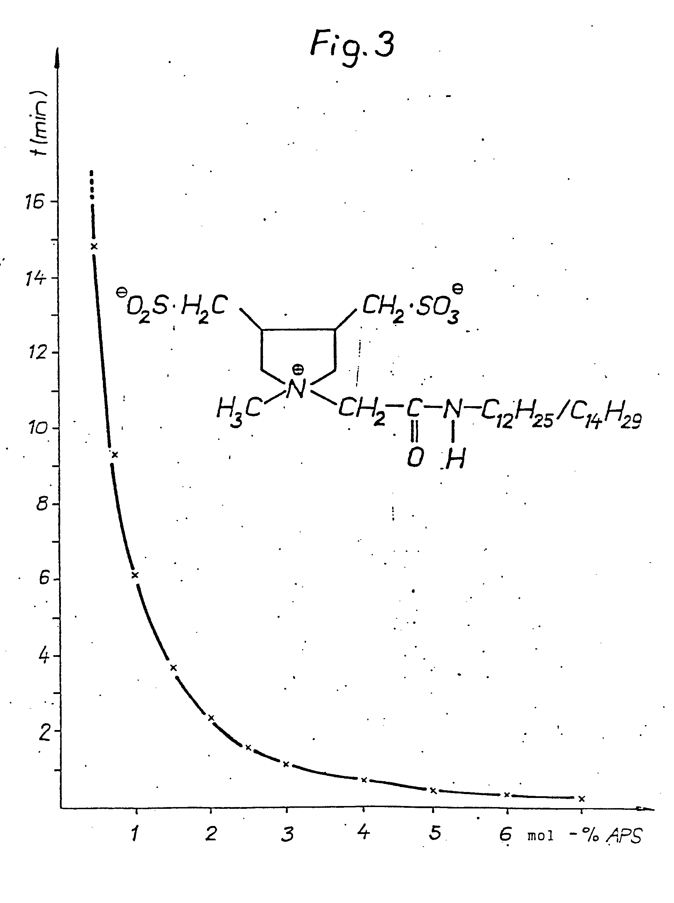

Die große Reaktionsgeschwindigkeit und die hohe Selektivität bei quantitativem Umsatz sind für eine in Lösung verlaufende, bei Raumtemperatur startende Radikalreaktion ganz überraschend. Bei der sauerstoffinduzierten Sulfocyclisierung der Diallylammoniumsalze in Gegenwart von Übergangsmetallen wird ohne großen Hydrogensulfitüberschuß gearbeitet, und zwar derart, daß die Komponenten über einen längeren Zeitraum simultan zum Reaktionsgemisch zugetropft werden, wobei durch vorgelegtes Neutralsulfit der pH-Wert der Reaktionsmischung im oberen Pufferbereich des Hydrogensulfit/Sulfit-Systems liegt. Diese Unterschiede in den Reaktionsbedingungen - im pH-Wert, in der Art der Vermischung der Reaktanden und des Angebots an schwefligsauren Salzen sowie der Reaktionsinitiierung - bewirken gegenüber dem bekannten Verfahren gemäß DD-A-154 444 einen völlig anderen Reaktionsverlauf. Die sehr kurzen Reaktionszeiten sowie die Möglichkeit des gleichzeitigen Vermischens der Komponenten und des Initiators ermöglichen die kontinuierliche Durchführung der Reaktion, indem die gleichzeitige Vermischung am Anfang einer Verweilzeitstrecke, z. B. eines Rohrreaktors, vorgenommen wird. Auf Grund der sehr hohen Raum-Zeit-Ausbeuten des erfindungsgemäßen Verfahrens können sowohl bei diskontinuierlicher als auch bei kontinuierlicher Reaktionsführung relativ kleine Reaktionsräume verwendet werden. Zur Initiierung der Sulfocyclosulfinierung werden Peroxodisulfate verwendet, bevorzugt im Bereich von 1 bis 3 mol-%; bei geringerer Initiatormenge erfolgt unvollständiger Umsatz; bei höherer Persulfatmenge wird zwar die Reaktionsgeschwindigkeit weiter gesteigert, jedoch besteht die Gefahr des unkontrollierbar heftigen Reaktionsverlaufs (vgl. Beispiel 12 sowie Fig. 2 und 3). Eine Erhöhung der Geschwindigkeit der Sulfocyclosulfinierungsreaktion wird auch erreicht, wenn die Konzentration der Ausgangsprodukte erhöht oder die Starttemperatur heraufgesetzt wird - was jedoch keine praktischen Vorteile (vgl. Beispiel 13) bringt. Die Initiierung der Reaktion ist nur mit Peroxodisulfat erfolgreich möglich; andere Perverbindungen, wie Wasserstoffperoxid (vgl. Beispiel 15) oder Perborat (vgl. Beispiel 16), sind als Initiatoren ungeeignet, da sie nur bevorzugt Sulfit zu Sulfat oxidieren; Luftsauerstoff als Initiator ist prinzipiell einsetzbar, ergibt aber verlängerte Reaktionszeiten und uneinheitliche Produkte (vgl. Beispiel 14). Oberhalb des optimalen pH-Wert-Bereiches entsteht in verstärktem Maße neben dem Sulfobetain-sulfinat das Sulfobetain, welches bei pH-Werten über 5 schließlich zum Hauptprodukt wird (vgl. Fig. 1, Tab. 1 und die Beispiele 1 bis 11). In Sonderfällen kann man jedoch so verfahren, daß in Anwesenheit von Übergangsmetallen (im vorgegebenen Konzentrationsbereich wie beim Verfahren nach DD-A-154 444) mit wenig Peroxodisulfat initiiert wird und anschließend die Reaktion mit Luftsauerstoff bis zum vollständigen Umsatz fortgeführt wird. Bei dieser Verfahrensweise läßt sich jedoch die Bildung von 3-Methyl-4-sulfonatomethyl-pyrrolidiniumbetain nicht ganz vermeiden, da dann bei verlängerter Reaktionszeit ein Verlust an Schwefeldioxid eintritt.

Die erhaltenen 3-Sulfinatomethyl-4-sulfonatomethyl-pyrrolidiniumbetaine Ia können aus ihrer Reaktionslösung entweder isoliert oder als Zwischenprodukte ohne Isolierung weiter umgesetzt werden.

Des weiteren werden neue, reaktive 1-(3'-Sulfo)propyl-3-sulfinatomethyl-4-sulfonatomethyl-pyrrolidiniumbetaine erhalten, wenn Triallylammoniumsalze der Formel III mit der dreifachen molaren Menge des Hydrogensulfits in Anwesenheit einer katalytischen Menge des Peroxodisulfats allein oder in Verbindung mit einer gleichzeitigen oder anschließenden Einwirkung von Luftsauerstoff bei pH-Werten von 2,5 bis 6,0, vorzugsweise 4,0 bis 5,5, umgesetzt werden.

Diese Trifunktionalisierung von Triallylammoniumsalzen zu Produkten mit drei sauerstoffhaltigen Schwefelfunktionen soll als Sulfocyclosulfinierung bezeichnet werden. Die durch diese Verfahrensweise überraschend ausgelöste exotherme Umsetzung unterscheidet sich grundlegend von allen bisher an Triallylammoniumsalzen durchgeführten Hydrogensulfitadditionen; der gleichzeitige Einbau einer Sulfinato-und zweier Sulfonatogruppen stellt eine neue Reaktion dar. Die Geschwindigkeit der nach einem Radikalmechanismus ablaufenden Reaktion ist auch überraschend; die Sulfocyclosulfinierung erfordert bei Initiatorkonzentrationen < 2 mol-% nur Minuten oder bei Konzentrationen > 2 mol-% sogar nur Sekunden bis zum praktisch vollständigen Umsatz. Diese Reaktion gehört damit zu den schnellsten in Lösung verlaufenden radikalischen Umsetzungen, welche die organische Chemie kennt.

Die Auslösung der Sulfocyclosulfinierungsreaktion ist spezifisch an die Anwesenheit von Salzen der Peroxodischwefelsäure, vorzugsweise Natrium-, Kalium- oder Ammoniumperoxodisulfat, gebunden; andere Perverbindungen, z. B. Wasserstoffperoxid oder Perborate, vermögen eine vergleichbare Reaktion nicht auszulösen, sondern oxidieren Sulfit lediglich zu Sulfat.

In speziellen Fällen ist es jedoch auch möglich, Peroxodisulfate und andere Oxidationsmittel, beispielsweise Sauerstoff oder insbesondere Luft, gemeinsam einzusetzen, derart, daß dann etwa 1 mol-% oder weniger eines Peroxodisulfats für die Initiierung der Reaktion in der Anfangsphase zudosiert und die Sulfocyclosulfinierung unter gleichzeitigem Einrühren oder Durchleiten von Luft zu Ende geführt wird. Mit dieser Verfahrensweise müssen aber verlängerte Reaktionszeiten in Kauf genommen werden, jedoch verändert sich die Zusammensetzung der Reaktionsprodukte nicht.

Die Selektivität des Reaktionsverlaufs ist aber in hohem Maße vom eingestellten Start-pH-Wert abhängig. So werden im Vorzugs-pH-Wert-Bereich 4,0 bis 5,5 die neuen Sulfobetainsulfinate-sulfonate mit einem Anteil von 50 bis 70 % neben den noch weiter sulfinierten Produkten.

1-(2,-Sulfinato-3'-sulfonatopropyl)-3-sulfinatomethyl-4-sulfonatomethyl-pyrrolidiniumbetainen A und den bekannten (vgl. DD-A-200 739) 1-(3'-Sulfonatopropyl)-3-methyl-4-sulfonatomethyl-pyrrolidiniumbetainen B erhalten:

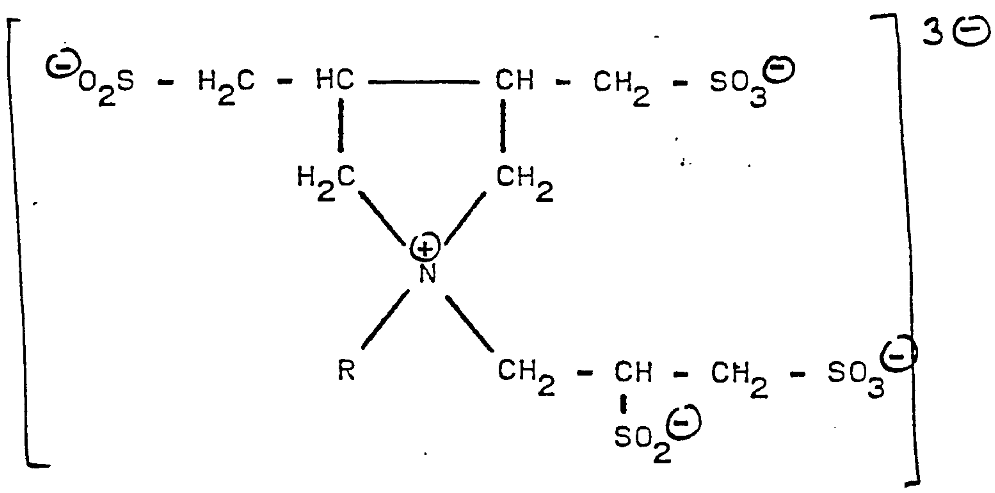

(A)

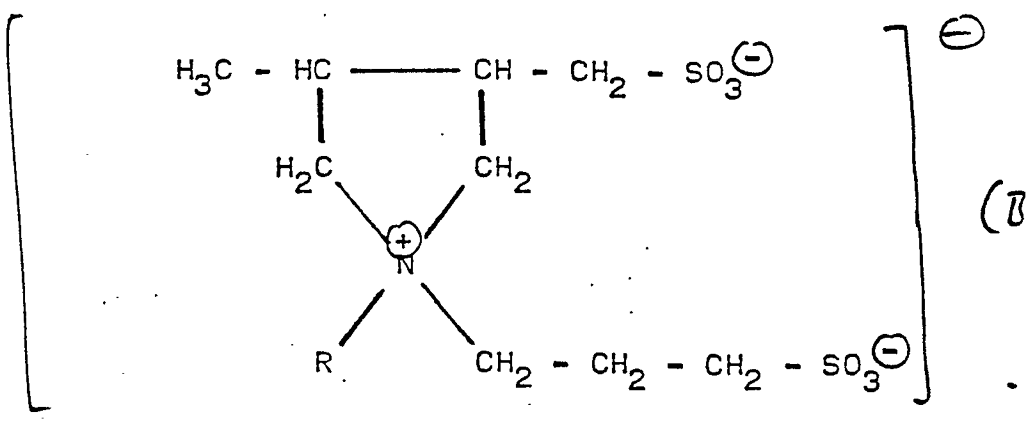

(B)

Senkt man den Start-pH-Wert unter 4 ab, so enthält das Reaktionsprodukt praktisch kein B mehr, während bei pH-Werten oberhalb von 5,5 praktisch kein A gebildet wird. Der für die Durchführung des Verfahrens günstige pH-Wert-Bereich 4,0 bis 5,5 wird darüber hinaus bereits durch Mischung der Triallylammoniumsalze mit technischer Hydrogensulfitlösung, gegebenenfalls unter Zusatz von geringen Mengen einer Base, erreicht.

Erfindungsgemäß können die Triallylammoniumsalze III auch mit der mindestens vierfachen molaren Menge des Hydrogensulfits in Gegenwart einer katalytischen Menge des Peroxodisulfats bei pH-Werten von 1,5 bis 2,5 umgesetzt werden.

Die erfindungsgemäße Durchführung der Reaktion erfolgt in einfacher Weise so, daß man 1 mol einer

wäßrigen Lösung eines Triallylammoniumsalzes mit 4 mol eines Alkali- oder Ammoniumhydrogensulfits versetzt, den pH-Wert des Reaktionsgemisches auf 1,5 bis 2,5 einstellt und dann eine katalytische Menge von 1 bis 4 mol-% eines Ammonium- oder Alkaliperoxodisulfats als Feststoff oder gelöst auf einmal zusetzt; bei Raumtemperatur beginnend, ist die exotherme Umsetzung zu den Sulfinaten in etwa 1 bis 3 Minuten beendet. Der Umsatz ist nahezu quantitativ, wie sich anhand des Sulfitverbrauchs und der bromatometrischen Produktbestimmung ermitteln läßt. $^1$H-NMR-spektroskopisch läßt sich die Beendigung der Reaktion am Verschwinden der Allylprotonensignale erkennen. Die erfindungsgemäße Eingrenzung des pH-Wert-Bereiches auf 1,5 bis 2,5 ist für die Selektivität entscheidend, da mit höherem pH-Wert der Anteil an mitgebildeten Sulfonaten erhöht wird. Auch bei sorgfältigem Ausschluß von Luftsauerstoff wird die Reaktion durch Peroxodisulfate eingeleitet, wodurch deren ausschlaggebende Rolle als Initiatoren belegt wird. In Sonderfällen läßt sich eine durch Peroxodisulfat eingeleitete Reaktion bei Peroxodisulfatmangel (weniger als 0,5 mol-%) mit Luftsauerstoff fortführen, allerdings nur mit Verlust an Selektivität und Zunahme von Reaktionsprodukten ohne Sulfinatogruppen.

In dem speziellen Falle, daß das eingesetzte Allylammoniumsalz ein Tetraallylammoniumhalogenid ist, erhält man in einer doppelten Sulfocyclosulfinierungsreaktion die isomeren Spiroverbindungen IV, welche Bis-(sulfobetainsulfinate) darstellen.

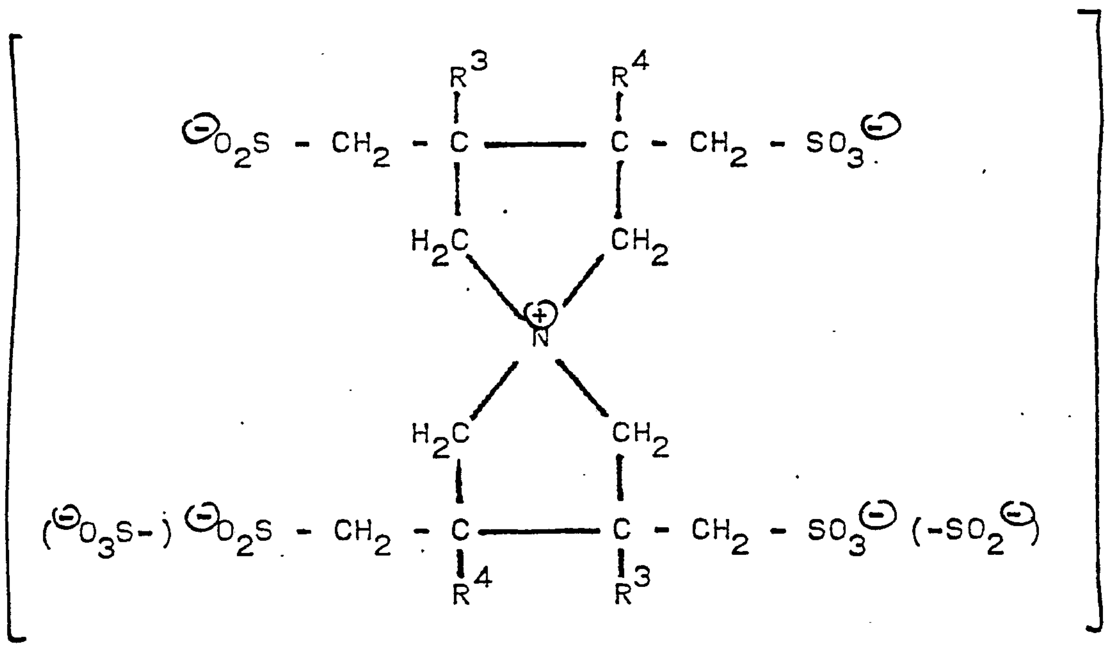

(IV)

Die neuen 3-sulfonatomethyl-4-sufonatomethyl-pyrrolidiniumbetaine der Formel Ib entstehen dagegen dadurch, daß Diallylammoniumsalze der Formel II pro Mol mit der zweifachen molaren Menge des Hydrogensulfits und der äquimolaren Menge Peroxodisulfat oder bei gleichzeitiger Anwesenheit anderer Oxidationsmittel mit insgesamt zwei Oxidationsäquivalenten bei pH-Werten von 2 bis 4 umgesetzt werden.

Diese Reaktion verläuft nach folgender Gleichung:

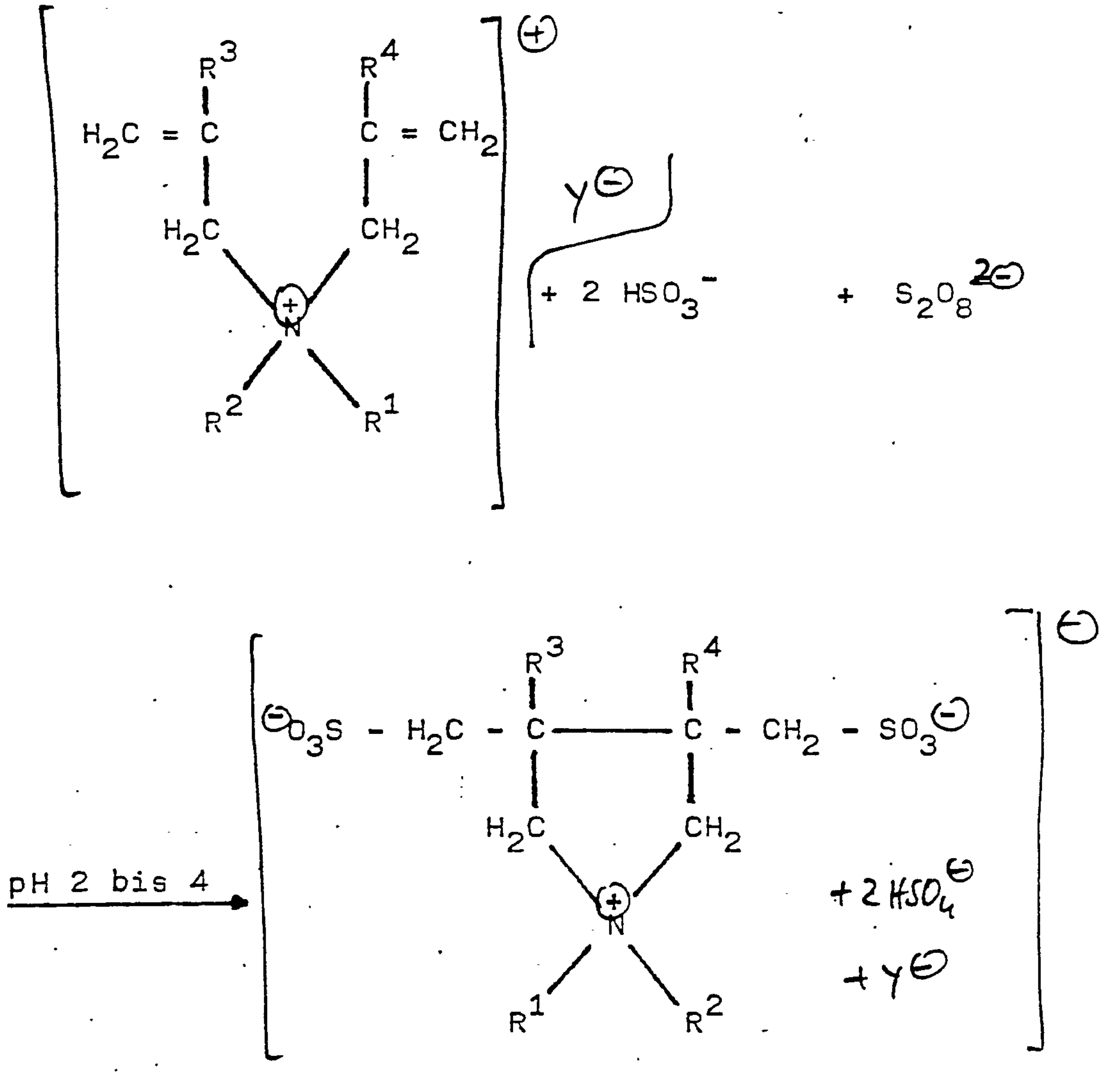


Die durch diese Verfahrensweise ausgelöste Reaktion unterscheidet sich in Verlauf und Endprodukt grundlegend von der zu den entsprechenden 3-sulfonatomethyl-pyrrolidiniumbetainen (DD-A-154 444) führenden Umsetzung mit nur 1 mol Hydrogensulfit pro Diallylammoniumsalz und Initiierung mit Luftsauerstoff /Übergangsmetall. Der Einbau einer zweiten Sulfonsäuregruppe unter diesen Bedingungen ist überraschend und wurde in dieser Form auch in anderen Verbindungsklassen bisher nicht festgestellt.

Die Reaktion ist spezifisch an die Anwesenheit von Peroxodisulfaten gebunden. Andere Perverbindungen, beispielsweise Wasserstoffperoxid oder Alkaliperborate, lösen eine vergleichbare Reaktion nicht aus, sondern oxidieren lediglich Sulfit zu Sulfat. Es ist jedoch möglich, in speziellen Fällen erfindungsgemäß Peroxodisulfate und andere Perverbindungen gemeinsam einzusetzen oder Peroxodisulfate und andere Oxidationsmittel derart zy kombinieren, daß dann weniger als die molare Menge Peroxodisulfat pro mol Allylverbindung erforderlich ist. Hierzu eignen sich z. B. Wasserstoffperoxid, Chlor, Chlorat, Bromat u. a. m.

Vorzugsweise arbeitet man erfindungsgemäß so, daß man in wäßriger Lösung die Diallylammoniumsalze mit 2 mol Hydrogensulfit mischt, auf den pH-Wert 2 (bis max. 4) einstellt und unter Rühren das Peroxodisulfat einträgt, wobei sich die fortschreitende Reaktion durch rasche Erwärmung der Reaktionsmischung zu erkennen gibt, die dabei bis zum Sieden kommen kann.

Langkettig substituierte Vertreter ergeben Pyrrolidiniumbetainsulfonate mit Tensideigenschaften, die bei der beschriebenen Verfahrensweise aus dem Reaktionsgemisch ausfallen und leicht isoliert und in reiner Form gewonnen werden können. In anderen Fällen wird vor der Aufarbeitung die entstandene Schwefelsäure neutralisiert und das Betainsulfonat im Bedarfsfalle extraktiv vom anorganischen Salzanteil abgetrennt.

In einer geeigneten Vorrichtung ist das erfindungsgemäße Verfahren kontinuierlich durchführbar, wenn die Komponenten im angegebenen Molverhältnis am Anfang einer Verweilzeitstrecke zusammengeführt werden.

Des weiteren werden neue 1-(3'-Sulfonatopropyl-3,4-disulfonatomethyl -pyrrolidiniumbetaine erhalten, wenn Triallylammoniumsalze der Formel III pro Mol mit der dreifachen molaren Menge des Hydrogensulfits und der äquimolaren Menge Peroxodisulfat oder bei gleichzeitiger Anwesenheit anderer Oxidationsmittel mit insgesamt zwei Oxidationsäquivalenten bei pH-Werten von 2,5 bis 6,0, vorzugsweise zwischen 4,0 und 5,5, umgesetzt

werden.

Die durch diese Verfahrensweise ausgelöste exotherme Umsetzung läuft ebenfalls schnell ab und sulfoniert die eingesetzten Triallylammoniumsalze in wenigen Minuten quantitativ. Die Selektivität des Reaktionsverlaufs ist jedoch in hohem Maße vom eingestellten Start-pH-Wert abhängig. So werden im pH-Wert-Bereich von 4,0 bis 5,5 die neuen Sulfobetaindisulfonate mit einem Anteil von 50 bis 70 % neben den vier Sulfonsäuregruppen enthaltenden 1-(2',3'-Disulfonatopropyl)-3,4-disulfonatomethyl-pyrrolidiniumbetainen C und den bekannten 1-(3'-Sulfonatopropyl)-3-methyl-4-sulfonatomethyl-pyrrolidiniumbetainen B erhalten.

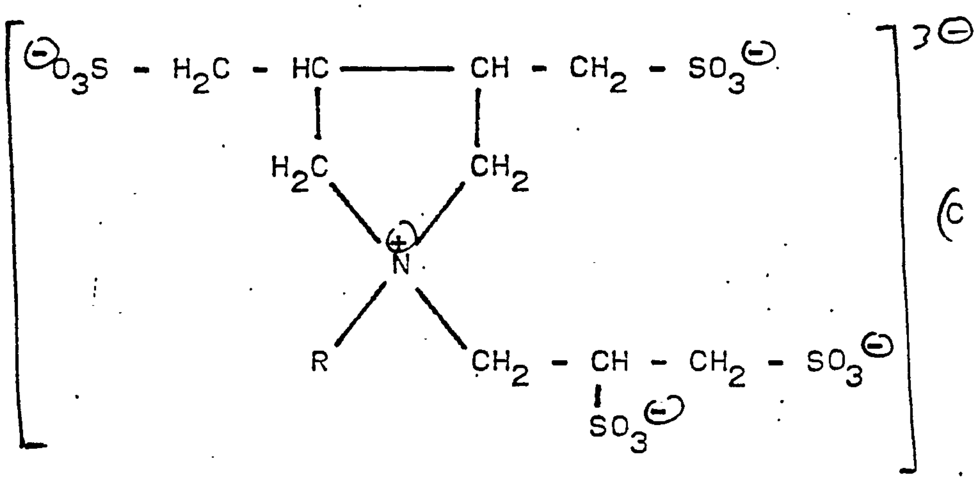

(C),

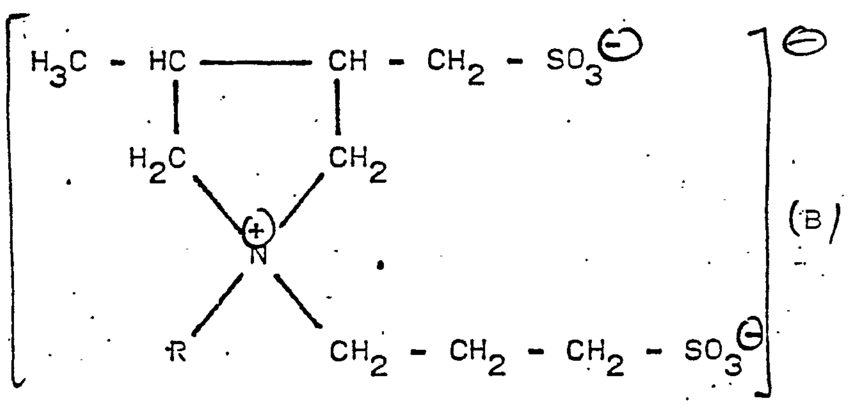

(B).

Senkt man den Start-pH-Wert unter 4, so enthält das Reaktionsprodukt praktisch keine Verbindung B mehr, während bei pH-Werten oberhalb von 5,5 praktisch keine Verbindung C gebildet wird. Der für die Durchführung des Verfahrens günstige pH-Wert-Bereich von 4,0 bis 5,5 wird darüber hinaus bereits durch Mischen der Triallylammoniumsalze mit technischer Hydrogensulfitlösung, gegebenenfalls unter Zusatz von geringen Mengen Natronlauge, erreicht.

Die zusätzliche Einführung weiterer Sulfonsäuregruppen unter den erfindungsgemäßen Bedingungen in Verbindungen der allgemeinen Formel B ist völlig überraschend und wurde in dieser Form auch in anderen Verbindungsklassen noch nie beobachtet.

Zur Herstellung der Sulfobetaindisulfonate arbeitet man vorzugsweise in wäßriger Lösung bei möglichst hohen Konzentrationen der Reaktionspartner, indem man die kristallinen Triallylammoniumsalze oder ihre Lösungen mit gesättigter, technischer Hydrogensulfitlösung mischt, den pH-Wert der Mischung vorzugsweise zwischen 4 und 5,5 einstellt und das Oxidationsmittel als Lösung oder in kristalliner Form zusetzt.

Die fortschreitende Umsetzung ergibt sich dabei durch sehr schnelle Erwärmung der Reaktionsmischung zu erkennen. Die Mischung kann sich dabei bis zum Sieden erwärmen, wenn die Reaktionspartner in hoher Konzentration vorliegen. Die stark exotherme Reaktion kann jedoch leicht beherrscht werden, wenn man das Oxidationsmittel oder die Oxidationsmittelkombination im Verlaufe einiger Minuten zusetzt oder durch Anwendung äußerer Kühlung die freiwerdende Reaktionswärme abführt.

Ebenso können Triallylammoniumsalze der Formel III pro Mol auch mit der vierfachen molaren Menge des Hydrogensulfits und der zweifachen molaren Menge des Peroxodisulfats oder bei gleichzeitiger Anwesenheit anderer Oxidationsmittel mit insgesamt vier Oxidationsäquivalenten bei pH-Werten von 1,5 bis 2,5 umgesetzt werden. Dabei entstehen neue 1-(2',3'-Disulfonatopropyl-3,4-disulfonatomethyl-pyrrolidiniumbetaine, die Sulfonatobetaine mit 3 zusätzlichen hydrophilen Sulfonatgruppen sind. Sie können aufgrund dieser ladungstragenden Gruppen zur Herstellung von Metallsalzen, als Leitsalze oder als leitfähige Beschichtungsmittel verwendet werden. Verbindungen mit derart vorteilhafter Wirkung sind bisher noch nicht bekannt.

Langkettig substituierte Vertreter der neuen Verbindungsklasse haben gute, in einem weiten pH-Wert-Bereich wirksame Tensideigenschaften.

Die durch diese erfindungsgemäße Verfahrensweise ausgelöste Reaktion verläuft rasch, exotherm und quantitativ und unterscheidet sich in Verlauf und Endprodukt grundlegend von der Sulfocyclisierung von Diallylammoniumsalzen gemäß DD-A-154 444 bei Initiierung mit Luftsauerstoff/Übergangsmetall. Der Einbau von 3 weiteren Sulfonsäuregruppen unter den erfindungsgemäßen Bedingungen ist überraschend und wurde in dieser Form auch in anderen Verbindungsklassen noch nie beobachtet. Alle weiteren Sachverhalte entsprechen den vorher beschriebenen.

Als Di- und Triallylammoniumsalze sind insbesondere die Chloride, Bromide, Methosulfate oder Sulfate in entsprechenden Äquivalentmengen geeignet.

Als weitere Oxidationsmittel, die im Gemisch mit dem Peroxodisulfat einsetzbar sind, werden vorzugsweise, wie bereits oben beschrieben, Chlor oder chlorabgebende Stoffe, Chlorate, Bromate, Wasserstoffperoxid oder Luft verwendet.

Die neuen 3-Sulfinatomethyl-4-sulfonatomethyl-pyrrolidiniumbetaine besitzen wertvolle Eigenschaften. Vertreter mit langkettigen Substituenten sind als spezielle Tenside verwendbar.

Des weiteren dienen diese neuen Verbindungen auch als reaktive Zwischenprodukte für andere Synthesen, da sie zusätzliche hydrophile und polare Gruppen besitzen: Die Sulfinatogruppe, die aufgrund ihrer Reaktivität (nucleophil, alkylierbar, komplexbildend, reduzierend) die neuen Verbindungen zu vielseitig verwendbaren Zwischenprodukten macht, und die Sulfonatogruppe, welche die Polarität und Löslichkeit der Stoffe erhöht und die Tensideigenschaften der Stoffgruppe bestimmt.

**Ausführungsbeispiele**

Beispiele 1 bis 10 (vgl. Tab. 1/Fig. 1)

Sulfocyclosulfinierung von Dimethyldiallylammoniumchlorid zu reinem Natrium-1,1-dimethyl-3-sulfinatomethyl-4-sulfonatomethyl-pyrrolidiniumbetain ($R^1 = R^2 = CH_3$, $R^3 = R^4 = H$ in der allgemeinen Formel Ia), Natrium-1,1-dimethyl-3-sulfinatomethyl-4-sulfonatomethyl-pyrrolidiniumbetain/1,1,3-Trimethyl-4-sulfonatomethyl-pyrrolidiniumbetain-Gemischen und reinem 1,1,3-Trimethyl-4-sulfonatomethyl-pyrrolidiniumbetain.

Allgemeine Arbeitsvorschrift zur Herstellung von reinem Natrium-1,1-dimethyl-3-sulfinatomethyl-sulfonato-pyrrolidiniumbetain und dessen Gemischen mit 1,1,3-Trimethyl-4-sulfonatomethyl-pyrrolidiniumbetain:

In einen mit Rührer; Thermometer und Glaselektrode versehenen Sulfierkolben wurden nacheinander 307,4 g (1 mol) 52,6 %-ige wäßrige technische Dimethyldiallylammoniumchloridlösung mit 4,85 % Natriumchloridgehalt, 516,5 g (2,02 mol) 40,7 %-ige technische Natriumhydrogensulfitlösung mit einem Eisengehalt von 60 mg/l, die entsprechende Menge 37 %-ige Salzsäure oder 33 %-ige Natronlauge zur Einstellung des jeweiligen pH-Wertes (vgl. Tab. 1) sowie soviel Wasser eingetragen und durch Rühren homogenisiert, daß eine Arbeitsmenge von 1000 g eingehalten wurde, um damit die Vergleichbarkeit der erzielten Ergebnisse zu gewährleisten.

In den Fällen, wo größere Mengen Natronlauge zur Einstellung des pH-Wertes erforderlich sind, erwärmt sich die Ausgangslösung. Sie sollte vor der Umsetzung auf Raumtemperatur abgekühlt werden. Zu der so vorbereiteten fahlgelben Startlösung fügt man auf einmal 5,4 g (2 mol-%) feingepulvertes Kaliumperoxodisulfat unter Rühren hinzu, das sofort aufgelöst worauf sich die Reaktionslösung insbesondere bei pH-Werten unter 5 blutrot färbt. Bei pH-Werten zwischen 1,7 und 4,5 erhöht sich die Reaktionstemperatur innerhalb von einer Minute (vgl. Tab. 1) um 45 bis 50°C. Nach Erreichen des Temperaturmaximums ist die Umsetzung beendet.

Bei pH-Werten von 5 bis 6,2 verlängern sich die Reaktionszeiten bis auf 20 Minuten. Nach erfolgter Umsetzung kann man die durch den Einsatz eisenhaltiger Chemikalien erfolgte Rotfärbung der Reaktionsmischung durch Fe(III)-sulfinate durch Zusatz von Komplexbildnern (z. B. Dimethylaminomethan-bis(phosphonsäure) entfärben oder durch Einstellen eines pH-Wertes um 7 das gelöste Eisensalz als Eisen(III)-hydroxid zur Fällung bringen und durch Filtration von der dann farblosen Reaktionsmischung abtrennen.

Tab. 1: Sulfocyclosulfinierung von Dimethyldiallylammoniumchlorid unter Variation des pH-Wertes

| Beispiel | pH-Wert | Zusatz von 37 %-ige HCl (g) | Zusatz von 33 %-iger NaOH (g) | Reaktionszeit bis zum Temperaturmaximum (min) |
|---|---|---|---|---|
| 1 | 1,7 | 60,0 | - | 1 |
| 2 | 2,0 | 35,0 | - | 1 |
| 3 | 2,5 | 12,8 | - | 1 |
| 4 | 3,0 | 5,5 | - | 1 |
| 5 | 4,0 | - | - | 1 |
| 6 | 4,5 | - | 4,0 | 1 |
| 7 | 5,0 | - | 7,6 | 2 |
| 8 | 5,5 | - | 20,0 | 4 |

| | | | | |
|---|---|---|---|---|
| 9 | 6,0 | - | 42,7 | 15 |
| 10 | 6,2 | - | 121,2 | 20 |

Ein aliquoter Teil der nach Tabelle 1 erhaltenen Reaktionslösungen wurde zur Trockne zu einem salzartigen Rückstand eingeengt und $^1H$-NMR-spektroskopisch untersucht, wobei die quantitative Zusammensetzung durch Vergleich der Intensitäten geeigneter Signale bestimmt wurde. Die Untersuchungsergebnisse sind in Fig. 1 zusammengefaßt. Danach sind reine Sulfobetainsulfinate nur bei einem pH-Wert $\leqslant$ 2 zugänglich (Beispiele 1 und 2). Bei höheren pH-Werten der Startlösung (Beispiele 3 bis 10) nimmt der Anteil an 1,1,3-Trimethyl-4-sulfonatomethyl-pyrrolidiniumbetain stetig zu.

Ein aliquoter Teil der nach Beispiel 2 (vgl. Tab. 1) hergestellten Reaktionslösung wurde zunächst mit Natronlauge auf den pH-Wert 7 eingestellt, mit wenigen Tropfen $H_2O_2$ versetzt und nach Abfiltrieren des unlöslichen Eisen(III)-hydroxids unter vermindertem Druck zur Trockne eingedampft. Der verbliebene Rückstand wurde $^{13}C$-NMR-spektroskopisch untersucht ($D_2O$, externer Standard TMS). Das Spektrum wies aus, daß das Natrium-1,1-dimethyl-3-sulfinatomethyl-4-sulfonatomethyl-pyrrolidiniumbetain vorwiegend in der cis-Konfiguration in bezug auf die Substituenten in der 3,4-Position neben geringen trans-Anteilen vorliegt.

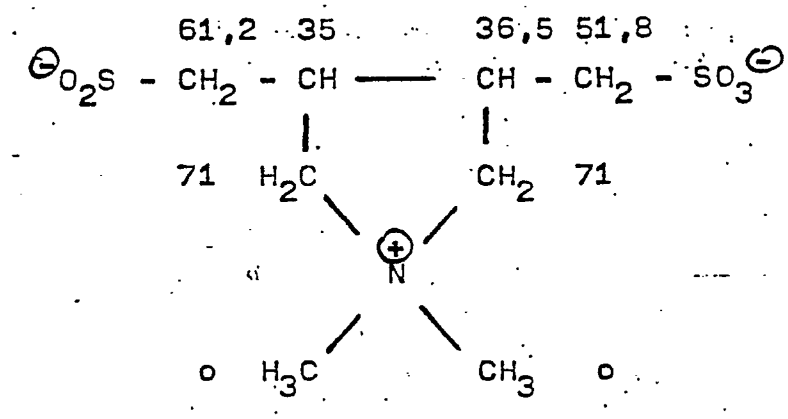

Die Zahlenangaben an den Atomsymbolen entsprechen den chemichen Verschiebungen für die cis-Konfiguration in ppm. o: 56,2; 56,0; 55,9/54,6; 54,5; 54,3. Die N-$CH_3$-Gruppen sind nicht äquivalent; Signalaufspaltung durch $^{14}N$-Quadrupolmoment. Die Signale der trans-Verbindung bei 38,6 ppm und 40,9 ppmentsprechen den beiden CH-Gruppen sowie bei 63,9 ppm der -$CH_2$-$SO_2^-$-Gruppe.

**Beispiel 11**

Reines 1,1,3-Trimethyl-4-sulfonatomethyl-pyrrolidiniumbetain durch Sulfocyclisierung von Dimethyldiallylammoniumchlorid bei pH-Werten $\geqslant$ 7,5 (vgl. Fig. 1, gestrichelte Linie).

In einem offenen Gefäß, ausgerüstet mit einer Glaselektrode und einem Intensivrührer, wurden 18,8 g (0,15 mol) Natriumsulfit in Leitungswasser zu 125 ml Lösung aufgelöst; der pH-Wert der Lösung betrug 9,1. Unter heftigem Rühren wurde dann Luft feinst verteilt in die Lösung eingerührt. Zu dieser tropfte man aus zwei Büretten gleichzeitig und gleichmäßig eine Lösung von 46,1 g (0,15 mol) 52,6 %-iger Dimethyldiallylammoniumchloridlösung, verdünnt mit Leitungswasser auf 50 ml, und eine Lösung von 38,35 g (0,15 mol) 40,7 %-iger technischer Natriumhydrogensulfitlösung, verdünnt mit Leitungswasser auf 50 ml, in dem Maße zu, daß ein pH-Wert von $\geqslant$ 7,5 aufrecht erhalten werden konnte. Zun Zutropfen der beiden Lösungen wurden 39 Minuten benötigt; während dieser Zeit stiegt die Reaktionstemperatur von 25 auf 39°C an. Der pH-Wert hielt sich in der Zutropfphase zwischen 7,65 und 8,0. Ein von der Reaktionslösung angefertigtes $^1H$-NMR-Spektrum wies den quantitativen Umsatz des Allylammoniumsalzes zum Sulfobetain aus.

Zur Prüfung auf Abwesenheit von Sulfinatanteilen vgl. Beispiele 1 bis 10. Zur Abtrennung des 1,1,3-Trimethyl-4-sulfonatomethyl-pyrrolidiniumbetains von den anorganischen Salzen wurde die gesamte Reaktionslösung unter vermindertem Druck zur Trockne eingeengt und der verbliebene kristalline Rückstand mit konzentrierter Salzsäure behandelt. Die salzsaure Lösung wurde anschließend durch Filtration von den anorganischen Salzen abgetrennt und erneut unter vermindertem Druck zu einem hochviskosen, noch salzsäurehaltigen Sirup eingeengt, aus dem nach einigem Stehen das Sulfobetain in glänzenden Blättchen zu kristallisieren begann. Fügte man zu einer in Wasser aufgelösten Probe des hochviskosen Sirups einige Tropfen einer Eisen(III)-sulfat-Lösung hinzu, färbte sich die Lösung nicht blutrot, was die Abwesenheit von Sulfinatanteilen beweist. Durch Zusatz von Ethanol kann das Betain augenblicklich zur Kristallisation gebracht werden, wobei ein weißer Kristallbrei entsteht. Das Sulfobetain ist aus Wasser umkristallisierbar. Sein $^{13}C$-NMR-Spektrum ergab chemische Verschiebungen für die einzelnen C-Atome, wie sie bereits in DD-A-154-444 (Beispiel 1a) beschrieben worden sind.

Verringert man z. B. die Menge an Natriumsulfit von 1 mol auf 0,6 mol pro Mol Dimethyldiallylammoniumchlorid und pro Mol Natriumhydrogensulfit, wird damit automatisch die Pufferkapazität der Reaktionslösung herabgesetzt, wodurch sich die Dauer der Zutropfzeit von 39 auf 75 Minuten erhöht, wenn ein pH-Wert von 7,5 aufrechterhalten werden soll. Bei weiterer Reduzierung des Natriumsulfit-Puffers können nur noch pH-Wert-Bereiche unterhalb von 7,5 realisiert werden, was die Bildung des Sulfobetainsulfinats fördert (vgl. Fig.1, gestrichelter Kurvenabschnitt).

**Beispiel 12**

Sulfocyclosulfinierung von Dimethyldiallylammoniumchlorid unter Variation der Initiatorkonzentration.

Dieses Beispiel erläutert die Abhängigkeit der Dauer der exothermen Sulfocyclosulfinierungsreaktion des Dimethyldiallylammoniumchlorids zum Natrium-1,1-dimethyl-3-sulfinatomethyl-4-sulfonatomethyl-pyrrolidiniumbetain ($R^1$ = $R^2$ = $CH_3$/ $R^3$ = $R^4$ = H in der allgemeinen Formel Ia) von der eingesetzten Menge Ammoniumperoxodisulfat (APS) bei einem pH-Wert von 2,5 und einer Konzentration von 1 mol der Diallylverbindung und 2,1 mol Natriumhydrogensulfit/kg Reaktionsgemisch (16,2 Masse-% Dimethyldiallylammoniumchlorid). Die eingesetzte Initiatormenge wurde zwischen 0 Mol-% und 8 Mol-% APS variiert. Das Gesamtergebnis veranschaulicht Fig. 2.

Stellvertretend für die Versuchsserie soll die Initiierung mit 4 Mol-% APS näher beschrieben werden:

In einem Sulfierkolben mit Rührer und Thermometer wurden 307,4 g (1 mol) 52,6 %-iges technisches Dimethyldiallylammoniumchlorid mit 4,85 % NaCl-Gehalt, 618,5 g (2,1 mol)-35,33 %-iger technischer Natriumhydrogensulfitlösung mit einem $Fe^{++}$-Gehalt von 8 mg/l und 12,8 g 37 %-iger Salzsäure unter Rühren miteinander vereinigt. Der pH-Wert betrug 2,5. Dann wurden in 52,2 g Leitungswasser 9,12 g (4 mol-%) APS aufgelöst und auf einmal unter Rühren zu der bereiteten Mischung zugefügt. Die Reaktionsmischung färbte sich augenblicklich rot und erwärmte sich nach 10 Sekunden von 23 auf 45°C; nach 15 Sekunden stieg die Temperatur weiter auf 68°C an und erreichte schließlich nach 20 Sekunden das Maximum von 71°C. Die Umsetzung war zu diesem Zeitpunkt quantitativ. Nach 1 Minute war die Reaktionstemperatur bereits wieder auf 70°C abgefallen. Die $^1H$-NMR-spektroskopische Ausbeutebestimmung wies einen Gehalt von 98 % Sulfobetainsulfinat in der Reaktionsmischung aus (vgl. Beispiel 3; Fig. 1). Bei Initiierung mit noch höheren Initiatorkonzentrationen, z. B. 8 mol-%, erhöht sich die Reaktionsgeschwindigkeit derart, daß das Reaktionsgemisch unmittelbar nach der Initiatorzugabe innerhalb von wenigen Sekunden heftig aufsiedet.

**Beispiel 13**

1,1-Dimethyl-3-sulfinsäuremethyl-4-sulfonatomethyl-pyrrolidiniumbetain ($R^1$ = $R^2$ = $CH_3$, $R^3$ = $R^4$ = H in der allgemeinen Formel Ia).

307,4 g (1 mol) 52,6 %-ige technische Dimethyldiallylammoniumchloridlösung, 516,5 g (2,02 mol) 40,7 %-ige technische Natriumhydrogensulfitlösung und 35 g 37 %-ige Salzsäure wurden zu einer Lösung vom pH-Wert 2 miteinander gemischt. Anschließend fügte man auf einmal 5,4 g (2 mol-%) feingepulvertes Kaliumperoxodisulfat unter Rühren hinzu. Augenblicklich färbte sich die Lösung rot; nach 30 Sekunden hatte sich die Temperatur der Lösung von 21 auf 70,5°C erhöht. Nach dieser Reaktionszeit war der Umsatz bereits quantitativ. (Bei Einsatz einer noch höheren Konzentration an Diallylverbindung (z. B. 80 %) wird die Reaktionsgeschwindigkeit so groß, daß das Reaktionsgemisch unmittelbar nach der Initiatorzugabe (2 mol-%) heftig aufsiedet).

Zur Abtrennung der Sulfobetainsulfinsäure von den anorganischen Salzen wurde die abgekühlte Reaktionslösung unter vermindertem Druck zu einem sirupösen, salzartigen Rückstand eingedampft und mit einer ausreichenden Menge konzentrierter Salzsäure (500 g) durchgearbeitet. Dann filtrierte man von den anorganischen Salzen - vorwiegend Natriumchlorid ab und dampfte das Filtrat erneut zu einer gelblichen, glasigen Masse ein, aus der ein Teil der Sulfinsäure nach kurzer Zeit auskristallisierte. Durch Zusatz von 500 ml Ethanol kann nach intensivem Durcharbeiten die vollständige Kristallisation erreicht werden. Nach Absaugen des Ethanols sowie zweimaligem Nachwaschen mit je 250 ml Ethanol, Absaugen und Trocknen bis zur Massenkonstanz wurden schließlich 222 g pulverförmige, farblose, kristalline Sulfinsäure mit einem Anteil von 1,8 % Natriumchlorid isoliert. Bromatometrisch wurde der Gehalt an Sulfinsäure zu 80 % bestimmt. Die Sulfinsäure beginnt sich bei einer Temperatur oberhalb 225°C zu zersetzen.

Ergebnisse der $^{13}C$-NMR-Spektrometrie der cis-Sulfinsäure-($D_2O$, externer Standard TMS):

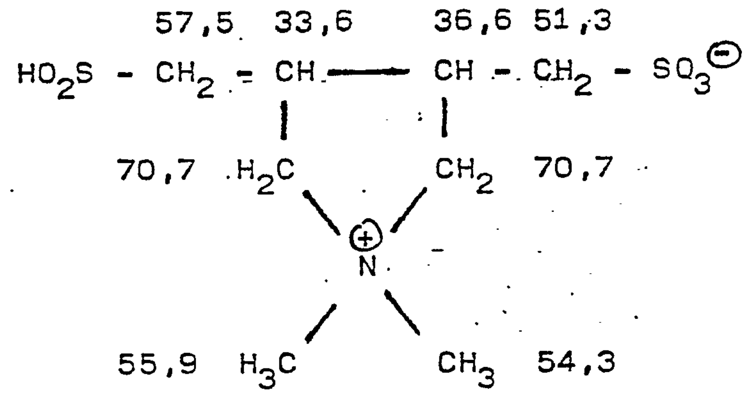

Die Zahlenangaben an den Atomsymbolen entsprechen den chemischen Verschiebungen in ppm.

Während für das Natriumsalz für die Gruppe $NaO_2S-CH_2-$ eine chemische Verschiebung von 61,2 ppm ermittelt wurde (vgl. Beispiel 2), lag der Wert für die Sulfinsäuregruppe $HO_2S-CH_2-$ bei 57,5 ppm.

Durch Neutralisation der Sulfobetainsulfinsäure mit beliebigen Basen können im Bedarfsfalle die jeweiligen Salze formelrein gewonnen werden.

Wiederholt man vorstehenden Versuch bei einer Anfangstemperatur von 0°C, ergibt sich zwar eine Verlängerung der Reaktionszeit, jedoch keine Verminderung der Ausbeute an Sulfobetainsulfinat. Den zeitlichen Verlauf der exothermen Sulfocyclosulfinierung zeigt folgende Übersicht:

| | | | | | | | |
|---|---|---|---|---|---|---|---|
| Zeit (min) | 0 | 1 | 2 | 2,5 | 3 | 3,3 | 5 |
| Temperatur (°C) | 0 | 1,5 | 3 | 25 | 41 | 50,5 | 50,3 |

Nach 2 Minuten färbt sich die zunächst fahlgelbe Reaktionslösung orange und nach 2,5 Minuten blutrot.

**Beispiel 14**

Dieses Beispiel und die beiden folgenden demonstrieren die Initiierung der Sulfocyclosulfinierung des Dimethyldiallylammoniumchlorids mit weiteren Initiatoren, die Sauerstoff, Wasserstoffperoxid und Natriumperborat, und veranschaulichen Unterlegenheit gegenüber dem erfindungsgemäßen Verfahren.

a) Sulfocyclosulfinierung unter Einrühren von Luftsauerstoff bei einem pH-Wert von 2,5
Es wurde wie in Beispiel 12 verfahren, wobei aber kein Ammoniumperoxidsulfat zugesetzt, sondern durch intensives Einrühren von Luft 1 mol Dimethyldiallylammoniumchlorid mit 2,1 mol Natriumhydrogensulfit pro kg Reaktionsmischung bei pH 2,5 zur Umsetzung gebracht wurde. Beginnend mit einer Starttemperatur von 23°C, erwärmte sich die Lösung in 38 Minuten auf eine Temperatur von 56°C (Temperaturmaximum, vgl. Fig. 2), die bei fortgesetztem Rühren allmählich wieder abfiel. Zum Zeitpunkt des Temperaturmaximums war jedoch noch kein vollständiger Umsatz erreicht; erst nach 2 Stunden konnte $^1H$-NMR-spektroskopisch kein Allylammoniumsalz mehr nachgewiesen werden.

b) Sulfocyclosulfinierung unter Durchleiten von Luft bei einem pH-Wert von 4
In einem 100-l-Faß aus Polyethylen wurden 23,05 kg (75 mol) 52,6 %-ige technische Dimethyldiallylammoniumchloridlösung mit 33,13 kg (112,5 mol) 35,33 %-iger technischer Natriumhydrogensulfitlösung gemischt. Der pH-Wert der Lösung betrug 4. Unter heftigem Rühren wurde Luft derart durch die Lösung geleitet, daß darin Luftbläschen fein verteilt waren. Die überschüssige Luft, die die Reaktionsmischung durchperlte, reicherte sich stark mit Schwefeldioxid an und wurde in einen Abzug geleitet. Den zeitlichen Verlauf der exothermen Sulfocyclosulfinierung zeigt folgende Übersicht:

| | | | | | | | | | |
|---|---|---|---|---|---|---|---|---|---|
| Zeit (min) | 0 | 10 | 20 | 30 | 40 | 45 | 50 | 60 | 120 |
| Temperatur (°C) | 15 | 28 | 37 | 63 | 84 | 86 | 85,5 | 84 | 73 |

Nach 2 Stunden enthielt der Ansatz kein Allylammoniumsalz mehr, während Sulfit noch jodometrisch nachweisbar war. Die analytische Untersuchung des Reaktionsproduktes zeigte, daß das Sulfobetainsulfinat nur in einer Ausbeute von ca. 35 % neben dem Sulfobetain vorlag. Bei der analogen Umsetzung bei pH 4 sowie Initiierung mit Kaliumperoxodisulfat konnte das Sulfobetainsulfinat in viel höherer Ausbeute bei kürzerer Reaktionszeit erhalten werden (vgl. Fig. 1 sowie Beispiel 5 in Tab. 1).

**Beispiel 15**

Sulfocyclosulfinierung von Dimethyldiallylammoniumchlorid mit $H_2O_2$

Es wurde wie in Beispiel 12 verfahren, wobei jedoch anstelle von Ammoniumperoxodisulfat 2 mol-% 30 %-iges Wasserstoffperoxid auf einmal zur Reaktionsmischung hinzugefügt wurden.

Die Temperatur der Lösung stieg danach in 15 Sekunden um 2°C an. Eine weitere Temperaturerhöhung wurde nicht beobachtet. Erst nach zweitsündigem Einrühren von Luft (analog Beispiel 14a) erhöhte sich die Temperatur weiter, d. h., erst durch Lufteinwirkung wurde die Reaktion zu Ende geführt.

**Beispiel 16**

Sulfocyclosulfinierung von Dimethyldiallylammoniumchlorid mit Natriumperborat

Man verfuhr wie in Beispiel 15 beschrieben, fügte jedoch anstelle von Wasserstoffperoxid 2 mol-% Natriumperborat zur Reaktionsmischung hinzu. Auch hierbei stieg die Reaktionstemperatur in 30 Sekunden nur um 2,5°C an. Es konnte nur unumgesetztes Ausgangsmaterial im Reaktionsprodukt nachgewiesen werden.

**Beispiel 17**

Sulfocyclosulfinierung von Dimethyl-di-2-methallylammoniumchlorid

Es wurde wie in Beispiel 12 verfahren, wobei die vereinigten Lösungen von 73 g Gesamtmasse aus 5,32 g (30 mmol) Dimethyl-di-2-methallylammoniumchlorid ($^{13}C$-NMR-Spektrum in ppm: 134,9 (C); 129,1 ($CH_2$ =); 72,2 (-$CH_2$-); 51,8; 51,6; 51,5 (N-$CH_3$); 25,0 (C-$CH_3$)); 65 mmol Natriumhydrogensulfit, Salzsäure, Leitungswasser und 5 mol-% Natriumperoxodisulfat bei einem pH-Wert von 2,1 miteinander umgesetzt wurden. Die reagierende Mischung erwärmte sich im Verlaufe der Umsetzung von 22 auf 33°C.

Das $^{13}C$-NMR-Spektrum des erhaltenen, neutralisierten Reaktionsproduktes zeigte, daß es sich nicht um ein einheitliches Produkt handelt, sondern um cis/trans-Isomere von 1,1,3,4-Tetramethyl-3-sulfinatomethyl-4-sulfonatomethyl-pyrrolidiniumbetain:

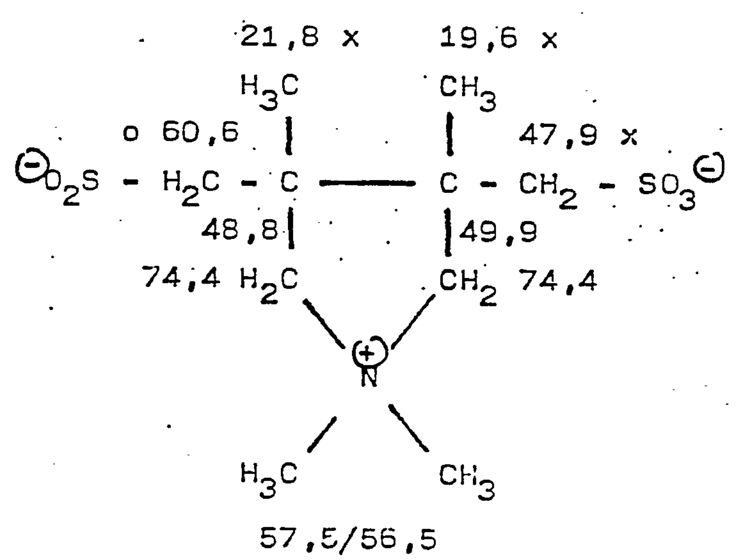


(x; o: Signale können auch vertauscht sein) und 1,1,3,3,4-Pentamethyl-4-sulfonatomethyl-pyrrolidiniumbetain

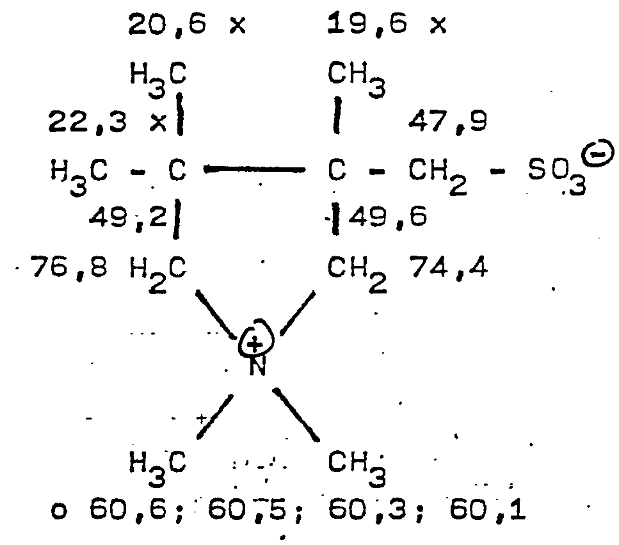

(x; o: Signale können auch vertauscht sein).

Die chemischen Verschiebungen sind nur für die cis-Verbindungen angeführt. Die N-$CH_3$-Gruppen-sind nicht äquivalent.

**Beispiel 18**

Natrium-1-methyl-3-sulfinatomethyl-4-sulfonatomethyl-pyyrolidiniumbetain ($R^1$ = $R^3$ = $R^4$ = H, $R^2$ = $CH_3$ in der allgemeinen Formel Ia) durch Sulfocyclosulfinierung von Methyldiallylaminhydrochlorid.

Es wurde wie in Beispiel 12 verfahren, wobei mit 2 mol-% Kaliumperoxodisulfat in einer Arbeitsmenge von 1 kg bei einem pH-Wert von 2 genau 1 mol Methyldiallylaminhydrochlorid und 2,02 mol Natriumhydrogensulfit miteinander umgesetzt wurden. Das erhaltene Reaktionsprodukt zeigte folgende Ergebnisse der $^{13}C$-NMR-Spektrometrie ($D_2O$, externer Standard TMS):

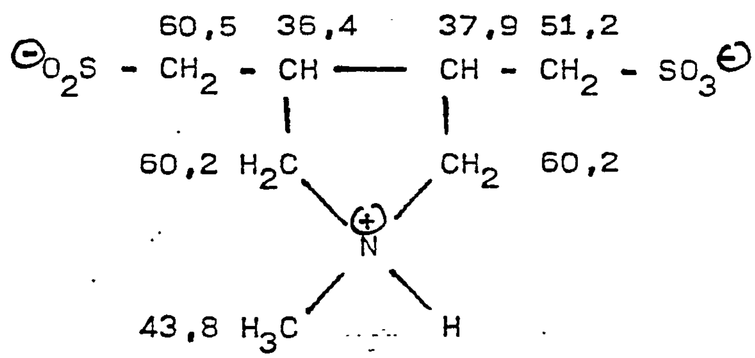

Die Zahlenangaben an den C-Atomsymbolen entsprechen den chemischen Verschiebungen für die cis-Konfiguration in ppm. Falls die freie Sulfinsäure isoliert werden soll, kann man diese nach der im Beispiel 13 beschriebenen Verfahrensweise von den anorganischen Salzen abtrennen. Durch Neutralisation der Sulfinsäure mit einem oder zwei Moläquivalenten einer beliebigen Base können so die jeweiligen Salze, die Sulfobetainsulfinate oder die 1-Methyl-pyrrolidin-3-sulfinate-4-sulfonate, formelrein gewonnen werden.

**Beispiel 19**

Natrium-3-sulfinatomethyl-4-sulfonatomethyl-pyrrolidiniumbetain ($R^1$ = $R^2$ = $R^3$ = $R^4$ = H in der allgemeinen Formel Ia).

Es wurde nach Beispiel 12 verfahren, wobei 2 mol-% Kaliumperoxodisulfat in einer Arbeitsmenge von 1 kg bei einem pH-Wert von 2 genau 1 mol Diallylaminhydrochlorid und 2,02 mol Natriumhydrogensulfit miteinander umgesetzt wurden.

Ergebnisse der $^{13}C$-NMR-Spektrometrie des Reaktionsprodukts ($D_2O$, externer Standard TMS):

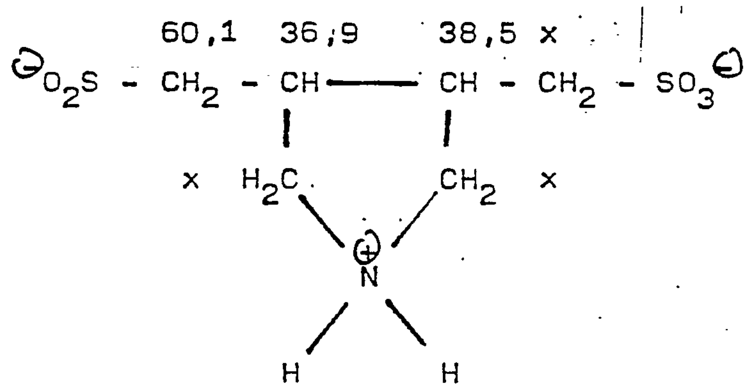

Die Zahlenangaben an den C-Atomsymbolen entsprechen den chemischen Verschiebungen für die cis-Konfiguration in ppm. x: 49,7; 50,2; 50,5.

Falls die freie Sulfinsäure isoliert werden soll, kann man diese nach der im Beispiel 13 beschriebenen Verfahrensweise von den anorganischen Salzen abtrennen.

Durch Neutralisation der Sulfinsäure mit beliebigen Basen können die jeweiligen Salze, die Sulfobetainsulfinate oder die Pyrrolidin-3-sulfinate-4-sulfonate, formelrein gewonnen werden.

**Beispiel 20**

3-Methyl-4-sulfonatomethyl-pyrrolidiniumbetain durch Sulfocyclisierung von Diallylaminhydrochlorid

Es wurde nach Beispiel 11 verfahren, wobei bei einem pH-Wert $\geqslant$ 7,5 Diallylaminhydrochlorid mit gepufferter Natriumhydrogensulfitlösung zur Umsetzung gebracht wurde.

Ergebnisse der $^{13}C$-NMR-Spektrometrie des Reaktionsproduktes ($D_2O$, externer Standard TMS):

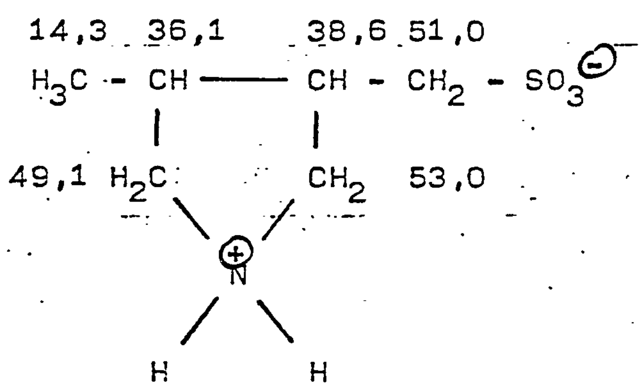

Die Zahlenangaben an den C-Atomsymbolen entsprechen den chemischen Verschiebungen für die cis-Konfiguration in ppm.

**Beispiel 21**

Natrium-1-benzyl-1-methyl-3-sulfinatomethyl-4-sulfonatomethyl-pyrrolidiniumbetain ($R^1$ = $CH_2$-$C_6H_5$, $R^2$ = $CH_3$, = $R^2$ $R_4$ = H in der allgemeinen Formel Ia) durch Sulfocyclosulfinierung von Benzylmethyldiallylammoniumchlorid

Es wurde wie in Beispiel 12 verfahren, wobei mit 2 mol-% Natriumperoxodisulfat in einer Arbeitsmenge von 1 kg bei einem pH-Wert von 2 genau 1 mol Benzylmethyldiallylammoniumchlorid und 2,02 mol Natriumhydrogensulfit miteinander umgesetzt wurden.

Ergebnisse der $^{13}C$-NMR-Spektrometrie des cis-Reaktionsproduktes (Angaben wie oben):

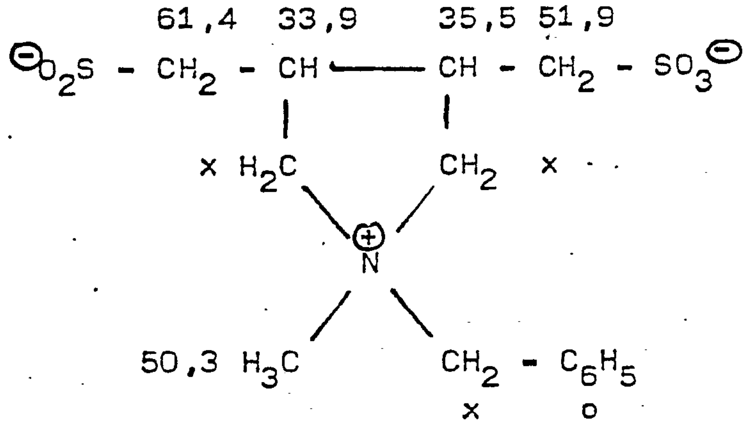

x: 68;5; 68,8; 70,2;
o: 129,6; 130,9;.132,4; 134,1
Die freie Sulfinsäure kann nach der in Beispiel 13 beschriebenen Arbeitsweise rein erhalten werden.

**Beispiele 22 und 23**

Natrium-1-fettalkyl-1-methyl-3-sulfinatomethyl-4-sulfonatomethyl-pyrrolidiniumbetaine ($R^1$ = Fettalkyl, $R^2$ = $CH_3$, R3 = $R^4$ = H in der allgemeinen Formel Ia) durch Sulfocyclosulfinierung von Methylfettalkyldiallylammoniumbromid

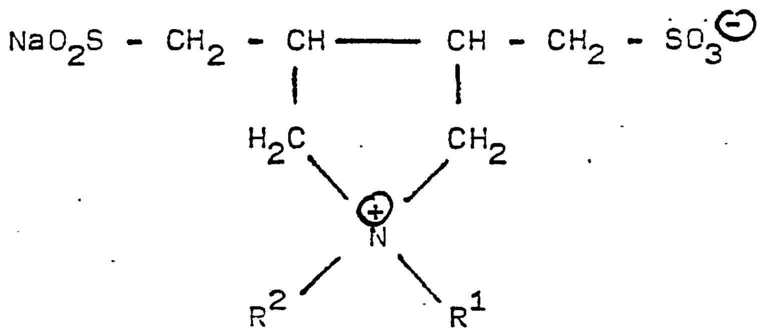

Tab. 2: Natrium-1-fettalkyl-1-methyl-3-sulfinatomethyl-4-sulfomethyl-pyrrolidiniumbetaine

| Beispiel | $R^1$ | $R^2$ | Schmelzpunkt (°C) |
|---|---|---|---|
| 22 | n-$C_{12}H_{25}$ | $CH_3$ | 226°C (Zers.) |
| 23 | n-$C_{16}H_{33}$ | $CH_3$ | 196°C (Zers., als Sulfinsäure) |

Zunächst wurde jeweils 1 mol des kristallinen Methylfettalkyldiallylammoniumbromids in 2,1 mol einer 40,7 %-igen technischen Natriumhydrogensulfitlösung aufgelöst; im Falle des Hexadecylammoniumsalzes mußte die Hydrogensulfitlösung auf etwa 40°C erwärmt werden, damit sich das Salz löste. Anschließend wurde mit konzentrierter Salzsäure der pH-Wert der Lösungen auf 2 eingestellt und die Umsetzung durch Zusatz von 2 mol-% einer 50 %-igen Ammoniumperoxodisulfatlösung initiiert. Unmittelbar nach der Initiatorzugabe färbten sich die reagierenden Lösungen rot und erreichten nach 50 Sekuden die maximale Reaktionstemperatur, wonach die Umsetzung bereits beendet war. Während das Dodecylsulfinat gelöst bleibt, beginnt das Hexadecylsulfinat aus der sich abkühlenden Lösung zu kristallisieren. Die Abtrennung der reinen Sufinate von den anorganischen Salzen kann auch durch Extraktion der eingedampften Reaktionslösungen mit Ethanol erfolgen.

Auch die Gewinnung der freien Sulfinsäuren kann nach der im Beispiel 13 beschriebenen Methode vorgenommen werden. Durch Neutralisation der Sulfinsäuren mit beliebigen Basen können die jeweiligen Salze formelrein gewonnen werden.

**Beispiel 24**

Natrium-1-dodecyl/tetradecylaminocarbonylmethyl-1-methyl-3-sulfinatomethyl-4-sulfonatomethyl-pyrrolidiniumbetain ($R^1 = CH_2CO\text{-}NH\text{-}C_{12}H_{25}/C_{14}H_{29}$) $R^2 = CH_3$; $R^3 = R^4 = H$ in der allgemeinen Formel Ia)

Sulfocyclosulfinierung von N,N-Diallyl-N-methyl-ammonioessigsäuredodecyl/tetradecylamid-chlorid unter Variation der Initiatorkonzentration:

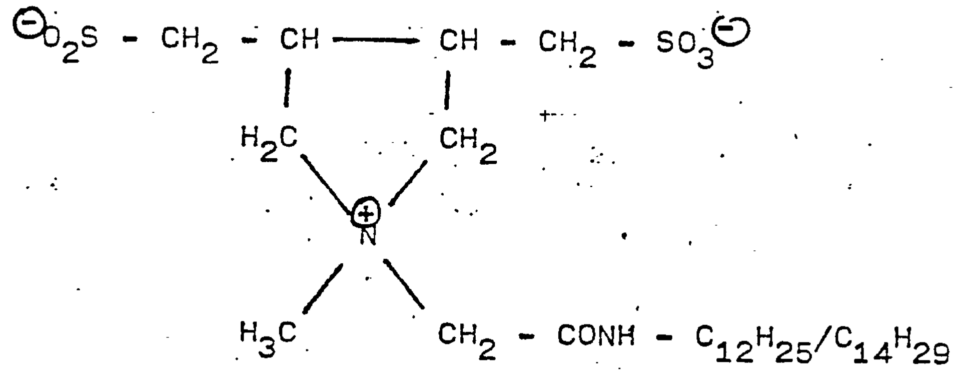

a) Synthese des Ausgangsproduktes N,N-Diallyl-N-methyl-ammonio-essigsäuremethylester-chlorid
1112 g (10 mol) Methyldiallylamin wurden in einem Rührgefäß vorgelegt. Dazu tropfte man bei einer Starttemperatur von 35°C 1085 g (10 mol) Chloressigsäuremethylester. Während des Zutropfens stieg die Temperatur auf 70°C an und mußte dann entweder durch Regulierung der Zutropfgeschwindigkeit oder durch gelegentliches Kühlen im Bereich von 70 bis 80°C gehalten werden. Nach etwa 30 Minuten war der Umsatz vollständig, und man erhielt eine klar durchsichtige, sehr viskose Flüssigkeit.

b) N,N-Diallyl-N-methylammonio-essigsäure-dodecyl/tetradecylamidchlorid
Zu dem vorstehend beschriebenen Methylesterchlorid tropfte man innerhalb von 30 Minuten 1990 g (10 mol) Dodecyl/tetradecylamin-Gemisch (Cocosamin, Komponentenverhältnis 1 : 1) hinzu, so daß die Reaktionstemperatur 80°C nicht überstieg. Das hochviskose Endprodukt hatte einen pH-Wert von 7.

In einer Versuchsserie wurde die Abhängigkeit der Dauer der exothermen Sulfocyclosulfinierung von 1 mol des vorstehend beschriebenen Amidchlorids mit 2,1 mol Natriumhydrogensulfit in 1,1 kg Reaktionsgemisch vom pH-Wert 2,2 untersucht (vgl. Beispiel 12).

Die eingesetzte Initiatormenge wurde zwischen 0 und 7 mol-% Ammoniumperoxodisulfat variiert. Die jeweilige Starttemperatur betrug 40°C. Das Gesamtergebnis veranschaulicht Fig.3. Bei Initiatormengen unter 0,5 mol-% wurden keine oder nur geringe Umsetzungsgrade erreicht, während bei Mengen über 7 mol-% das Reaktionsgemisch unmittelbar nach Initiatorzugabe innerhalb von wenigen Sekunden heftig aufsiedete. Den zeitlichen Verlauf der exothermen Sulfocyclosulfinierung durch Initiierung mit 2 mol-% Ammoniumperoxodisulfat zeigt die folgende Übersicht:

| | | | | | | | | | |
|---|---|---|---|---|---|---|---|---|---|
| Zeit (s) | 0 | 5 | 15 | 25 | 35 | 100 | 140 | 150 | 170 |
| Temperatur (°C) | 40 | 48 | 63 | 67 | 71 | 75 | 78 | 78 | 76 |

Die anfänglich fahlgelbe Suspension färbt sich kurz nach der Initiatorzugabe orange, wird homogen und sieht dann durch eingerührte Luftbläschen milchig weiß aus. Die abgekühlte Reaktionslösung stellt eine homogene klar durchsichtige Lösung von fahlgelber Farbe dar. Beim Verdünnen einer Probe mit Wasser fällt der größere Teil des entstandenen Sulfobetainsulfinats aus. Die entstandene Suspension schäumt beim Durchschütteln sehr stark. Die Abtrennung der reinen Sulfobetainsulfinsäure vom Schmp. 219°C (Zers.) von den anorganischen Salzen kann durch Extraktion der eingedampften sauren Lösung erfolgen.

**Beispiele 25 bis 27**

Natrium-1-alkylaminocarbonylmethyl-1-methyl-3-sulfinatomethyl-4-sulfonatomethyl-pyrrolidiniumbetaine ($R^1 = CH_3$, $R^2 = CH_2\text{-}CO\text{-}$alkylamid, $R^3 = R^4 = H$ in der allgemeinen Formel Ia)

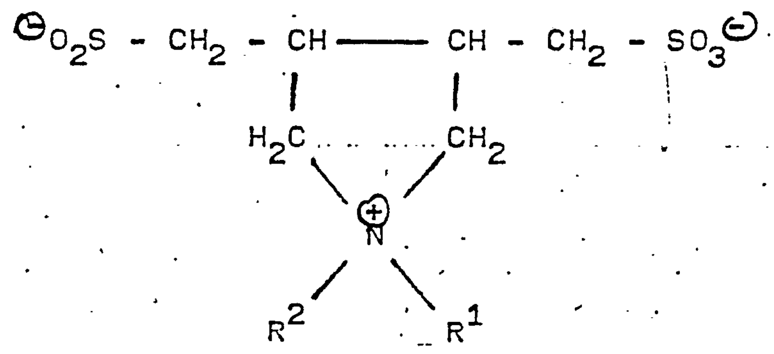

Die Herstellung der Ausgangsprodukte, der N,N-Diallyl-N-methyl-ammonio-essigsäure-alkylamid-chloride, erfolgte nach der in Beispiel 24 beschriebenen Arbeitsweise. Die Sulfocyclosulfinierung zu den Sulfobetainsulfinaten erfolgte nach den Beispielen 22 und 23.

In Tabelle 3 sind die erhaltenen Reaktionsprodukte charakterisiert.

Tab. 3: Natrium-1-alkylaminocarbonylmethyl-1-methyl-3-sulfinatomethyl-4-sulfonatomethyl-pyrrolidiniumbetaine

| Beispiel | $R^1$ | $R^2$ -$CH_2$-CO-Alkylamid | Schmelzpunkt (°C) |
|---|---|---|---|
| 25 | $CH_3$ | -$CH_2CONH$-$C_4H_9$ | 264°C (Zers.) |
| 26 | $CH_3$ | -$CH_2$-CONH-$C_6H_{13}$ | 168°C (Zers., als Sulfinsäure) |
| 27 | $CH_3$ | -$CH_2$-CONH-$C_8H_{17}$ | 218°C (Zers.) |

**Beispiel 28**

Natrium-1-/di-(dodecyl/tetradecyl-aminocarbonyl)/methyl-1-methyl-3-sulfinatomethyl-4-sulfonatometyl-pyrrolidiniumbetain ($R^1$ = CH(CONH-$C_{12}H_{25}$/$C_{14}H_{29}$)$_2$/ $R^2$ = $CH_3$, $R^3$ = $R^4$ = H in der allgemeinen Formel Ia) durch Sulfocyclosulfinierung von N,N-Diallyl-N-methyl-ammonio-malonsäure-didodecyl/tetradecylamid-bromid

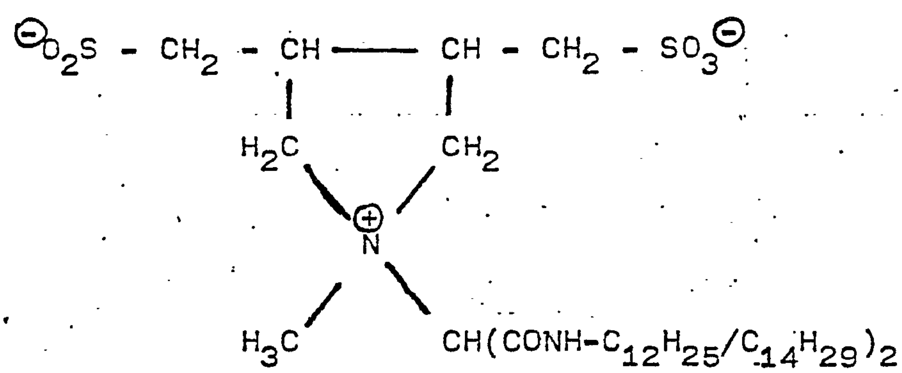

N,N-Diallyl-N-methyl-ammonio-malonsäure-di-dodecyl/tetradecylamid-bromid:

111 g (1 mol) Methyldiallylamin wurden in 200 ml Ethanol gelöst. Zu dieser Lösung tropfte man unter Rühren bei 40°C 239 g (1 mol) Brommalonsäurediethylester und erwärmte danach noch 2 Stunden auf 80°C. Zu dieser Lösung fügte man anschließend 398 g (2 mol) Dodecyl/tetradecylamin-Gemisch (Cocosamin, Komponentenverhältnis 1 : 1) allmählich hinzu.

Zur Vervollständigung der Umsetzung wurde noch 4 Stunden auf 80°C erwärmt. Im Vakuum wurde dann bei 40°C etwa die Hälfte des Lösungsmittels entfernt.

Natrium-1-/di-(dodecyl/tetradecylaminocarbonyl)/methyl-1-methyl-3-sulfinatomethyl-4-sulfomethyl-pyrrolidiniumbetain:

Das verbleibende Rohprodukt wurde mit 400 ml Wasser verdünnt; zur Lösung wuden 630 g einer 40,7 %-igen

Natriumhydrogensulfitlösung gegeben. Mit konzentrierter Salzsäure wurde ein pH-Wert von 2,0 eingestellt. Anschließend wurde das Gemisch auf 50°C erwärmt. Bei Erreichen dieser Temperatur erhielt man eine gelbliche trübe Lösung, die anschließend mit 5,7 g (2,5 mol-%) Ammoniumperoxodisulfat versetzt wurde. Dabei stieg die Temperatur auf 82°C an. Beim Abkühlen fiel das entstandene Sulfobetainsulfinat aus. Es wurde abgetrennt und aus Ethanol umkristallisiert (Schmp. 78°C).

**Beispiel 29**

Natrium-1,1-dimethyl-3,4-disulfonatomethyl-pyrrolidiniumbetain ($R^1$ = $R^2$ = $CH_3$, = $R^4$ = H in der allgemeinen Formel Ib) aus Dimethyldiallylammoniumchlorid.

In einem mit Rührer, Rückflußkühler, Tropftrichter und Thermometer ausgerüsteten Sulfierkolben wurden 349,2 g (1 mol) 46,3 %-ige technische Dimethyldiallylammoniumchloridlösung, 533,6 g (2 mol) 39 %-ige technische Natriumhydrogensulfitlösung mit einem Eisengehalt von 9 mg/mol $NaHSO_3$ sowie 30 g 37 %-ige Salzsäure miteinander zu einer homogenen Lösung vom pH-Wert 2,1 vermischt. Zu der so vorbereiteten fahlgelben Startlösung fügte man eine 40 %-ige wäßrige Natriumperoxodisulfatlösung, die aus 238,1 g (1 mol) Natriumperoxodisulfat und 357,15 g Wasser bereitet worden war, in dem Maße hinzu, daß sich die reagierende Lösung in etwa einer Minute, beginnend bei Raumtemperatur, zum Sieden erwärmte. Während dieser Zeit hatte sich die Lösung rot gefärbt.

Die Zudosierung der Persulfatlösung wurde zügig so fort gesetzt, daß die Reaktionswärme der ab 103°C siedenden Lösung leicht durch Siedekühlung abgeführt werden konnte, was insgesamt 5 Minuten erforderte.

Nachdem man etwa drei Viertel der Peroxodisulfatlösung zudosiert hatte, hellte sich die Reaktionslösung zunehmend auf und war am Ende schließlich gelbgrün. Ein von der Lösung angefertigtes $^1$H-NMR-Spektrum bestätigte die quantitative und selektive Umwandlung des Diallylammoniumsalzes in das Sulfobetainsulfonat. Nach Neutralisation der Lösung mit 33 %-iger Natronlauge flockten die durch Verwendung technischer Chemikalien darin enthaltenen Eisensalze als Eisen-(III)hydroxid aus und konnten zusammen mit dem größten Teil des auskristallisierten Natriumsulfats abfiltriert werden. Ein vom farblosen Filtrat angefertigtes $^{13}$C-NMR-Spektrum zeigte, daß das Sulfobetainsulfonat hauptsächlich in der cis-Konfiguration neben geringen trans-Anteilen vorliegt.

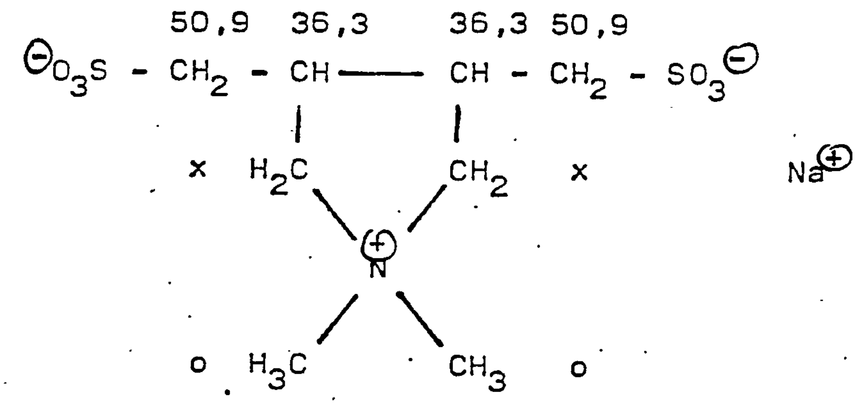

x : 70,8; 70,7; 70,5. Signalaufspaltung durch $^{14}$N-Quadrupol-moment
o : 56;1; 55,9; 55,7/54,5; 54,3; 54,1.
Die $N\text{-}CH_3$-Gruppen sind nicht äquivalent; Signalaufspaltung durch $^{14}$N-Quadrupolmoment
trans-Verbindung: 70,2 ($N\text{-}CH_2$); 53,7 ($N\text{-}CH_3$); 40,2 (CH).

Soll das Sulfobetainsulfonat vollständig von seinen anorganischen Begleitsalzen abgetrennt und das freie 1,1-Dimethyl-3-sulfomethyl-4-sulfonatonethyl-pyrrolidiniumbetain isoliert werden, kann man wie folgt verfahren: Nach Einengen der oben gewonnenen Reaktionslösung zur Trockne versetzt man den erhaltenen Salzrückstand mit der ausreichenden Menge konzentrierter Salzsäure und arbeitet ihn durch, filtriert von den ungelösten Natriumsalzen ab und dampft die salzsaure Lösung der Sulfobetainsulfonsäure unter vermindertem Druck zur Trockne ein. Kurz darauf beginnt die Sulfonsäure zu kristallisieren; durch Zusatz von Ethanol kann die vollständige Kristallisation erreicht werden. Durch Absaugen des Ethanols und Trocknen des Kristallbreis kann die farblose kristalline Sulfobetainsulfonsäure, die sich ab 220°C zersetzt, isoliert werden. Das $^{13}$C-NMR-Spektrum dieser Säure ist mit dem des entsprechenden Natriumsalzes völlig identisch. Durch erneutes Lösen der Sulfonsäure in der äquimolaren Menge Natronlauge und Einengen der erhaltenen Lösung kann das Natriumbetainsulfonat rein und in kristalliner Form gewonnen werden. Durch Neutralisation der Sulfobetainsulfonsäure mit beliebigen Basen können im Bedarfsfalle die jeweiligen Salze formelrein gewonnen werden.

**Beispiel 30**

Dieses Beispiel demonstriert den Einfluß des Start-pH-Wertes auf die Selektivität der ablaufenden Reaktion.

Es wurde wie in Beispiel 29 verfahren, wobei 69,84 g (0,2 mol) 46,3 %-ige Dimethyldiallylammoniumchloridlösung mit 106,7 g (0,2 mol) 39 %-iger Natriumhydrogensulfitlösung zu einer homogenen Lösung vermischt wurden, deren pH-Wert 4,1 war. Dazu tropfte man nun im Verlaufe von 4 Minuten 119,05 g(0,2 mol) einer 40 %-igen Natriumperoxodisulfatlösung. Bereits nach 2 Minuten siedete die reagienende Mischung, deren Wärmeüberschuß leicht durch Siedekühlung abgeführt werden konnte. Die folgende Übersicht zeigt den zeitlichen Verlauf der exothermen Reaktion während der Zudosierungsphase des Oxidationsmittels.

| Zeit (min) | 0 | 1 | 1,5 | 2 | 3 | 4 |
|---|---|---|---|---|---|---|
| Temperatur (°C) | 22 | 94 | 99 | 103 | 103 | 100 |

Die abgekühlte farblose Reaktionslösung vom pH-Wert 0,3 wurde mit 33 %-iger Natronlauge neutralisiert und vom auskristallisierten Natriumsulfat und dem ausgefallenen Eisen(III)-hydroxid abfiltriert. Die quantitative Zusammensetzung des Filtrats wurde durch Vergleich der Intensitäten geeigneter Signale $^1H$-NMR-spektroskopisch bestimmt und ergab, daß neben 90 % 1,1-Dimethyl-3,4-disulfonatomethyl-pyrrolidiniumbetain 10 % 1,1,3-Trimethyl-4-sulfonatomethyl-pyrrolidiniumbetain enthalten waren (vgl. DD-A-154 444).

**Beispiel 31**

Dieses und das folgende Beispiel 32 veranschaulichen die Eignung von kombinierten Oxidationsmitteln.

Es wurde nach Beispiel 29 verfahren, wobei aus Dimethyldiallylammoniumchloridlösung, Natriumhydrogensulfitlösung und Salzsäure eine homogene Startlösung vom pH-Wert 2,1 hergestellt wurde, der zunächst in etwa 1,5 Minuten 10 mol-% einer 50,0 %-igen Ammoniumperoxodisulfatlösung und anschließend 90 mol-% 30 %-iges Wasserstoffperoxid in einer solchen Geschwindigkeit hinzugefügt wurden, daß die exotherme Reaktion durch Siedekühlung beherrscht werd konnte. $^1H$-NMR-spektroskopisch konnte nachgewiesen werden, daß das Diallylammoniumsalz selektiv und quantitativ zum Sulfobetainsulfonat umgewandelt worden war. Die Wiederholung dieses Versuches ausschließlich mit Wasserstoffperoxid als Oxidationsmittel führte neben unverändertem Ausgangsmaterial und seinem Polymeren lediglich zu einer Oxidation des Sulfits zu Sulfat.

**Beispiel 32**

Es wurde nach Beispiel 31 verfahren, wobei anstelle von 90 mol-% Wasserstoffperoxid als Oxidationsmittel Chlor eingesetzt wurde, indem man Chlor so in die reagierende Mischung einleitete, daß die Reaktionswärme gut abgeführt werden konnte. $^1H$-NMR-spektroskopisch konnte nur das Sulfobetainsulfonat nachgewiesen werden. Die Wiederholung des Versuches ausschließlich mit Chlor als Oxidationsmittel führte unter Sulfitoxidation zum Sulfat.

**Beispiel 33**

Natrium-1,1,3,4-tetramethyl-3,4-disulfonatomethyl-pyrrolidiniumbetain aus Dimethyl-di-2-methallylammoniumchlorid

($R^1 = R^2 = R^3 = R^4 = CH_3$ in der allgemeinen Formel Ib).

Es wurde wie in Beispiel 29 verfahren, wobei anstelle von Dimethyldiallylammoniumchlorid 10,64 g (30 mmol) 50 %-ige wäßrige Dimethyl-di-2-methallylammoniumchloridlösung, 65 mmol 39 %-ige Natriumhydrogensulfitlösung und Salzsäure als homogene Lösung vom Start-pH-Wert 2,1 mit 65 mmol 40 %-iger Natriumperoxodisulfatlösung miteinander umgesetzt wurden. Das $^{13}C$-NMR-Spektrum des erhaltenen, neutralisierten und weitgehend vom Natriumsulfat abgetrennten Reaktionsproduktes zeigte daß es sich nicht um ein einheitliches Produkt handelte, sondern um cis/trans-Isomere von 1,1,3,4-Tetramethyl-3,4-disulfonatomethyl-pyrrolidiniumbetain und 1,1,3,3,4-Pentamethyl-4-sulfonatomethyl-pyrrolidinium-betain.

Die chemischen Verschiebungen sind nur für die cis-Verbindungen angeführt. Die N-$CH_3$-Gruppen sind nicht äquivalent.

x: Signale können auch vertauscht sein.

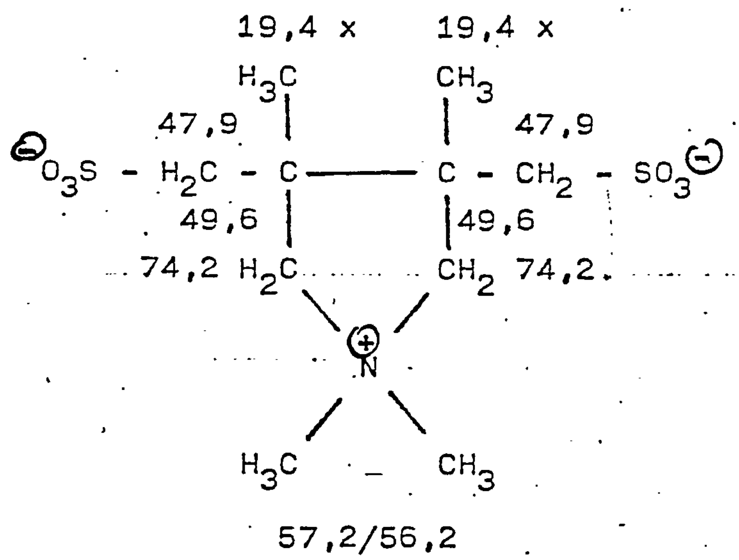

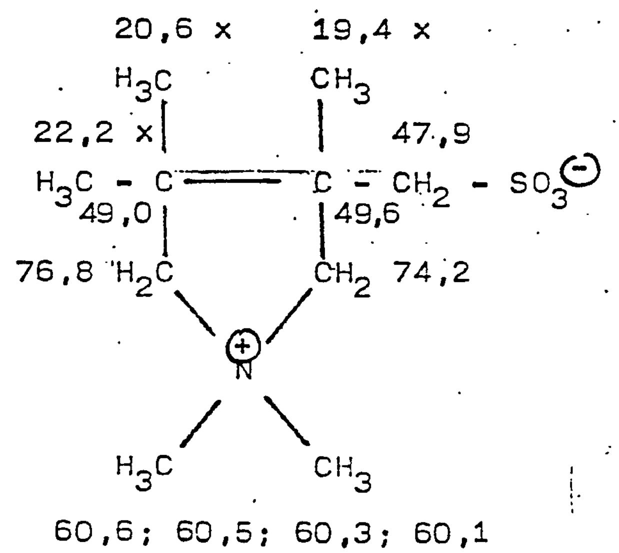

**Beispiel 34**

Kalium-1-methyl-3,4-disulfonatomethyl-pyrrolidiniumbetain
$R^1 = R^3 = R^4 = H$, $R^2 = CH_3$ in der allgemeinen Formel Ib) aus Methyldiallylammoniumchlorid.
Es wurde eine homogene Lösung vom pH-Wert 2,0 aus 1 mol Methyldiallylamin, 1 mol Kaliummetabisulfit ($K_2S_2O_5$) und 37 %-iger Salzsäure hergestellt, worauf nach dem vorstehend beschriebenen Beispiel 29 verfahren wurde.
Das Sulfobetainsulfonat wurde quantitativ erhalten.
Ergebnisse der $^{13}C$-NMR-Spektrometrie:

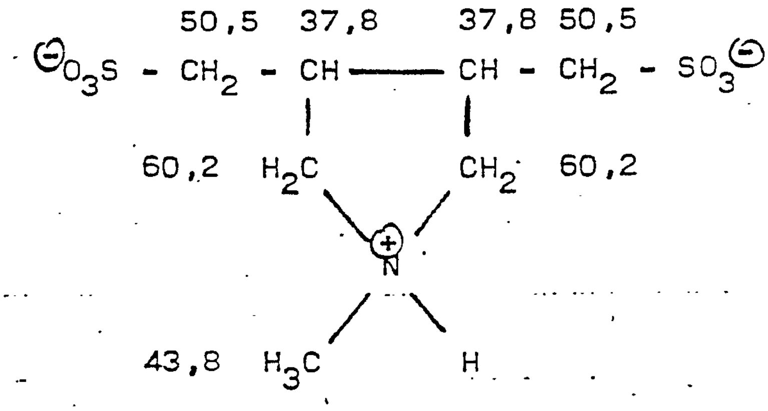

Falls die freie Sulfonsäure gewonnen werden soll, kann man diese nach der in Beispiel 29 beschriebenen Arbeitsweise von den anorganischen Salzen abtrennen und isolieren. Durch Neutralisation der Sulfonsäure mit einem oder zwei Moläquivalenten einer beliebigen Base können so die jeweiligen Salze des 1-Methyl-3,4-disulfonatomethyl-pyrrolidiniumbetains oder des 1-Methyl-3,4-disulfonatomethyl-pyrrolidins-formelrein gewonnen werden.

**Beispiel 35**

Natrium-3,4-disulfonatomethyl-pyrrolidiniumbetain
($R^1 = R^2 = R^3 = R^4 = H$ in der allgemeinen Formel Ib).
Es wurde eine homogene Lösung vom pH-Wert 2,0 aus 1 mol Diallylamin, 2 mol Natriumhydrogensulfit und Salzsäure hergestellt, worauf nach einem der vorstehend beschriebenen Beispiele verfahren wurde. Das Sulfobetainsulfonat wurde in quantitativer Ausbeute erhalten. Ergebnisse der $^{13}C$-NMR-Spektrometrie (cis-Konfiguration):

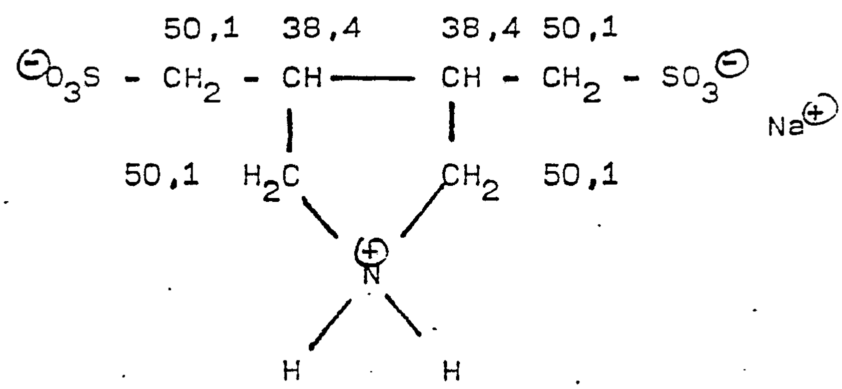

Während die chemischen Verschiebungen der Gruppen $N\text{-}CH_2$ und $CH_2\text{-}SO_3$ für die cis-Verbindung bei 50,1 ppm zusammenfallen, zeigt die trans-Verbindung erwartungsgemäß 3 Signale: 40,4 ppm (CH); 51,0 ppm ($CH_2\text{-}SO_3^-$); 53,6 ppm ($N\text{-}CH_2$). Falls die freie Sulfonsäure gewonnen werden soll, kann mann diese nach der in Beispiel 29 beschriebenen Arbeitsweise von den anorganischen Salzen abtrennen und isolieren. Durch Neutralisation der Sulfobetainsulfonsäure mit einem oder zwei Moläquivalenten einer beliebigen Base können so die jeweiligen Salze des 3,4-Disulfonatomethyl-pyrrolidiniumbetains oder des 3,4-Disulfonatomethylpyrrolidins formelrein gewonnen werden.
Ergebnisse der $^{13}C$-NMR-Spektrometrie des cis-Dinatrium-3,4-disulfonatomethyl-pyrrolidins:

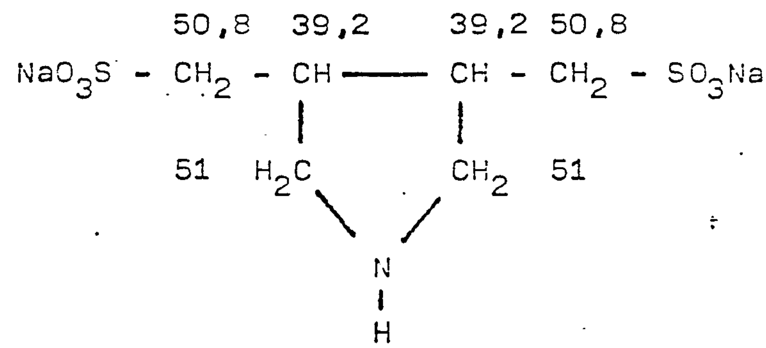

trans-Konfiguration: 42,1 ppm (CH); 52 ppm ($CH_2SO_3Na$); 55 ppm ($N\text{-}CH_2$).

**Beispiel 36**

Natrium-1-benzyl-1-methyl-3,4-disulfonatomethyl-pyrrolidiniumbetain ($R^1$ = $CH_2\text{-}C_6H_5$, $R^2$ = $CH_3$, $R^3$ = $R^4$ = H in der allgemeinen Formel Ib) aus Benzylmethyldiallylammoniumchlorid,

Es wurde eine homogene Lösung vom pH-Wert 2,0 aus 1 mol 50 %-iger Benzylmethyldiallylammoniumchlorid-Lösung, 2 mol Natriumhydrogensulfit und Salzsäure hergestellt worauf nach einem der vorstehend beschriebenen Beispiele werfahren wurde. Das Sulfobetainsulfonat wurde in quantitativer Ausbeute erhalten.

Ergebnisse der $^{13}C$-NMR-Spektrometrie:

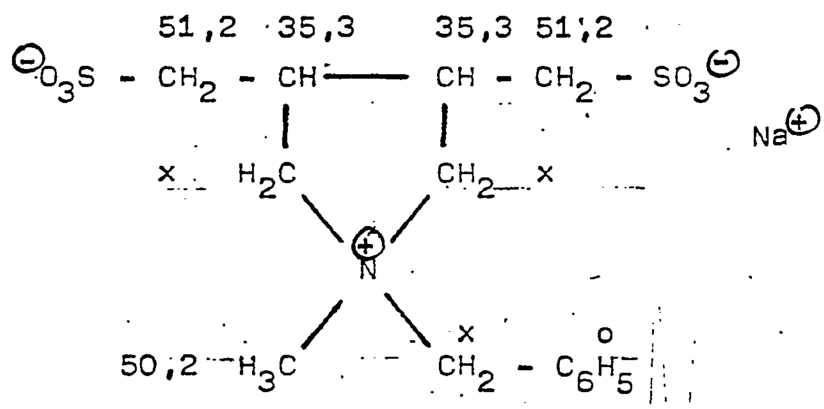

x: 68,3; 68,6; 70,2;

o: 129,5; 130,9; 132,3; 134.

Im Bedarfsfalle kann die freie Sulfobetainsulfonsäure nach der in Beispiel 29 beschriebenen Verfahrensweise gewonnen werden.

**Beispiel 37**

Natrium-1-propyl-1-methyl-3,4-disulfonatomethyl-pyrrolidiniumbetain ($R^1$ = $-CH_2\text{-}CH_2\text{-}CH_3$, $R^2$ = $CH_3$, $R^3$ = $R^4$ = H in der allgemeinen Formel Ib).

Es wurde nach Beispiel 29 verfahren, wobei 23,4 g (0,1 mol) Propylmethyldiallylammoniumbromid, 53,4 g (0,2 mol) 39 %-ige Natriumhydrogensulfitlösung, 4,5 g 37 %-ige Salzsäure und 47 g Wasser zu einer homogenen Lösung vermischt werden, deren pH-Wert 2,0 war. Zu dieser Lösung fügte man im Verlaufe von 7 Minuten feingepulvertes kristallines Kaliumperoxodisulfat portionsweise hinzu. Die anfänglich rötliche Reaktionslösung entfärbte sich gegen Ende der Persulfatzugabe vollständig. Der exotherme Reaktionsverlauf während der Zudosierungsphase des Oxydationsmittels ist folgender Übersicht zu entnehmen:

| Zeit (min) | 0 | 1 | 2 | 3 | 5 | 6 | 7 |
|---|---|---|---|---|---|---|---|
| Temperatur (°C) | 21 | 32 | 48 | 61 | 88 | 92 | 94 |

Das $^1$H-NMR-Spektrum des neutralisierten farblosen Filtrates zeigte, daß das Sulfobetainsulfonat in quantitativer Ausbeute erhalten worden war. Ergebnisse der $^{13}C$-NMR-Spektrometrie:

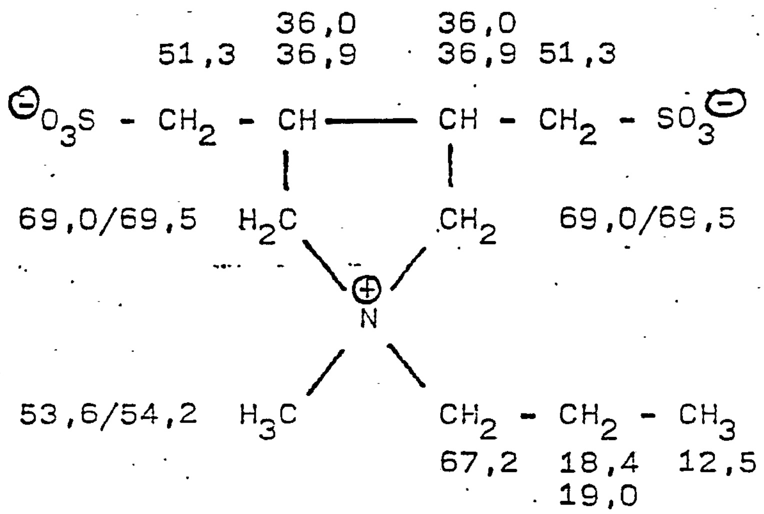


Die Verdopplung der Signale bei den chemischen Verschiebungen einzelner Kohlenstoffatome weist darauf hin, daß das erhaltene Sulfobetainsulfonat ein Produktgemisch aus den beiden möglichen cis-Isomeren in bezug auf die Sulfonatomethylgruppen in den Positionen 3 und 4 darstellt.

**Beispiele 38 bis 42**

Natrium-1-alkyli-1-methyl-3,4-disulfonatomethyl-pyrrolidiniumbetain ($R^1$ = n-Alkyl, $R^2$ = $CH_3$, $R^3$ = $R^4$ = H in der allgemeinen Formel Ib) aus Alkylmethyldiallylammoniumsalzen

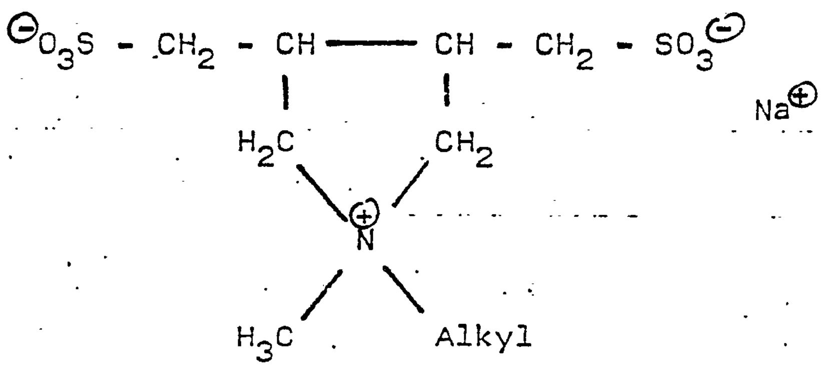

Es wurde nach einem der vorstehend beschriebenen Beispiele verfahren, wobei eine homogene Lösung vom pH-Wert 2,0 bis 2,5 aus 1 mol Alkylmethyldiallylammoniumsalz, 2 mol eines Hydrogensulfits, Salzsäure und Wasser hergestellt wurde. Im Falle der längerkettigen Alkylmethyldiallylammoniumsalze (Alkyl > $C_{14}$) mußte die anfängliche Suspension auf etwa 40°C erwärmt werden, damit eine homogene Startlösung entstand sowie eine zu große Verdünnung vermieden wurde. Nach erfolgter Umsetzung mit 1 mol eines Peroxodisulfats oder einer Peroxodisulfat-Oxidationsmittel-Kombination fielen insbesondere die längerkettigen Reaktionsprodukte (Alkyl > $C_{12}$) schon während der Umsetzung oder aus der sich abkühlenden Lösung aus. Je nachdem, ob die Reaktionsmischung vorher neutralisiert wurde, konnten entweder die freie Sulfobetainsulfonsäure oder die entsprechenden Sulfobetainsulfonate abgetrennt werden.

Im Falle der kürzerkettigen Sulfobetainsulfonate trennt man diese von den anorganischen Begleitsalzen entweder nach der im Beispiel 29 beschriebenen Methode oder durch Extraktion des Rückstandes mit Ethanol/Wasser (Verhältnis 70 : 30) nach Eindampfen der Lösung.

Tab. 4: Natrium-1-alkyl-1-methyl-3,4-disulfomethyl-pyrrolidiniumbetaine

| Beispiel | $R^1$ (n-Alkyl) | Zersetzungspunkt (°C) |
|---|---|---|
| 38 | $C_{10}$-$H_{21}$ | 225 |
| 39 | $C_{12}H_{25}$ | 215 |
| 40 | $C_{14}H_{29}$ | 261+) |
| 41 | $C_{16}H_{33}$ | 220 |
| 42 | $C_{18}H_{37}$ | 228 |

+) freie Sulfonsäure; sie beginnt sich teilweise ab 228°C zu zersetzen; bei 261°C schmilzt sie unter vollständiger Zersetzung.

**Beispiel 43**

Natrium-1-butylaminocarbonylmethyl-1-methyl-3,4-disulfonatomethyl-pyrrolidiniumbetain ($R^1$ = $CH_2$-CO-NH-$C_4H_9$, $R^2$ = $CH_3$, $R^3$ = $R^4$ = H in der allgemeinen Formel Ib).

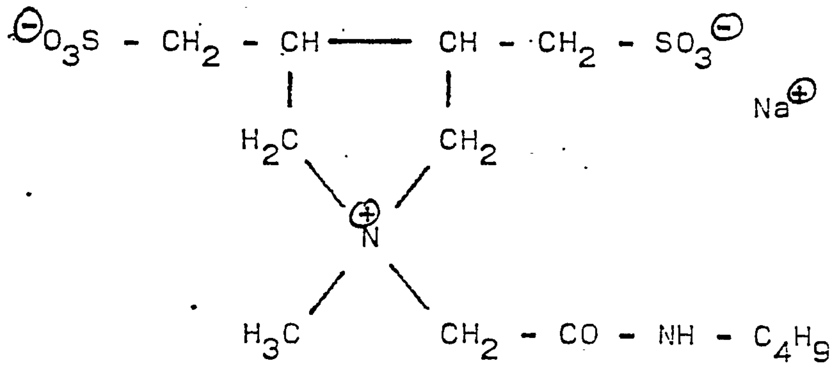

Es wurde nach Beispiel 31 verfahren, wobei die homogene Lösung vom pH-Wert 2,0 bis 2,5 aus 1 mol n-Butylaminocarbonylmethyl-methyldiallylammoniumchlorid, das aus Methyldiallylamin, Chloressigsäuremethylester und n-Butylamin hergestellt war, 2 mol Natriumhydrogensulfit, Salzsäure und Wasser mit Ammoniumperoxodisulfat/Wasserstoffperoxid unter Anwendung äußerer Kühlung so umgesetzt daß eine Reaktionstemperatur von ungefähr 50°C nicht überschritten wurde. Nach erfolgter Umsetzung wurde sofort mit Natronlauge neutralisiert, um die Hydrolyse der Säureamidfunktion zu vermeiden. Will man jedoch analog Beispiel 29 nur mit Peroxodisulfat umsetzen, sollte, nachdem etwa 10 mol-% der Peroxodisulfatlösung zum Diallylammoniumsalz zudosiert worden sind, gleichzeitig mindestens die zweifache molare Menge Natronlauge mit der restlichen Menge Peroxodisulfatlösung unter Einhaltung einer Temperatur, die nicht höher als 50°C sein sollte, hinzugefügt werden.

Das Sulfobetainsulfonat (Zersetzungspunkt > 238°C) wurde durch Extraktion des nach Eindämpfen der Reaktionslösung erhaltenen Rückstandes mit wäßrigem Ethanol (60 %-ig) von seinen anorganischen Begleitsalzen abgetrennt.

**Beispiel 44**

Natrium-1-dicyclohexylaminocarbonylmethyl-1-methyl-3,4-disulfonatomethyl-pyrrolidiniumbetain ($R^1$ = $CH_2$-CO-N$(C_6H_{11})_2$, $R^2$ = $CH_3$, $R^3$ = $R^4$ = H in der allgemeinen Formel Ib)

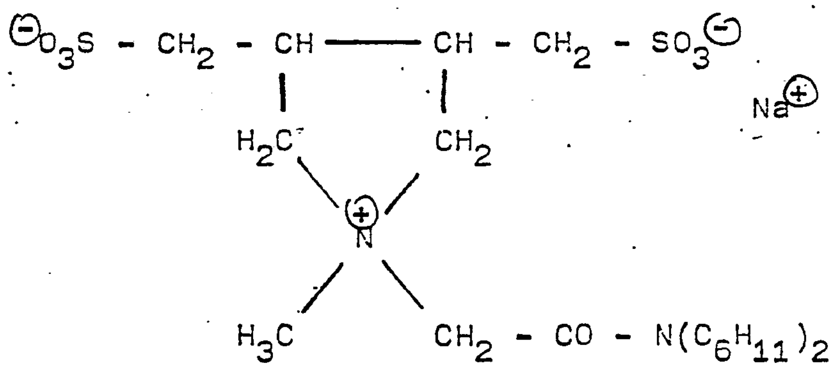

Es wurde nach Beispiel 43 verfahren, wobei Dicyclohexylaminocarbonylmethyl-methyldiallylammoniumchlorid, das aus Methyldiallylamin, Chloressigsäuremethylester und Dicyclohexylamin hergestellt war, quantitativ zum Sulfobetainsulfonat (Zersetzungspunkt > 270° C) umgesetzt wurde.

**Beispiel 45**

Natrium-1-dodecyl/tetradecylaminocarbonylmethyl-1-methyl-3,4-disulfonatomethyl-pyrrolidiniumbetain ($R^1 = CH_2\text{-}CO\text{-}NH\text{-}C_{12}H_{25}/C_{14}H_{29}$, $R^2 = CH_3$, $R^3 = R^4 = H$) in der allgemeinen Formel Ib)

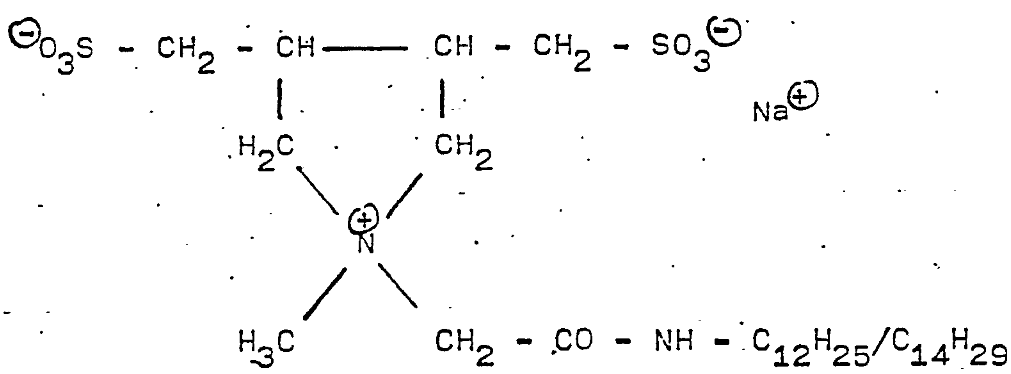

Es wurde nach Beispiel 43 verfahren, wobei Dodecyl/tetradecylaminocarbonylmethyl-methyldiallylammoniumchlorid, das aus Methyldiallylamin, Chloressigsäuremethylester und Dodecyl/tetradecylamin-Gemisch (Cocosamin, Komponentenverhältnis etwa 1 : 1) hergestellt war, quantitativ zum Sulfobetainsulfonat (Zersetzungspunkt > 246° C) umgesetzt wurde.

**Beispiel 46**

Natrium-1-/di-(dodecyl/tetradecylaminocarbonyl)/methyl-1-methyl-3,4-disulfonatomethyl-pyrrolidiniumbetain
($R^1 = CH(CO\text{-}NH\text{-}C_{12}H_{25}/C_{14}H_{29})_2$, $R^2 = CH_3$, $R^3 = R^4 = H$ in der allgemeinen Formel Ib)

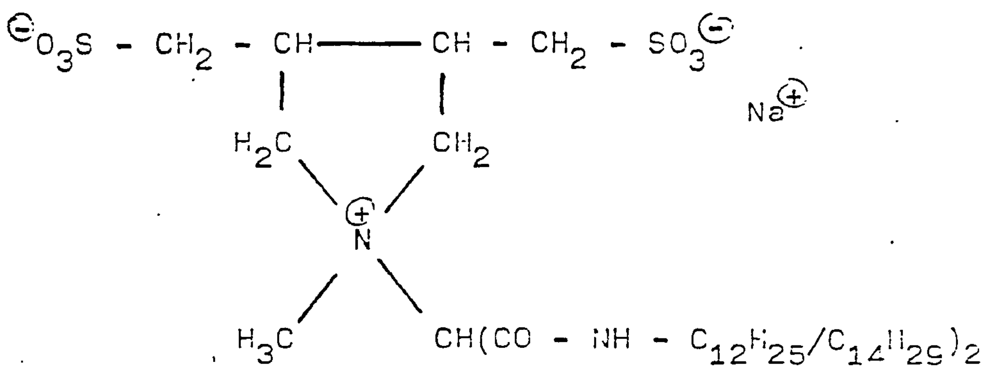

Es wurde nach Beispiel 42 verfahren, wobei Di-(dodecyl/tetradecylaminocarbonyl)methyl-methyldiallylammoniumbromid, das man aus Methyldiallylamin, Brommalonsäurediethylester und Dodecyl/tetradecylamin-Gemisch (Cocosamin, Komponentenverhältnis 1 : 1) hergestellt war, bei einer Reaktionstemperatur von 40°C zum Sulfobetainsulfonat (Schmelzpunkt 139 bis 148°C, Umkristallisation aus Ethanol) umgesetzt wurde.

**Beispiel 47**

Sulfocyclosulfinierung von Triallylaminhydrochlorid zum Trinatrium-1-(2'-sulfinato-3'-sulfonatopropyl-3-sulfinato-methyl-4-sulfonatomethyl-pyrrolidiniumbetain

($R^1 = -CH_2-CH_2-CH_2-SO_3^-, R^2 = R^3 = R^4 = H$
|
$SO_2^-$

in der allgemeinen Formel Ia).

In einem mit Rührer, Thermometer und Glaselektrode versehenen Sulfierkolben wurden zu einer Mischung von 12 g 37 %-iger Salzsäure und 10 g Wasser 13,7 g (0,1 mol) frisch destilliertes Triallylamin unter Kühlung hinzugefügt. Danach wurde die salzsaure Triallylaminhydrochloridlösung mit 108,7 g (0,425 mol) 40,7 %-iger technischer Natriumhydrogensulfitlösung mit einem Eisengehalt von 60 mg/l versetzt. Die homogene fahlgelbe Startlösung hatte einen pH-Wert von 2,1. Nun fügte man unter Rühren auf einmal 1,08 g (4 mol-%) feingepulvertes Kaliumperoxodisulfat zu, wobei sich das Peroxodisulfat sofort auflöste die reagierende Mischung sich erwärmte und blutrot färbte. Den zeitlichen Verlauf der exothermen Sulfocyclosulfinierung zeigt folgende Übersicht:

| | | | | | |
|---|---|---|---|---|---|
| Zeit (s) | 0 | 20 | 30 | 40 | 60 |
| Temp. (°C) | 23 | 35 | 63 | 68 | 67 |

Nach Erreichen des Temperaturmaximums ist die Umsetzung beendet. Die Rotfärbung der Reaktionsmischung ist auf den Einsatz eisenhaltiger technischer Chemikalien durch Bildung von Eisen(III)-sulfinat zurückzuführen. Durch Zusatz geeigneter Komplexbildner kann die Lösung entfärbt werden. Zur Isolierung der freien Säure 1-(2'-Sulfino-3'-sulfopropyl-3-sulfinomethyl-4-sulfonatomethyl-pyrrolidiniumbetain verfährt man wie folgt:

Die Reaktionslösung wird im Vakuum unter Austreiben von $SO_2$ eingeengt, mit wenig Natronlauge auf pH 7 gebracht; dann werden einige Tropfen Wasserstoffperoxidlösung zugefügt und das ausgefällte Eisen(III)-hydroxid abfiltriert.

Nach Einengen des Filtrats zur Trockne wird das Produkt in konzentrierter Salzsäure aufgenommen und das Natriumchlorid durch Filtration abgetrennt. Einengen der salzsauren Lösung und anschließende Zugabe von Ethanol liefert die freie Sulfinsäure.

Ergebnisse der $^{13}C$-NMR-Spektrometrie ($D_2O$, externer Standard TMS):

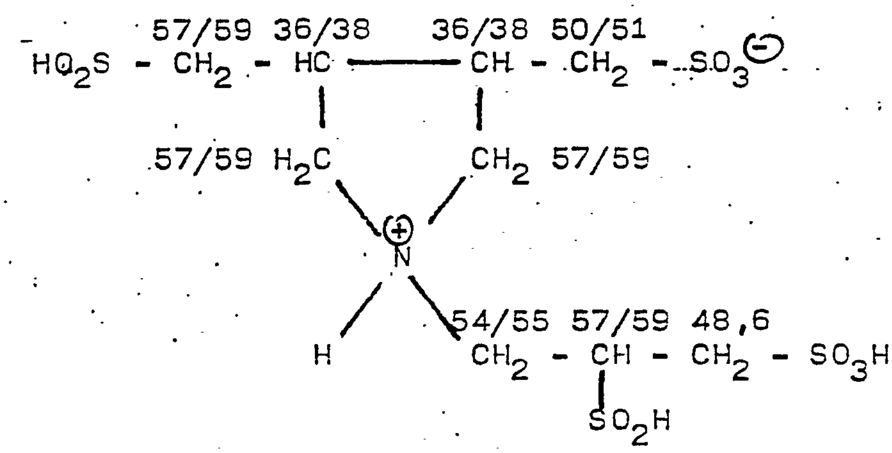

Die Zahlenangaben an den Atomsymbolen entsprechen den chemischen Verschiebungen in ppm. Eine exakte Signalzuordnung wurde nicht getroffen, da die Sulfobetainsulfon-disulfinsäure als cis/trans-Isomerengemisch (vier Isomere) vorlag.

**Beispiel 48**

Sulfocyclosulfinierung von N,N,N-Triallyl-ammonio-essigsäuredodecyl/tetradecylamid-chlorid zum Trinatrium-1-dodecyl/tetradecylaminocarbonylmethyl-1-(2'-sulfinato-3'-sulfonatopropyl)-3-sulfinatomethyl-4-sulfonatomethyl-pyrrolidiniumbetain ($R^1$ =

$-CH_2-CH-CH_2-SO_3^-$,
$\quad\quad\;|$
$\quad\quad SO_2^-$

$R^2 = -CH_2-CO-NH-C_{12}H_{25}/C_{14}H_{29}$, $R^3 = R^4 = H$ in der allgemeinen Formel Ia).

Synthese des Ausgangsproduktes N,N,N-Triallyl-ammonio-essigsäuremethylester-chlorid:

13,7 g (0,1 mol) frisch destilliertes Triallylamin und 10,85 g (0,1 mol) säurefreier Chloressigsäuremethylester wurden in 30 ml Methanol so lange zum Sieden erhitzt, bis der pH-Wert der Reaktionslösung auf 7 abgefallen war.

N,N,N-Triallyl-ammonio-essigsäure-dodecyl/tetradecylamidchlorid.

Zu dem vorstehend beschriebenen Methylesterchlorid wurde eine Lösung von 19,9 g (0,1 mol) Dodecyl/tetradecylamin-Gemisch (Cocosamin, Komponentenverhältnis 1 : 1) und 100 ml Methanol hinzugefügt, worauf die Lösung bis zum Erreichen eines pH-Wertes um 7 erneut erhitzt wurde. Anschließend wurde das Methanol unter vermindertem Druck abdestilliert und der verbleibende Rückstand so zur Sulfocyclosulfinierung eingesetzt.

Es wurde dazu wie in Beispiel 47 verfahren, wobei 0,1 mol des oben hergestellten Dodecyl/tetradecylamidchlorids in 0,42 mol einer 40,7 %-igen technischen Natriumhydrogensulfitlösung und 80 g Wasser aufgenommen und mit konzentrierter Salzsäure auf pH 2,0 eingestellt wurden. Zu der so erhaltenen milchigweißen Startlösung von 32°C fügte man auf einmal 4 mol-% einer 50 %-igen Ammoniumperoxodisulfatlösung unter Rühren hinzu. Bereits nach 15 Sekunden begann ein Teil des Umsetzungsproduktes aus der reagierenden Lösung auszufallen.

Den gesamten zeitlichen Verlauf der exothermen Sulfocyclosulfinierung zeigt folgende Übersicht:

| Zeit (s). | 0 | 15 | 35 | 60 | 80 | 120 |
|---|---|---|---|---|---|---|
| Temperatur (°C) | 32 | 35 | 42 | 49 | 50 | 49 |

Nach erfolgter Umsetzung wurde das Reaktionsgemisch mit Natronlauge neutralisiert, und bei Abkühlung kristallisierte das Sulfobetaindisulfinat-sulfonat als beigefarbene Masse, die sich leicht von der Mutterlauge abtrennen ließ, nahezu quantitativ aus.

Ein völlig farbloses, kristallines sowie von anorganischen Salzen und Eisen(III)-hydroxid freies Produkt vom Schmelzpunkt > 216°C (Zers.) erhält man, wenn das neutralisierte Rohprodukt aus Ethanol umkristallisiert wird.

Die freie Sulfobetain-disulfinsäure-sulfonsäure kann leicht kristallin isoliert werden, wenn man zur heiß gesättigten Lösung des Trinatriumsalzes die äquivalente Menge konzentrierte Salzsäure hinzufügt und abkühlen läßt.

**Beispiel 49**

Sulfocyclosulfinierung von N,N,N-Triallyl-ammonio-essigsäureoctadecylamid-chlorid zum Trinatrium-1-octadecyl-aminocarbonylmethyl-1-(2'-sulfinato-3'-sulfonatopropyl-3-sulfinatomethyl-4-sulfonatomethyl-pyrrolidiniumbetain

($R^1$ = $-CH_2-CH(SO_2^-)-CH_2-SO_3^-$, $R^2$ = $CH_2-CO-NH-C_{18}H_{37}$)

(SO₂⁻ steht am mittleren CH)

$R^3$ = $R^4$ = H in der allgemeinen Formel Ia).

Es wurde nach Beispiel 47 verfahren, wobei 0,1 mol N,N,N-Triallyl-ammonio-essigsäure-octadecylamid-chlorid (hergestellt aus Triallylamin, Chloressigsäuremethylester und Octadecylamin nach der in Beispiel 48 angegebenen Vorschrift) in 108,7 g (0,425 mol) 40,7 %-iger technischer Natriumhydrogensulfitlösung und 140 g Wasser unter Erwärmen aufgenommen wurden.

Dann wurde der pH-Wert mit 37 %-iger Salzsäure auf 2,0 eingestellt, worauf die Startlösung von 49°C auf einmal mit 4 mol-% einer 30 %-igen Natriumperoxodisulfatlösung versetzt wurde. Den zeitlichen Temperaturverlauf der Sulfocyclosulfinierungsreaktion zeigt die folgende Übersicht:

| Zeit (s). | 0 | 15 | 30 | 80 | 150 | 180 |
|---|---|---|---|---|---|---|
| Temperatur (°C) | 49 | 59 | 62,5 | 63 | 63 | 62,5 |

Noch während der Umsetzung begann sich das Endprodukt aus der Reaktionslösung als beigefarbene Masse abzuscheiden und konnte nach Abkühlen quantitativ von der Mutterlauge abgetrennt werden. Die weitere Reinigung des Sulfinats bzw. die Isolierung der freien Sulfobetain-disulfinsäure-sulfonsäure kann analog den in Beispiel 48 beschriebenen Methoden erfolgen. Schmelzpunkt der Sulfobetain-disulfinsäure-sulfonsäure (Ethanol) 98°C.

**Beispiele 50 bis 52**

Sulfocyclosulfinierung von Alkyltriallylammoniumbromid zu Trinatrium-1-alkyl-1-(2'-sulfinato-3'-sulfonatopropyl)-3-sulfinatomethyl-4-sulfonatomethyl-pyrrolidiniumbetain

($R^1$ = $-CH_2-CH(SO_2^-)-CH_2-SO_3^-$, $R^2$ = Alkyl, $R^3$ =

$R^4$ = H in der allgemeinen Formel Ia).

Es wurde nach einem der vorstehend beschriebenen Beispiele verfahren, wobei die vereinigten Lösungen vom pH-Wert 2,0 aus dem jeweiligen Alkyltriallylammoniumbromid und Natriumhydrogensulfit mit Ammonium- oder Alkaliperoxodisulfat umgesetzt wurden.

Nach erfolgter Sulfocyclosulfinierung beginnt insbesondere die Octadecyl-sulfobetain-disulfinsäure-sulfonsäure aus der sich abkühlenden Reaktionslösung zu kristallisieren und kann so leicht von der Mutterlauge getrennt und im Bedarfsfalle nach den vorstehend beschriebenen Methoden weiter gereinigt werden. Die kürzerkettigen Sulfocyclosulfinierungsproduke werden mit 33 %-iger Natronlauge neutralisiert und lassen sich im Bedarfsfalle von den anorganischen Salzen durch Extraktion ihrer eingedampften Lösung mit Ethanol/Wasser (2 : 1) abtrennen.

| Beispiel | $R^2$ (Alkyl) | Schmelzpunkt (°C) |
|---|---|---|
| 50 | $C_8H_{17}$ | > 244 (Zers.) |
| 51 | $C_{12}H_{25}$ | > 226 (Zers.) |
| 52 | $C_{18}H_{37}$ | 118+) |

+) als Sulfobetain-disulfinsäure-sulfonsäure

**Beispiel 53**

Sulfocyclosulfinierung von Tetraallylammoniumbromid

Es wurde wie in Beispiel 47 verfahren, wobei eine homogene Lösung (pH-Wert 2,3) von 25,8 g (0,1 mol) kristallinem Tetraallylammoniumbromid, 25,8 g Wasser und 112 g (0,42 mol) einer 39 %-igen technischen Natriumhydrogensulfitlösung sowie 4,5 g 37 %-ige Salzsäure mit 1,08 g (4 mol-%) feingepulvertem Kaliumperoxodisulfat umgesetzt wurden. Den zeitlichen Verlauf der exothermen Sulfocyclosulfinierung zeigt folgende Übersicht:

| | | | | | | |
|---|---|---|---|---|---|---|
| Zeit (s) | 0 | 15 | 30 | 40 | 60 | 120 |
| Temperatur (°C) | 28 | 29 | 41 | 71 | 72 | 71 |

Nach beendeter Umsetzung hatte sich der pH-Wert auf 2,0 erniedrigt. Zur Entfernung von überschüssigem Schwefeldioxid werden unter vermindertem Druck 1 bis 2 ml Wasser ab destilliert, worauf der pH-Wert der Lösung mit 33 %-iger Natronlauge auf 7 eingestellt wurde.

Vom Trinatriumsalz der isomeren Spiro-sulfobetain-disulfinsulfonsäuren wurden $^{13}C$-NMR-spektrometrie folgende Ergebnisse erhalten ($D_2O$, externer Standard TMS):

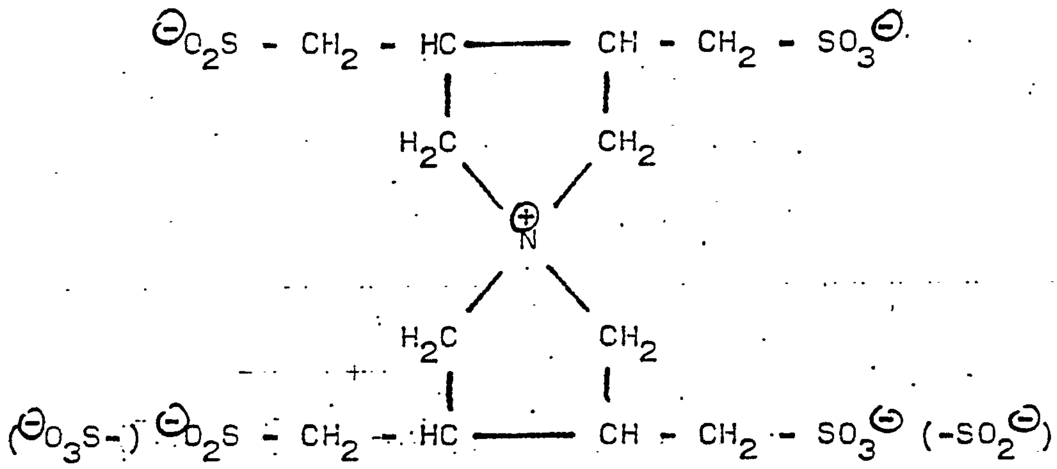

Die Zahlenangaben entsprechen den chemischen Verschiebungen in ppm (in Klammern die Angaben für die trans-verknüpften Isomeren).

| | |
|---|---|
| -CH | : 35,0; 35,2; 36,7; 36,7 (38,6; 40,7) |
| $-CH_2SO_3^-$ | : 51,8; 51,8 |
| $-CH_2SO_2^-$ | : 61,3; 61,5 |
| $-CH_2N^+$ | : 69,6; 69,9; 69,9; 70,8 |

Die chemischen Verschiebungen für die einzelnen Gruppen treten mehrfach auf, da auch hier für cis-(Sulfonatomethyl- bzw. Sulfinatomethylgruppen)-Verbindungen zwei Diastereomere möglich sind.

**Beispiel 54**

Trinatrium-1-methyl-1-(2',3'-disulfonatopropyl)-3,4-disulfonatomethyl-pyrrolidiniumbetain ($R^1$ =
$-CH_2-CH-CH_2-SO_3^-$,
    |
    $SO_3$

$R^2 = CH_3$, $R^3 = R^4$ = H in der allgemeinen Formel Ib) aus Methyltriallylammoniumchlorid.

In einem mit Rührer, Rückflußkühler, Tropftrichter und Thermometer ausgerüsteten Sulfierkolben wurden 238 g (0,5 mol) 39,43 %-ige wäßrige Methyltriallylammoniumchloridlösung, 533,6 g (2 mol) 39 %-ige technische wäßrige Natriumhydrogensulfitlösung mit einem Eisengehalt von 9 mg/mol $NaHSO_3$ sowie 35 g 37 %-ige Salzsäure miteinander zu einer homogenen Lösung vermischt, deren pH-Wert 2,0 (Glaselektrode) betrug. Zur so vorbereiteten fahlgelben Startlösung wurde eine 40 %-ige wäßrige Natriumperoxodisulfatlösung, die aus 238,1 g (1 mol) und 357,15 g Wasser bereitet war, zügig hinzu gefügt. Nach 1,5 Minuten waren etwa 50 % dieser Lösung zudosiert; die inzwischen blutrot gefärbte Mischung hatte sich von 21 auf 100°C erwärmt und begann zu sieden. Man setzte die Zugabe des Oxidationsmittels fort, wobei sich die Reaktionslösung zunehmend

aufhellte und schließlich gelb wurde, nachdem etwa 70 % der Peroxodisulfatlösung hinzugefügt waren. Nach 4 Minuten war die Zudosierung der Peroxodisulfatlösung beendet, wobei die Reaktionswärme leicht durch Siedekühlung abgeführt werden konnte.

Die folgende Übersicht zeigt den zeitlichen Verlauf der exothermen Reaktion während der Zudosierungsphase des Oxidationsmittels:

| Zeit (min) | 0 | 0,5 | 1 | 1,5 | 3 | 4 |
|---|---|---|---|---|---|---|
| Temperatur (°C) | 21 | 65 | 80 | 100 | 102 | 98 |

Ein zu diesem Zeitpunkt von der Lösung angefertigtes $^1H$-NMR- Spektrum bestätigte die quantitative und selektive Umwandlung des Methyltriallylammoniumchlorids in das Sulfobetaintrisulfonat. Nach Neutralisation der stark sauren Reaktionslösung mit 33 %-iger Natronläuge flockte das durch Verwendung von technischer Natriumhydrogensulfitlösung darin enthaltene Eisensalz als Eisen(III)-hydroxid aus und konnte zusammen mit dem größten Teil des auskristallisierten Natriumsulfats abfiltriert werden. Das farblose Filtrat zeigte $^{13}C$-NMR-spektrometrisch folgende Ergebnisse:

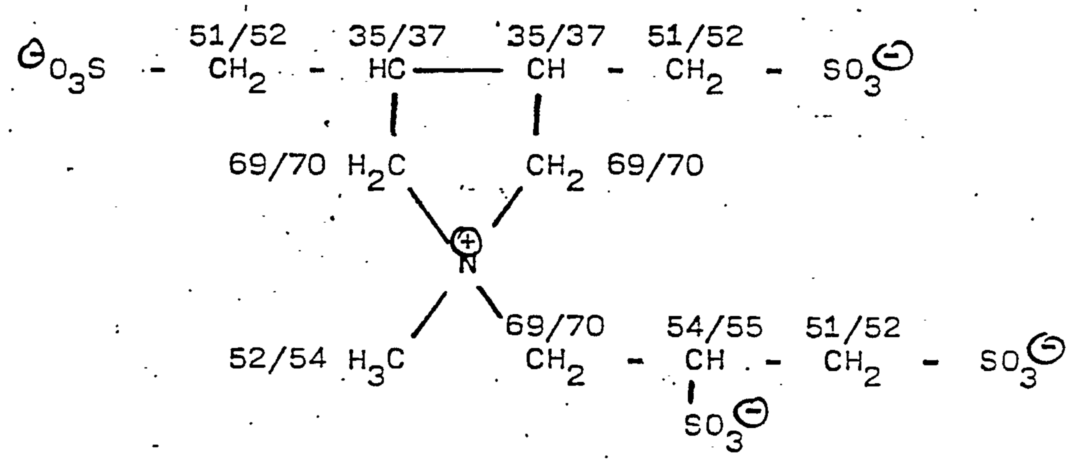

Eine exakte Signalzuordnung wurde nicht getroffen, da die Sulfobetain-trisulfonsäure als cis/trans-Isomerengemisch (drei Isomere) vorlag.

Wenn der pH-Wert der homogenen Startlösung auf Werte > 2,5 eingestellt wird, nimmt die Selektivität der ablaufenden Reaktion ab, wobei in zunehmendem Maße neben 1-Methyl-1-(2',3'-di-2-sulfonatopropyl)-3,4-disulfonatomethylpyrrolidiniumbetain auch 1,3-Dimethyl-1-(3'-sulfonatopropyl)-4-sulfonatomethyl-pyrrolidiniumbetain (vgl. DD-A-200 739, Beispiel 2) gebildet wird, welches schließlich zum Hauptprodukt der Umsetzung werden kann.

## Beispiel 55

1-(2',3'-Disulfopropyl)-3-sulfomethyl-4-sulfonatomethyl-pyrrolidiniumbetain ($R^1$ =
$-CH_2-CH-CH_2-SO_3^-$,
   |
   $SO_3^-$

$R^2 = R^2 = R^4 = H$ in der allgemeinen Formel Ib) aus Triallylaminhydrochlorid.

Es wurde wie in Beispiel 54 verfahren, wobei 13,72 g (0,1 mol) Triallylamin, 16 g Wasser, 16 g 37 %-ige Salzsäure sowie 106,8 g (0,4 mol) 39 %-ige Natriumhydrogensulfitlösung zu einer homogenen Lösung vermischt wurden, deren pH-Wert 2,0 war. Zu dieser Lösung wurde im Verlaufe von 2,5 Minuten 0,2 mol einer 40 %- igen Natriumperoxodisulfatlösung hinzu getropft. Die folgende Übersicht verdeutlicht den zeitlichen Verlauf der exothermen Reaktion während der Zudosierungsphase des Oxidationsmittels:

| Zeit (min) | 0 | 0,5 | 1 | 1,5 | 2,5 |
|---|---|---|---|---|---|
| Temperatur (°C) | 22 | 70 | 90 | 104 | 104 |

Die anfänglich fahlgelbe Lösung wurde bald nach der Peroxodisulfatzugabe blutrot, hellte sich dann allmählich auf und war zum Schluß klar gelb. Die Umsetzung war zu diesem Zeitpunkt quantitativ und selektiv

zum Sulfobetaintrisulfonat erfolgt, wie $^1H$-NMR-spektroskopisch nachgewiesen werden konnte.

Soll jedoch die Sulfobetaintrisulfonsäure vollständig von ihren anorganischen Begleitsalzen abgetrennt werden, kann man wie folgt verfahren:

Nach Einengen der oben gewonnenen Reaktionslösung zur Trockne versetzt man den erhaltenen Salzrückstand mit der ausreichenden Menge konzentrierter Salzsäure und arbeitet ihn durch, filtriert von den ungelösten Natriumsalzen ab und dampft die salzsaure Lösung der Sulfobetaintrisulfonsäure unter vermindertem Druck ein. Durch Zusatz von Ethanol zum verbliebenen Rückstand kann die Sulfonsäure ausgefällt werden.

Ergebnisse der $^{13}C$-NMR-spektrometrie:

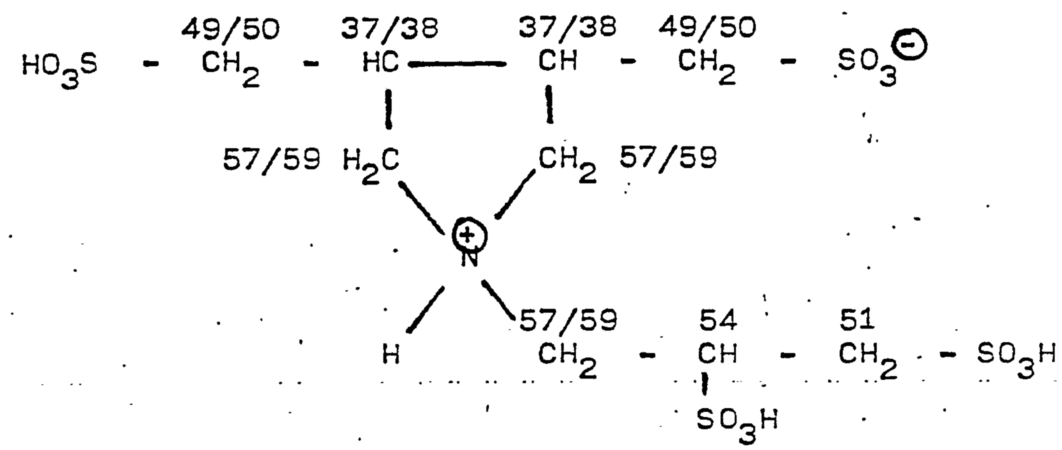

Eine exakte Signalzuordnung wurde nicht getroffen, da die Sulfobetaintrisulfonsäure als cis/trans-Isomerengemisch (drei Isomere) vorliegt.

**Beispiel 56**

Dieses Beispiel veranschaulicht die Eignung von Oxidationsmittel-Kombinationen:

Es wurde nach Beispiel 55 verfahren, wobei aus 0,1 mol Triallyamin 0,4 mol Natriumhydrogensulfit, Salzsäure und Wasser eine homogene Startlösung vom pH-Wert 2,0 hergestellt wurde. Zunächst fügt man in etwa 1,5 Minuten 15 mol-% einer 50 %-igen Ammoniumperoxodisulfatlösung und anschließend 85 mol-% 30 %-iges Wasserstoffperoxid in einer solchen Geschwindigkeit hinzu, daß die exotherme Reaktion durch Siedekühlung beherrscht werden konnte, wofür 3,5 Minuten benötigt wurden. $^1H$-NMR-spektroskopisch konnte nachgewiesen werden, daß das Triallylaminhydrochlorid quantitativ zum Sulfobetaintrisulfonat umgewandelt wurde.

Die Wiederholung dieses Versuchs ausschließlich mit Wasserstoffperoxid als Oxidationsmittel führte neben unverändertem Ausgangsmaterial und Polymeranteilen lediglich zu einer Oxidation des Sulfits zu Sulfat.

**Beispiel 57**

Trinatrium-1-octyl-1-(2',3'-disulfonatopropyl)-3,4-disulfonatomethyl-pyrrolidiniumbetain ($R^1$ =
$-CH_2-CH-CH_2-SO_3^-$,
$\quad\quad\quad\; |$
$\quad\quad\; SO_3^-$

$R^2 = C_8H_{17}$, $R^3 = R^4 = H$ in der allgemeinen Formel Ib) aus Octyltriallylammoniumbromid.

Es wurde nach einem der vorstehend beschriebenen Beispiele verfahren, wobei eine homogene Lösung vom pH-Wert 2,0 aus 1 mol Octyltriallylammoniumbromid, 4 mol eines Hydrogensulfits, Salzsäure und Wasser mit 2 mol einer 50 %-igen Ammoniumperoxodisulfatlösung umgesetzt wurde.

Da sich die freie Sulfobetaintrisulfonsäure nicht von der Reaktionslösung abtrennte, wurde die Lösung mit 33 %-iger Natlonlauge neutralisiert, filtriert und zur Trockne eingeengt und das Sulfobetaintrisulfonat mit dem Zersetzungspunkt > 264°C mit einem Ethanol/Wasser-Gemisch (1 : 1) von den anorganischen Salzen abgetrennt.

**Beispiel 58**

1-Tetradecyl-1-(2',3'-disulfonatopropyl)-3-sulfomethyl-4-sulfonatomethyl-pyrrolidiniumbetain ($R^1$ =
$-CH_2-CH-CH_2-SO_3^-$,
|
$SO_3^-$

$R^2 = C_{14}H_{29}$, $R^3 = R^4 = H$ in der allgemeinen Formel Ib) aus Tetradecyltriallylammoniumbromid.

Es wurde nach einem der vorstehend beschriebenen Beispiele verfahren, wobei 1 mol Tetradecyltriallyammoniumbromid mit Natriumperoxodisulfat umgesetzt wurde. Noch während der Umsetzung begann sich die Sulfobetaintrisulfonsäure aus der stark sauren Reaktionslösung in gelblich braunen Flocken auszuscheiden.

Nach Umkristallisation (Ethanol) wurde die Sulfobetaintrisulfonsäure mit einem Schmelzpunkt von 162°C (Zers.) erhalten.

**Beispiel 59**

Trinatrium-1-dodecyl/tetradecylaminocarbonylmethyl-1-(2',3'-disulfonatopropyl)-3,4-disulfonatomethyl-pyrrolidiniumbetain

($R^1$ = $-CH_2-CH-CH_2-SO_3^-$, $R^2 = CH_2-CO-NH-C_{12}H_{24}/C_{14}H_{29}$,
|
$SO_3^-$

$R^3 = R^4 = H$ in der allgemeinen Formel Ib).

Es wurde nach einem der vorstehend beschriebenen Beispiele verfahren, wobei eine homogene Lösung vom pH-Wert 2,0 aus 1 mol Dodecyl/tetradecylaminocarbonylmethyl-triallylammoniumchlorid, das aus Triallylamin, Chloressigsäuremethylester und Dodecyl/tetradecylamin-Gemisch (Cocosamin, Komponentenverhältnis etwa 1 : 1) hergestellt war, sowie 4 mol Natriumhydrogensulfit und Salzsäure mit Natriumperoxodisulfat/Wasserstoffperoxid unter Anwendung äußerer Kühlung so umgesetzt wurden, daß die Reaktionstemperatur nicht über 60°C stieg. Nach erfolgter Umsetzung wurde sofort mit 33 %-iger Natronlauge neutralisiert, um eine Hydrolyse der Säureamidfunktion zu vermeiden.

Will man jedoch nach Beispiel 54 nur mit Peroxodisulfat umsetzen sollte, nachdem etwa 10 mol-% der Peroxodisulfatlösung zum Triallylammoniumsalz hinzugesetzt worden sind, gleichzeitig mit dem restlichen Peroxodisulfat mindestens die zweifache molare Menge Natronlauge unter Einhaltung einer Temperatur um 60°C hinzugefügt werden.

Das in quantitativer Ausbeute erhaltene Sulfobetaintrisulfonat wurde nach Einengung der Reaktionslösung bis zur Trockne mit einem Ethanol/Wasser-Gemisch (2 : 1) von den anorganischen Salzen abgetrennt und umkristallisiert. Der Zersetzungspunkt des Trinatriumsalzes liegt bei > 252°C.

**Beispiel 60**

Trinatrium-1-octadecylaminocarbonylmethyl-1-(2',3'-disufonatopropyl)-3,4-disulfonatomethyl-pyrrolidiniumbetain

($R^1$ = $-CH_2-CH-CH_2-SO_3^-$, $R^2 = CH_2-CO-NH-C_{18}H_{37}$,
|
$SO_3^-$

$R^3 = R^4 = H$ in der allgemeinen Formel Ib).

Es wurde nach Beispiel 59 verfahren, wobei Octadecylaminocarbonylmethyltriallylammoniumchlorid, das aus Triallylamin, Chloressigsäuremethylester und Octadecylamin hergestellt war, quantitativ zum Sulfobetaintrisulfonat umgesetzt wurde. Das Trinatriumsalz wurde durch ein Ethanol/Wasser-Gemisch (2 : 1) aus der zur Trockne eingeengten Reaktionsmischung von den anorganischen Salzen abgetrennt. Es zersetzt sichbei > 248°C.

**Beispiel 61**

Trinatrium-1,1-(2',3'-disulfonatomethyl)-tetramethylen-3,4-disulfomethyl-pyrrolidiniumbetain aus Tetraallylammoniumbromid.

Es wurde wie in Beispiel 54 verfahren, wobei eine homogene Lösung vom Start-pH-Wert 2,05 aus 25,8 g (0,1 mol) kristallinem Tetraallylammoniumbromid, 25,8 g Wasser, 8,3 g 37 %-iger Salzsäure und 106,8 g (0,4 mol) 39 %-iger Natriumhydrogensulfitlösung mit 119,05 g (0,2 mol) 40 %-iger Natriumperoxodisulfatlösung in 4,5 Minuten umgesetzt wurde. Den zeitlichen Verlauf der exothermen Reaktion während der Zudosierungsphase des Peroxodisulfats zeigt die folgende Übersicht:

| Zeit (min). | 0 | 1 | 2 | 3 | 4 | 4,5 |
|---|---|---|---|---|---|---|
| Temperatur (°C) | 26 | 82 | 96 | 101 | 101 | 100 |

Zu diesem Zeitpunkt war das Tetraallylammoniumbromid bereits quantitativ zum Zielprodukt umgesetzt worden, wie anhand des $^1H$-NMR-Spektrums festgestellt werden konnte.

Nachdem die Reaktionslösung mit Natronlauge neutralisiert sowie im Kühlschrank aufbewahrt und danach vom ausgeflockten Eisen(III)-hydroxid und auskristallisierten Natriumsulfathydraten abgetrennt worden war, erhielt man nach Abdestillieren des Wassers vom Filtrat ein farbloses Kristallpulver.

Ergebnisse der $^{13}C$-NMR-Spektrometrie:

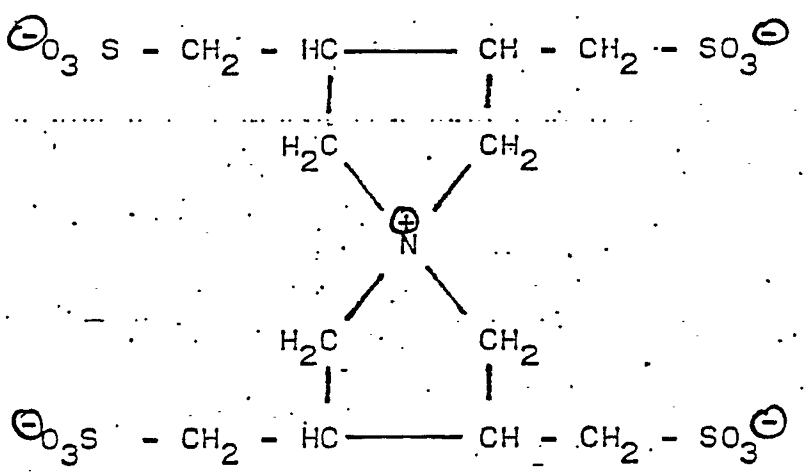

| | |
|---|---|
| -CH | : 36,4; (39,8) |
| $-CH_2-SO_3^-$ | : 50,8; (53,6) |
| $-CH_2-N+$ | : 69,7; 59,2; (70;6) |

Die Zahlenangaben in Klammern entsprechen den chemischen Verschiebungen für die trans-konfigurierten Isomeren.

**Beispiel 62**

Dinatrium-1-methyl-1-(3'-sulfonatopropyl)-3-sulfinatomethyl-4-sulfonatomethyl-pyrrolidiniumbetain ($R^1$ = $-CH_2-CH_2-CH_2-SO_3^-$, $R^2$ = $CH_3$, $R^3$ = $R^4$ = H in der allgemeinen Formel Ia) durch Sulfocyclosulfinierung von Methyltriallylammoniumchlorid.

In einem mit Rührer, Thermometer und Glaselektrode versehenen Sulfierkolben wurden 95,2 g (0,2 mol) 39,43 %-ige wäßrige Methyltriallylammoniumchloridlösung und 162,8 g (0,61 mol) 39 %-ige technische wäßrige Natriumhydrogensulfitlösung mit einem Eisengehalt von 9 mg/mol $NaHSO_3$ miteinander zu einer homogenen Lösung vom pH-Wert 4,3 gemischt. Nun fügte man unter Rühren zu der fahlgelben Startlösung auf einmal 1,9 g (4 mol-%) Natriumperoxodisulfat hinzu, wobei sich das Peroxodisulfat sofort auflöste und die reagierende Mischung sich erwärmte und orange färbte.

Den zeitlichen Verlauf der exothermen Sulfocyclosulfinierung zeigt folgende Übersicht:

| Zeit (s) | 0 | 15 | 25 | 40 | 60 |
|---|---|---|---|---|---|
| Temperatur (°C) | 23 | 42 | 61 | 64 | 63 |

Nach Erreichen des Temperaturmaximums war die Umsetzung beendet. Das $^1H$-NMR-Spektrum der Reaktionslösung wies die quantitative Sulfocyclosulfinierung des Methyltriallylammoniumchlorids aus. Das Sulfobetain-sulfinat-sulfonat wurde neben dem Sulfobetain-disulfinat-sulfonat und dem Sufobetain-sulfonat in etwa 50 bis 60 %-iger Ausbeute erhalten.

Zur Abtrennung der freien Sulfobetain-sulfinsäure-sulfonsäure wurde wie folgt verfahren:

Nach Einengen der oben gewonnenen Reaktionslösung unter vermindertem Druck zur Trockne wurde der erhaltene Salzrückstand mit der ausreichenden Menge konz. Salzsäure versetzt, durchgearbeitet, vom unlöslichen Natriumchlorid abfiltriert und erneut unter vermindertem Druck eingeengt. Durch Zusatz von Alkohol zum verbliebenen öligen Rückstand konnte das Sulfocyclosulfinierungsprodukt ausgefällt werden.

Das $^{13}C$-NMR-Spektrum des salzsauren Rückstandes zeigt, daß die chemischen Verschiebungen (ppm) einzelner Signale der Kohlenstoffatome Verdopplungen aufweisen, was das Vorliegen der folgenden Strukturisomeren beweist:

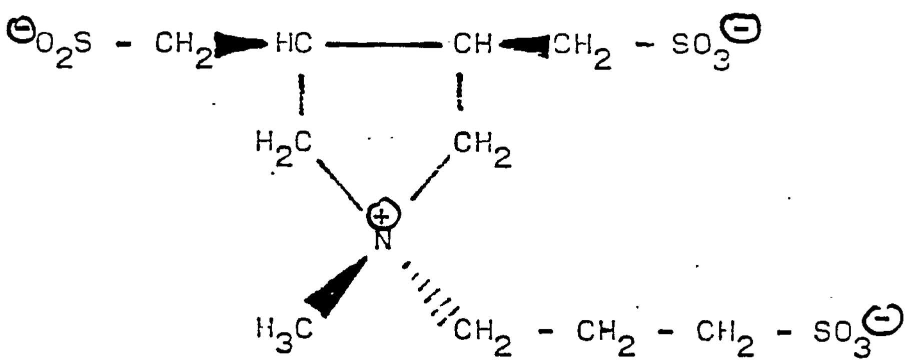

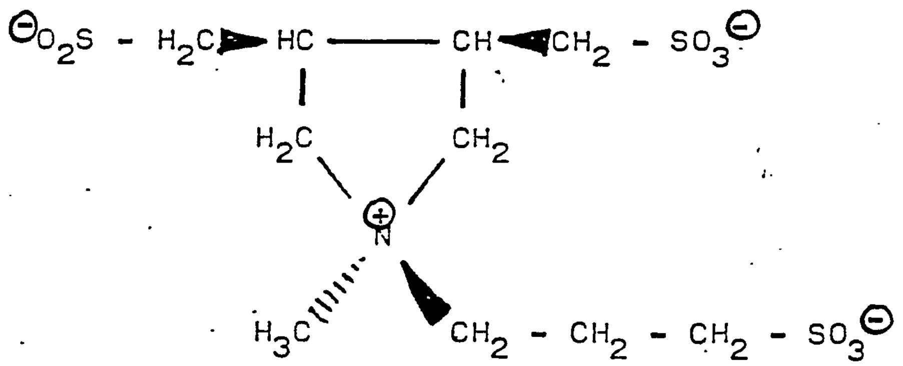

| | |
|---|---|
| N-$CH^2$ (Ring) | : 69,2; 69,5 |
| N-$CH^2$ | : 63,6; 66,5 |
| $CH_2$-$SO_2^-$ | : 57,1; 57,3 |
| N-$CH_3$ | : 50,8; 53,1 |
| $CH_2$-$SO_3$ (Ring) | : 51,1; 51,3 |
| $CH_2$-$SO_3$ | : 49,2 |
| <u>CH</u>-$CH_2$-$SO_3^-$ | : 36,0; 36,8 |
| <u>CH</u>-$CH_2$-$SO_2$ | : 33,0; 33,9 |
| $CH_2$ | :20,5; 21,1 |

Ein durch Sulfocylosulfinierung von Methyldiallyl-3-sulfonatopropyl-ammoniumbetain (vgl. DD-A-200 739) bei pH 2,5 mit der zweifachen molaren Menge Hydrogensulfit und 4 mol-% Peroxodisulfat nach obiger Verfahrensweise erhaltenes Produkt zeigte ein identisches $^{13}C$-NMR-Spektrum.

**Beispiel 63**

Es wurde nach Beispiel 62 verfahren, wobei 0,2 mol Methyltriallylammoniumchlorid, 0,6 mol Natriumhydrogensulfit einer entsprechenden Lösung und 14 g 33 %-ige Natronlauge zu einer homogenen Mischung vom pH-Wert 5,4 vermischt wurden, wonach 4 mol-% Kaliumperoxodisulfat auf einmal hinzugefügt wurden. Als Reaktionsprodukt wurde das Sulfobetain-sulfinat-sulfonat zu etwa 2/3 und das Sulfobetain-sulfonat zu etwa 1/3 erhalten.

**Beisple 64**

Es wurde nach Beispiel 62 verfahren, wobei 0,2 mol Methyltriallylammoniumchloridlösung, 0,7 mol Natriumhydrogensulfitlösung und 2,8 g 37 %-ige Salzsäure zu einer homogenen Lösung vermischt wurden, die mit 4 mol-% Ammoniumperoxodisulfat auf einmal versetzt wurde. Als Reaktionsprodukt wurde das Sulfobetain-sulfinat-sulfonat sowie das Sulfobetain-disulfinat-sulfonat zu etwa gleichen Teilen neben einer geringen Menge von etwa 5 % des Sulfobetainsulfonats erhalten.

**Beispiel 65**

Dieses Beispiel demonstriert die Möglichkeit, daß zur Initiierung der Sulfocyclosulfinierung Peroxodisulfat auch mit andere Oxidationsmitteln, wie Luft, gemeinsam angewandt werden kann, wenn weniger als etwa 1 mol-% Peroxodisulfat zum Einsatz kommt. Gegenüber Beispiel 62 verlängert sich die Reaktionszeit bis zum vollständigen Umsatz beträchtlich.

In einem nach Beispiel 62 zusätzlich mit einem Gaseinleitungsrohr ausgerüsteten Sulfierkolben wurde auch die in diesem Beispiel beschriebene Startlösung vom pH-Wert 4,3 aus 0,2 mol Methyltriallylammoniumchloridlösung und 0,61 mol Natriumhydrogensulfit hergestellt und vorgelegt. Unter heftigem Rühren wurde nun ein schwacher Luftstrom derart durch die Lösung geleitet, daß darin ständig Luftbläschen fein verteilt waden; gleichzeitig wurden auf einmal 333 mg (0,7 mol-%) Natriumperoxodisulfat, welches vorher in 2 g Wasser gelöst wurde, hinzugefügt. Unmittelbar nach der Peroxodisulfatzugabe färbte sich die zunächst fahlgelbe Lösung hell-orange, hatte jedoch nach 3 Minuten wieder ihre ursprüngliche Färbung. Im Verlaufe von 16 Minuten nach dem Beginn der Umsetzung erwärmte sich die reagierende Mischung von 25 auf 50,5° C, dem Temperaturmaximum, um sich bei förtgesetztem Rühren allmählich wieder abzukühlen.

Den gesamten zeitlichen Verlauf der exothermen Sulfocyclosulfonierung mit dem Oxidationsmittelgemisch zeigt die folgende Übersicht:

| Zeit (min) | 0 | 1 | 1,5 | 2 | 5 | 11 | 16 | 20 | 80 | 140 |
|---|---|---|---|---|---|---|---|---|---|---|
| Temp. (° C) | 25 | 40 | 43 | 45 | 48 | 50 | 50,5 | 50 | 37 | 30 |

Zum Zeitpunkt des Temperaturmaximums war jedoch noch kein vollständiger Umsatz erreicht; erst ungefähr 4 Stunden später konnte $^1$H-NMR-spektroskopisch kein Triallylammoniumsalz mehr nachgewiesen werden. Der pH-Wert der Reaktionslösung war am Schluß der Umsetzung auf 5,2 gestiegen. Die Zusammensetzung des Produktgemisches entsprach der des Beispiels 62.

**Beispiel 66**

Dinatrium-1-tetradecyl-1-(3'-sulfonatopropyl)-3-sulfinatomethyl- 4-sulfonatomethyl-pyrrolidiniumbetain ($R^1$ = $CH_2$-$CH_2$-$CH_2$-$SO_3^-$, $R^2$ = $C_{14}H_{29}$, $R^3$ = $R^4$ = H in der allgemeinen Formel Ia) durch Sulfocyclosulfinie-rung von Tetradecyltriallylammoniumbromid.

Es wurde nach Beispiel 62 verfahren, wobei die homogene Lösung vom pH-Wert 4,5 aus 0,1 mol Tetradecyltriallylammoniumbromid und 0,3 mol Natriumhydrogensulfit mit 4 mol-% Kaliumperoxodisulfat gesetzt wurde. Die neutralisierte klare Reaktionslösung kann entweder so für weitere Umsetzungen verwendet oder im Bedarfsfalle zur Trockne eingeengt werden, um durch Extraktion mit Ethanol/Wasser (2 : 1) das Sulfonierungsprodukt von den anorganischen Salzen abzutrennen. Das aus Ethanol/Wasser umkristallisierte Extraktionsprodukt zersetzte sich bei einer Temperatur oberhalb 270° C.

**Beispiel 67**

Dinatrium-1-dodecyl/tetradecylaminocarbonylmethyl-1-(3'-sulfonatopropyl)-3-sulfinatomethyl-4-sulfonato-methyl-pyrrolidiniumbetain ($R^1$ = -$CH_2$-$CH_2$-$CH_2$-$SO_3^-$, $R^2$ = $CH_2$-CO-NH-$C_{12}H_{25}$/$C_{14}H_{29}$, $R^3$ = $R^4$ = H in der allgemeinen Formel Ia) durch Sulfocyclosulfinierung von Dodecyl/tetradecylaminocarbonylmethyl-triallylammoniumchlorid.

Es wurde nach Beispiel 65 verfahren, wobei Dodecyl/tetradecylaminocarbonylmethyl-triallylammoniumchlorid, das aus Triallylamin, Chloressigsäuremethylester und Dodecyl/tetradecylamin-Gemisch (Cocosamin, Komponentenverhältnis 1 : 1) hergestellt war, zum Sulfobetain-sulfinat-sulfonat-Gemisch umgesetzt wurde.

Das mit Ethanol/Wasser (2 : 1) von der eingedampften Reaktionslösung gewonnene Extraktionsprodukt, welches noch einmal aus Ethanol/Wasser umkristallisiert wurde, war einen Schmelzpunkt von 212° C (unter Zersetzung).

**Beispiel 68**

Dinatrium-1-methyl-1-(3'-sulfonatopropyl-3,4-disulfonatomethyl-pyrrolidiniumbetain ($R^1$ = $-CH_2-CH_2-CH_2-SO_3$, $R^2 = CH_3$, $R^3 = R^4$ = H in der allgemeinen Formel Ib), aus Methyltriallylammoniumchlorid.

In einem mit Rührer, Rückflußkühler, Tropftrichter und Thermometer ausgerüsteten Sulfierkolben wurden 95,2 g (0,2 mol) 39,43 %-ige wäßrige Methyltriallylammoniumchloridlösung, 160,1 (0,6 mol) 39 %-ige technische wäßrige Natriumhydrogensulfitlösung mit einem Eisengehalt von 9 mg/mol $NaHSO_3$ sowie 3 Tropfen 33 %-ige Natronlauge miteinander zu einer homogenen Lösung vermischt, deren pH-Wert 4,5 (Glaselektrode) betrug. Zur so vorbereiteten fahlgelben Startlösung fügte man eine 40 %-ige wäßrige Natriumperoxodisulfatlösung, die aus 47,62 g (0,2 mol) und 71,4 g Wasser bereitet war, zügig hinzu.

Nach 2,5 Minuten waren etwa 50 % der Peroxodisulfatlösung zudosiert die inzwischen hell-orange gefärbte Mischung hatte sich von 23 auf 101°C erwärmt und siedete. Man setzte die Zugabe des Oxidationsmittels so fort, daß die Reaktionswärme leicht durch Siedekühlung abgeführt werden konnte.

Im Verlaufe der weiteren Zudosierung der Peroxodisulfatlösung hellte sich die Reaktionsmischung zunehmend auf und war zum Schluß nach 3,5 Minuen praktisch farblos.

Die folgende Übersicht zeigt den zeitlichen Verlauf des exothermen Reaktion während der Zudosierungsphase des Oxidationsmittels:

| Zeit (min) | 0 | 1 | 2 | 2,5 | 3 | 3,5 |
|---|---|---|---|---|---|---|
| Temperatur (°C) | 23 | 80 | 93 | 101 | 101 | 100 |

Das $^1H$-NMR-Spektrum der Reaktionslösung wies die quantitative Sulfonierung des Methyltriallylammoniumchlorids aus.

Das Sulfobetaindisulfonat wurde neben dem Sulfobetaintrisulfonat und dem Sulfobetainsulfonat in etwa 50 bis 60 %-iger Ausbeute erhalten. Nach Neutralisation der stark sauren Reaktsonslösung mit 33 %-iger Natronlauge flockte das durch Verwendung von technischer Natriumhydrogensulfitlösung darin enthaltene Eisensalz als Eisen(III)-hydroxid aus und konnte zusammen mit dem größten Teil des auskristallisierten Natriumsulfats abfiltriert werden. Das $^{13}C$-NMR-Spektrum des farblosen Filtrats zeigte, daß die chemischen Verschiebungen (ppm) einzelner Kohlenstoffatome Verdopplungen aufwiesen, was das Vorliegen der folgenden Strukturisomeren beweist:

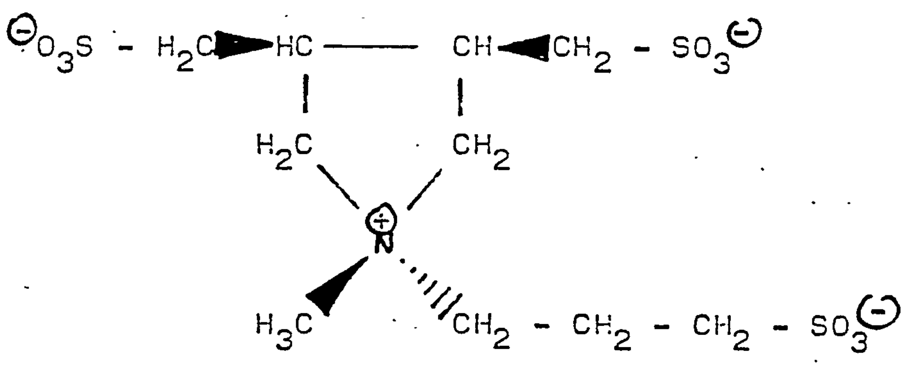

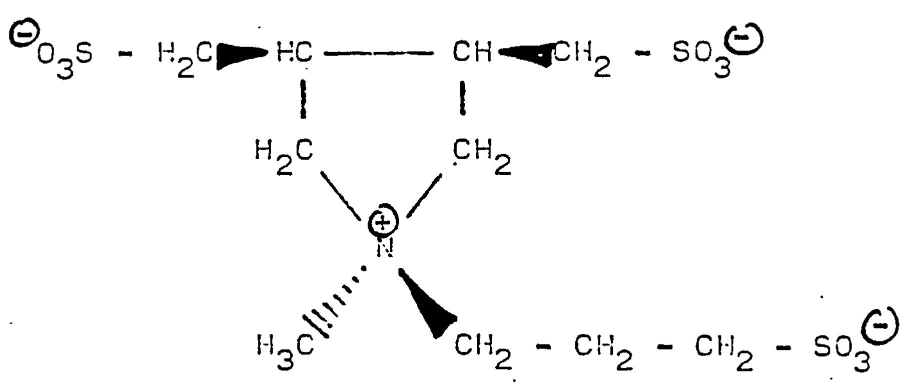

| | |
|---|---|
| N-$CH_2$ (Ring) | : 69,0; 69,5 |
| N-$CH_2$ | : 66,5; 63,6 |
| N-$CH_3$ | : 53,2 |
| $CH_2SO_3^-$ (Ring) | : 51,0 |
| $CH_2$-$SO_3^-$ | : 49,2 |
| CH | : 36;6; 35,8 |
| $CH_2$ | : 21,2; 20,7 |

Ein durch Sulfonierung von Methyl-diallyl-3-sulfonatopropyl-ammoniumbetain (vgl. DD-A-200 739) bei einem pH-Wert von 2,5 mit der zweifachenmolaren Menge Hydrogensulfit und der äquimolaren Menge Peroxodisulfat nach obiger Verfahrensweise erhaltenes Produkt zeigte ein identisches $^{13}C$-NMR-Spektrum.

**Beispiel 69**

Es wurde nach Beispiel 68 verfahren, wobei 0,2 mol Methyltriallylammoniumchloridlösung, 0,6 mol Natriumhydrogensulfit (als Lösung) und 14 g 33 %-ige Natronlauge zu einer homogenen Lösung vom pH-Wert 5,5 vermischt wurden; man fugte dann 0,2 mol 40 %-ige Natriumperoxodisulfatlösung allmählich hinzu. Als Reaktionsprodukt wurde das Sulfobetaindisulfonat zu etwa 2/3 und das Sulfobetainsulfonat zu etwa 1/3 erhalten.

**Beispiel 70**

Es wurde nach Beispiel 68 verfahren, wobei 0,2 mol Methyltriallylammoniumchloridlösung, 0,7 mol Natriumhydrogensulfit (als Lösung) und 2,8 g 37 %-ige Salzsäure zu einer homogenen Lösung vom pH-Wert 3,0 vermischt wurden, der dann 0,3 mol 40 %-ige Natriumperoxodisulfatlösung allmählich gefügt wurden. Als Reaktionsprodukt wurde das Sulfobetaindisulfonat sowie das Sulfobetaintrisulfonat zu etwa gleichen Teilen neben einer geringen Menge von etwa 5 % des Sulfobetainsulfonats erhalten.

**Beispiel 71**

Dieses Beispiel veranschaulicht die Eignung einer Oxidationsmittelkombination.

Es wurde nach Beispiel 68 verfahren, wobei eine homogene Startlösung vom pH-Wert 4,5 aus Methyltriallylammoniumchlorid, Natriumhydrogensulfit und Natronlauge hergestellt wurde. Zunächst fügte man in etwa 1,5 Minuten 15 mol-% einer 50 %-igen Ammoniumperoxodisulfatlösung und anschließend 85 mol-% 30 %-iges Wasserstoffperoxid in einer solchen Geschwindigkeit hinzu, daß die exotherme Reaktion durch Siedekühlung beherrscht werden konnte, wofür nochmals etwa 2 Minuten benötigt wurden. Die Produktzusammensetzung war die gleiche wie in Beispiel 68.

Will man die Sulfonierungsprodukte vollständig von seinen anorganischen Begleitsalzen abtrennen, kann man wie folgt verfahren:

Nach Einengen der oben gewonnenen Reaktionslösung zur Trockne versetzt man den erhaltenen Salzrückstand mit der ausreichenden Menge konzentrierter Salzsäure und arbeitet ihn durch, filtriert von den ungelösten Natriumsalzen ab und dampft die salzsaure Lösung der Sulfobetaindisulfonsäure unter vermindertem Druck ein. Durch Zusatz von Ethanol zum verbleibenden Rückstand kann die Sulfonsäure ausgefällt werden.

**Beispiel 72**

Dinatrium-1-tetradecyl-1-(3'-sulfonatopropyl)-3,4-disulfonatomethyl-pyrrolidiniumbetain ($R^1$ = -$CH_2$-$CH_2$-$CH_2$-$SO_3^-$, $R^2$ = $C_{14}H_{29}$, $R^3$ = $R^4$ = H in der allgemeinen Formel Ib) aus Tetradecyltriallylammoniumbromid.

Es wurde nach einem der vorstehend beschriebenen Beispiele verfahren, wobei die vereinigten Lösungen vom pH-Wert 4,5 aus 0,1 mol Tetradecyltriallylammoniumbromid und 0,3 mol Natriumhydrogensulfit mit Natriumperoxodisulfat umgesetzt wurden.

Die neutralisierte klare Reaktionslösung kann im Bedarfsfalle zur Trockne eingeengt werden, um durch Extraktion mit Ethanol/Wasser (2 : 1) das Sulfonierungsprodukt von den anorganischen Salzen abzutrennen. Das aus Ethanol/Wasser umkristallisierte Produkt zersetzte sich bei einer Temperatur oberhalb 284° C.

**Beispiel 73**

Dinatrium-1-dodecyl/tetradecylaminocarbonylmethyl-1-(3'-sulfonatopropyl)-3,4-disulfonatomethyl-pyrrolidiniumbetain ($R^1$ = $-CH_2-CH_2-CH_2-SO_3^-$, $R^2$ = $CH_2-CO-NH-C_{12}H_{25}/C_{14}H_{29}$, $R^3$ = $R^4$ = H in der allgemeinen Formel Ib).

Es wurde nach Beispiel 71 verfahren, wobei Dodecyl/tetradecylaminocarbonylmethyltriallylammoniumchlorid, das aus Triallylamin, Chloressigsäuremethylester und Dodecyl/tetradecylamingemisch (Cocosamin, Komponentenverhältnis etwa 1 : 1) hergestellt war, zum Sulfobetaindisulfonatgemisch umgesetzt wurde. Nach Neutralisation mit 33 %-iger Natronlauge und Einengung bis zur Trockne wurde das Sulfobetaindisulfonatgemisch mit Ethanol/Wasser (Gewichtsverhältnis 2 : 1) extrahiert und aus Ethanol/Wasser umkristallisiert. Das Reaktionsprodukt zersetzte sich bei 234° C.

**Patentansprüche**

1. 3-Sulfinatomethyl- und 3-Sulfonatomethyl-4-sulfonatomethyl-pyrrolidiniumbetaine der Formeln Ia und Ib,

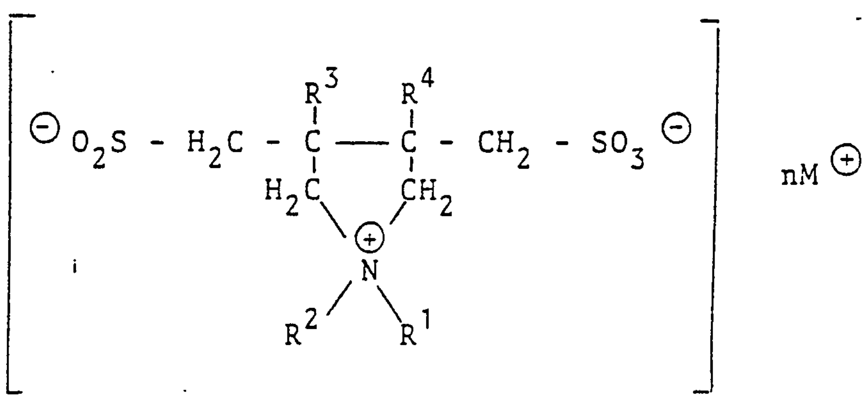

(Ia),

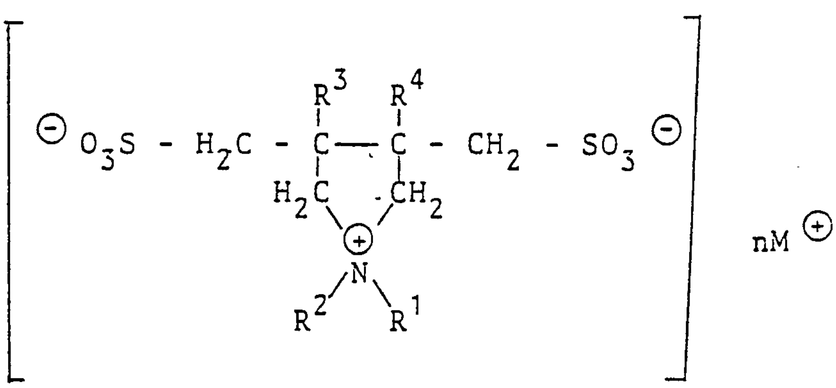

(Ib),

worin bedeuten:

$R^1$ Wasserstoff, geradkettiges oder verzweigtes $C_{1-22}$-Alkyl, wobei in der Kette -NH-CO- oder -CO-NH- enthalten sein können,
Benzyl,
$-CH_2-CH_2-CH_2-SO_3^{\ominus}$,
$-CH_2-CH-CH_2-SO_3^{\ominus}$ oder
|
$SO_2^{\ominus}$
$-CH_2-CH-CH_2-SO_3^{\ominus}$ darstellen,
|
$SO_3^{\ominus}$

$R^2$ unabhängig von $R^1$ Wasserstoff, geradkettiges oder verzweigtes $C_{1-22}$-Alkyl, wobei in der Kette -NH-CO- oder -CO-NH- enthalten sein können, oder gemeinsam mit $R^1$ eine am $N^{\oplus}$-Atom des Pyrrolidiniumrings gebundene Tetramethylengruppe, die in den Positionen 2 und 3 durch eine Sulfinatomethyl- und eine Sulfonatomethylgruppe substituiert ist,

$R^3$, $R^4$ Wasserstoff oder Methyl,
$M^{\ominus}$ gleiche oder verschiedene Kationen aus der Gruppe $H^{\oplus}$, $Na^{\oplus}$, $K^{\oplus}$ und $NH_4^{\oplus}$ und
n 1, 2 oder 3.

2. Verfahren zur Herstellung der 3-Sulfinatomethyl- oder 3-Sulfonatomethyl-4-sulfonatomethyl-pyrrolidinium-betaine der Formeln Ia und Ib nach Anspruch 1, gekennzeichnet durch Umsetzung von
- Diallylammoniumsalzen der Formel II,

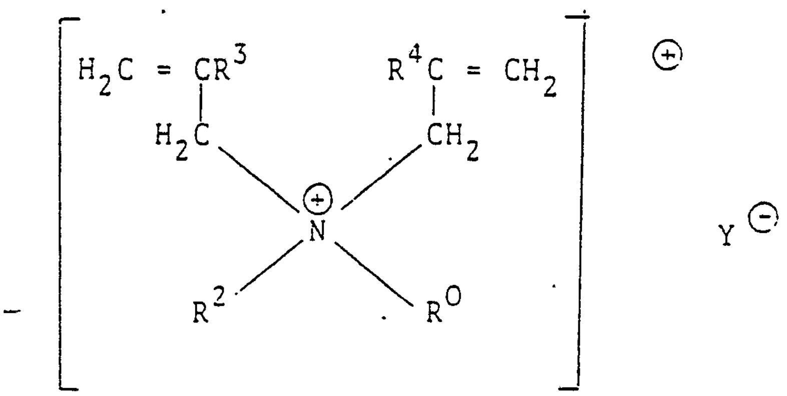

(II),

worin darstellen:
$R^2$, $R^3$ und $R^4$ dasselbe wie in Anspruch 1,
$R^0$ unabhängig von $R^2$ dieselben Bedeutungen wie $R^2$ oder gemeinsam mit $R^2$ eine am $N^{\oplus}$-Atom des Pyrrolidiniumrings gebundene Tetramethylengruppe, die in den Positionen 2 und 3 durch eine Sulfinatomethyl- und eine Sulfonatomethylgruppe substituiert ist,
und
$Y^{\ominus}$ Chlorid, Bromid, Methosulfat oder Sulfat,

- Triallylammoniumsalzen der Formel III,

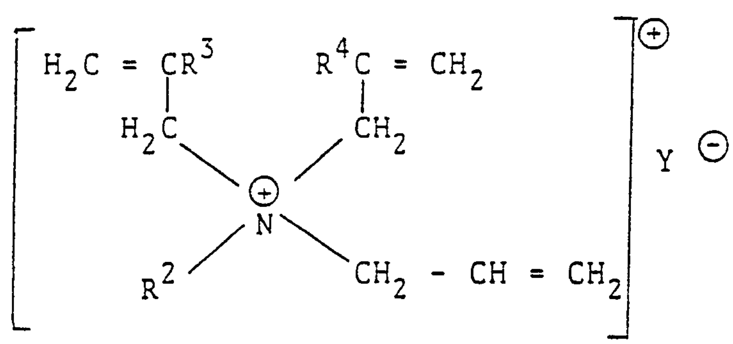

(III)

mit $R^2$, $R^3$, $R^4$ und $Y^{\ominus}$ wie oben
oder
- entsprechenden Tetraallylammoniumsalzen
mit äquimolaren Mengen eines Hydrogensulfits in Anwesenheit von Peroxodisulfaten allein oder im Gemisch mit unter Chlor, chlorabgebenden Stoffen, Chlorat, Bromat, Wasserstoffperoxid und Luft ausgewählten weiteren Oxidationsmitteln im pH-Bereich von 1,5 bis 6.

3. Verfahren nach Anspruch 2 zur Herstellung der 3-Sulfinatomethyl-4-sulfonatomethyl-pyrrolidinium-betaine der Formel Ia nach Anspruch 1,
gekennzeichnet durch
a) Umsetzung der Diallylammoniumsalze der Formel II mit der zweifachen molaren Menge des Hydrogensulfits in Gegenwart einer katalytischen Menge des Peroxodisulfats bei pH-Werten von 2 bis 4;
b) wenn $R^1$ gleich $-CH_2-CH_2-CH_2-SO_3^{\ominus}$ ist:
Umsetzung der Triallylammoniumsalze der Formel III mit der dreifachen molaren Menge des Hydrogensulfits in Gegenwart einer katalytischen Menge des Peroxodisulfats allein oder in Verbindung mit einer gleichzeitigen oder anschließenden Einwirkung von Luftsauerstoff bei pH-Werten von 2,5 bis 6 und vorzugsweise von 4,0 bis 5,5;
c) wenn $R^1$ gleich
$-CH_2-CH-CH_2-SO_3^{\ominus}$ ist:

|
$SO_2^{\ominus}$

Umsetzung der Triallylammoniumsalze der Formel III mit der mindestens vierfachen molaren Menge des Hydrogensulfits in Gegenwart einer katalytischen Menge des Peroxodisulfats bei pH-Werten von 1,5 bis 2,5.

4. Verfahren nach Anspruch 2 zur Herstellung der 3-Sulfonatomethyl-4-sulfonatomethyl-pyrrolidiniumbetaine der Formel Ib nach Anspruch 1,
gekennzeichnet durch

a) Umsetzung der Diallylammoniumsalze der Formel II pro Mol mit der zweifachen molaren Menge des Hydrogensulfits und der äquimolaren Menge Peroxodisulfat oder bei gleichzeitiger Anwesenheit unter Chlor, chlorabgebenden Stoffen Chlorat, Bromat, Wasserstoffperoxid und Luft ausgewählten weiteren Oxidationsmittel mit insgesamt zwei Oxidationsäquivalenten bei pH-Werten von 2 bis 4;

b) wenn $R^1$ gleich $-CH_2-CH_2-CH_2-SO_3^{\ominus}$ ist:
Umsetzung der Triallylammoniumsalze der Formel III pro Mol mit der dreifachen molaren Menge des Hydrogensulfits und der äquimolaren Menge Peroxodisulfat oder bei gleichzeitiger Anwesenheit unter Chlor, chlorabgebenden Stoffen, Chlorat, Bromat, Wasserstoffperoxid und Luft ausgewählten weiteren Oxidationsmittel mit insgesamt zwei Oxidationsäquivalenten bei pH-Werten von 2,5 bis 6 und vorzugsweise von 4,0 bis 5,5;

c) wenn $R^1$ gleich
$-CH_2-CH-CH_2-SO_3^{\ominus}$ ist:
|
$SO_3^{\ominus}$
Umsetzung der Triallylammoniumsalze der Formel III pro Mol mit der vierfachen molaren Menge des Hydrogensulfits und der zweifachen molaren Menge des Peroxodisulfats oder bei gleichzeitiger Anwesenheit unter Chlor, chlorabgebenden Stoffen, Chlorat, Bromat, Wasserstoffperoxid und Luft ausgewählten weiteren Oxidationsmittel mit insgesamt vier Oxidationsäquivalenten bei pH-Werten von 1,5 bis 2,5.

5. Tensidzusammensetzungen, dadurch gekennzeichnet, daß sie mindestens ein 3-Sulfinatomethyl- oder 3-Sulfonatomethyl-4-sulfonatomethyl-pyrrolidiniumbetain der Formeln Ia oder Ib nach Anspruch 1 enthalten.

## Claims

1. 3-Sulphinatomethyl-4-sulphonatomethyl-pyrrolidinium betaines and 3-sulphonatomethyl-4-sulphonatomethyl-pyrrolidinium betaines of the formulae Ia and Ib,

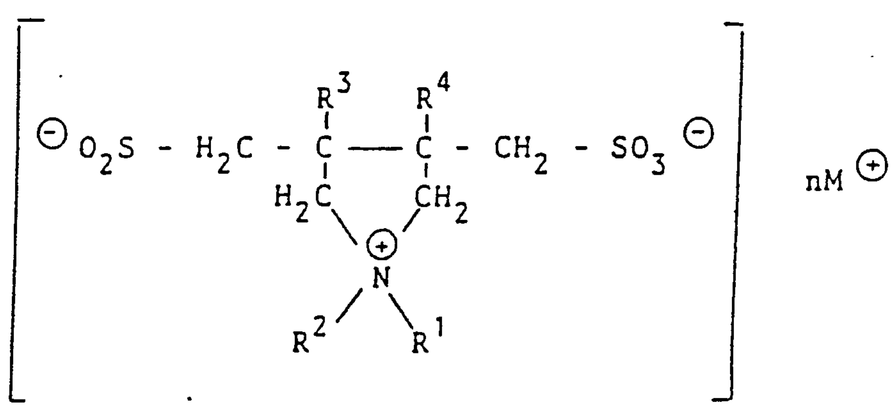

(Ia),

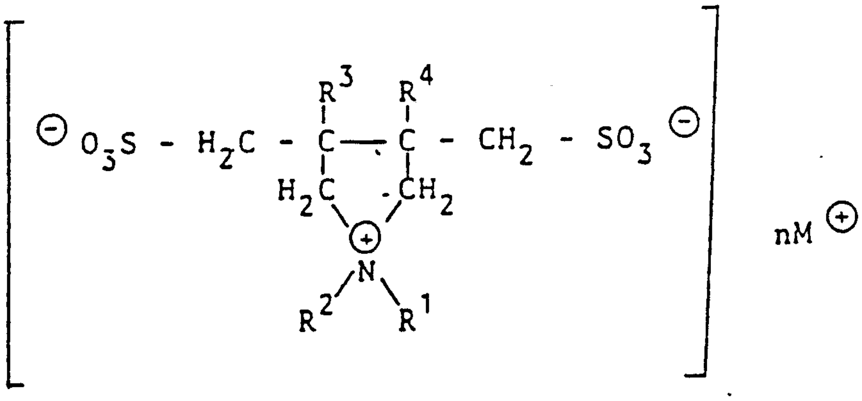

(Ib),

wherein:

$R^1$ denotes hydrogen, straight-chain or branched $C_{1-22}$-alkyl, it being possible for -NH-CO- or -CO-NH- to be contained in the chain,
benzyl,
$-CH_2-CH_2-CH_2-SO_3^{\ominus}$,
$-CH_2-CH-CH_2-SO_3^{\ominus}$ or
|
$SO_2^{\ominus}$
$-CH_2-CH-CH_2-SO_3^{\ominus}$,
|
$SO_3^{\ominus}$

$R^2$ independently of $R^1$ denotes hydrogen, straight-chain or branched $C_{1-22}$-alkyl, it being possible for -NH-$CO_1$ or -CO-NH- to be contained in the chain, or together with $R^1$ denotes a tetramethylene group substituted in the 2 and 3 positions by a sulphinatomethyl group and a sulphonatomethyl group and which is bound to the $N^{\oplus}$ atom of the pyrrolidinium ring,

$R^3$, $R^4$ denote hydrogen or methyl,

$M^{\oplus}$ denotes the same or different cations from the group $H^{\oplus}$, $Na^{\oplus}$, $K^{\oplus}$ and $NH_4^{\oplus}$
and

n denotes 1, 2 or 3.

2. Process for the preparation of 3-sulphinatomethyl-4-sulphonatomethyl-pyrrolidinium betaines or 3-sulphonatomethyl-4-sulphonatomethyl-pyrrolidinium betaines of the formulae Ia and Ib according to claim 1, characterised by the reaction of

- diallylammonium salts of the formula II,

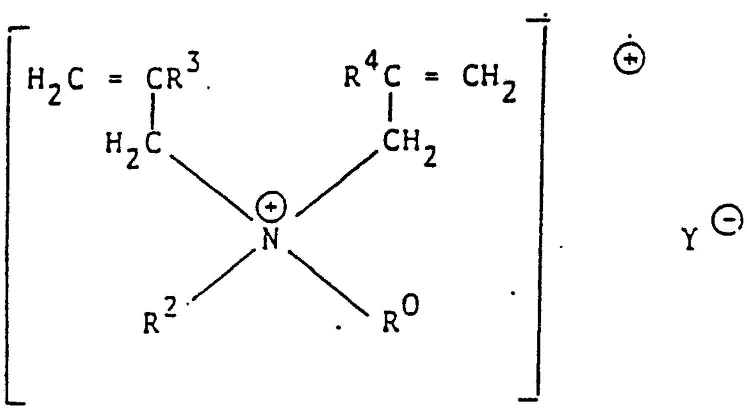

(II),

wherein

$R^2$, $R^3$ and $R^4$ are the same as in claim 1,

$R^0$ independently of $R^2$ has the same meanings as $R^2$, or together with $R^2$ is a tetramethylene group substituted in the 2 and 3 positions by a sulphinatomethyl group and a sulphonatomethyl group and which is bound to the $N^{\oplus}$ atom of the pyrrolidinium ring,
and

$Y^{\ominus}$ is chloride, bromide, methosulphate or sulphate.

- triallylammonium salts of the formula III,

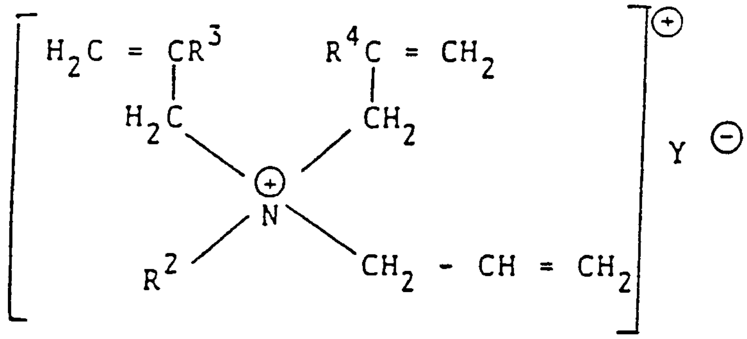

(III)

having $R^2$, $R^3$, $R^4$ and Y⊕ as above
or
- corresponding tetraallylammonium salts
with equimolar amounts of a hydrogen sulphite in the presence of peroxodisulphates on their own or mixed with further oxidation agents selected from chlorine, chlorine-releasing substances, chlorate, bromate, hydrogen peroxide and air, in the pH range from 1.5 to 6.

3. Process according to claim 2 for the preparation of 3-sulphinatomethyl-4-sulphonatomethyl-pyrrolidinium betaines of the formula Ia according to claim 1,
characterised by

a) reaction of diallylammonium salts of the formula II with twice the molar amount of hydrogen sulphite in the presence of a catalytic amount of peroxodisulphate at pH values of 2 to 4;

b) if $R^1$ is $-CH_2-CH_2-CH_2-SO_3^{\ominus}$:
reaction of triallylammonium salts of the formula III with three times the molar amount of hydrogen sulphite in the presence of a catalytic amount of peroxodisulphate on its own or in conjunction with a simultaneous or subsequent action of air oxygen at pH values of 2.5 to 6 and preferably of 4.0 to 5.5;

c) if $R^1$ is
$-CH_2-CH-CH_2-SO_3^{\ominus}$:
|
$SO_2^{\ominus}$
reaction of triallylammonium salts of the formula III with at least four times the molar amount of hydrogen sulphite in the presence of a catalytic amount of peroxodisulphate at pH values of 1.5 to 2.5.

4. Process according to claim 2 for the preparation of 3-sulphonatomethyl-4-sulphonatomethyl-pyrrolidinium betaines of the formula Ib according to claim 1,
characterised by

a) reaction of diallylammonium salts of the formula II per mole with twice the molar amount of hydrogen sulphite and the equimolar amount of peroxodisulphate, or when further oxidation agents selected from chlorine, chlorine-releasing substances, chlorate, bromate, hydrogen peroxide and air are also present, with a total of two oxidation equivalents at pH values of 2 to 4;

b) if $R^1$ is $-CH_2-CH_2-CH_2-SO_3^{\ominus}$:
reaction of triallylammonium salts of the formula III per mole with three times the molar amount of hydrogen sulphite and the equimolar amount of peroxodisulphate, or when further oxidation agents selected from chlorine, chlorine-releasing substances, chlorate, bromate, hydrogen peroxide and air are also present, with a total of two oxidation equivalents at pH values of 2.5 to 6 and preferably of 4.0 to 5.5;

c) if $R^1$ is
$-CH_2-CH-CH_2-SO_3^{\ominus}$:
|
$SO_3^{\ominus}$
reaction of triallylammonium salts of the formula III per mole with four times the molar amount of hydrogen sulphite and twice the molar amount of peroxodisulphate, or when further oxidation agents selected from chlorine, chlorine-releasing substances, chlorate, bromate, hydrogen peroxide and air are also present, with a total of four oxidation equivalents at pH values of 1.5 to 2.5.

5. Surfactant compositions characterised in that they contain at least one 3-sulphinatomethyl-4-sulphonatomethyl-pyrrolidinium betaine or 3-sulphonatomethyl-4-sulphonatomethyl-pyrrolidinium betaine of the formulae Ia or Ib according to claim 1.

## Revendications

1. 3-sulfinatométhyl- et 3-sulfonatométhyl-4-sulfonatométhyl-pyrrolidiniumbetaïnes de formules Ia et Ib,

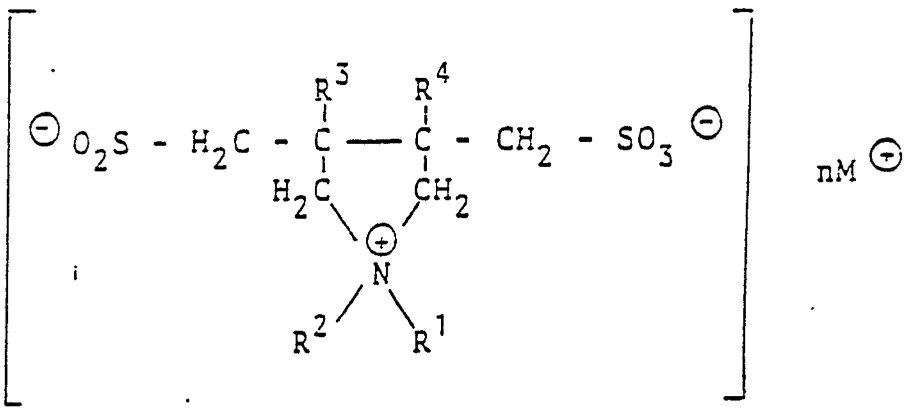

(Ia),

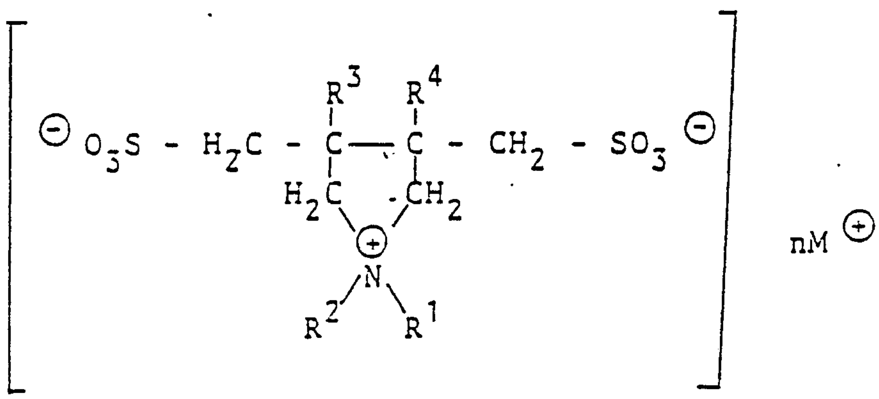

(Ib),

dans lesquelles

$R^1$ représente un atome d'hydrogène, un groupe alkyle en $C_{1-22}$ à chaine linéaire ou ramifiée, la chaîne pouvant contenir -NH-CO- ou -CO-NH-,
un groupe benzyle, $-CH_2-CH_2-CH_2-SO_3^{\ominus}$,
$-CH_2-CH-CH_2-SO_3^{\ominus}$ ou
      |
      $SO_2^{\ominus}$
$-CH_2-CH-CH_2-SO_3^{\ominus}$
      |
      $SO_3^{\ominus}$

$R^2$ représente independamment de $R^1$ un atome d'hydrogène, un groupe alkyle en $C_{1-22}$ à chaîne linéaire ou ramifiée, la chaîne pouvant contenir -NH-CO- ou -CO-NH-, ou ensemble avec $R^1$ un groupe tétraméthylène lié à l'atome $N^{\oplus}$ du cycle pyrrolidinium, substitué en positions 2 et 3 par un groupe sulfinatométhyle et un groupe sulfonatométhyle,

$R^3$, $R^4$ représentent un atome d'hydrogène ou un groupe méthyle,

$M^{\oplus}$ représente des cations identiques ou différents, du groupe $H^{\oplus}$, $Na^{\oplus}$, $K^{\oplus}$, $NH_4^{\oplus}$
et

n signifie 1, 2 ou 3.

2. Procédé pour la fabrication des 3-sulfinatométhyl-ou 3-sulfonatométhyl-4-sulfonatométhyl-pyrrolidinium-bétaïnes de formules Ia et Ib selon la revendication 1,
caractérisé en ce que l'on fait réagir
- des sels de diallylammonium de formule II,

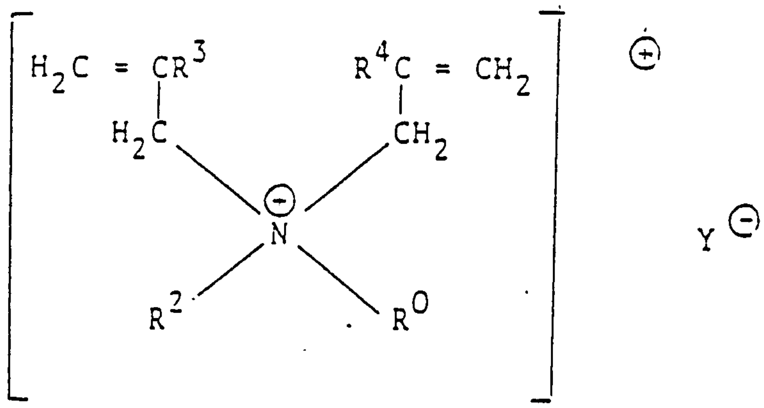

(II),

dans laquelle

$R^2$, $R^3$ et $R^4$ ont les significations décrites dans la revendication 1,

$R^0$ possède indépendamment de $R^2$ les mêmes significations que $R^2$ ou représente ensemble avec $R^2$ un groupe tétraméthylène lié à l'atome $N^{\oplus}$ du cycle pyrrolidinium, substitué en positions 2 et 3 par un groupe sulfinatométhyle et un groupe sulfonatométhyle,

et

$Y^-$ signifie chlorure, bromure, méthosulfate ou sulfate,

- des sels de triallylammonium de formule III,

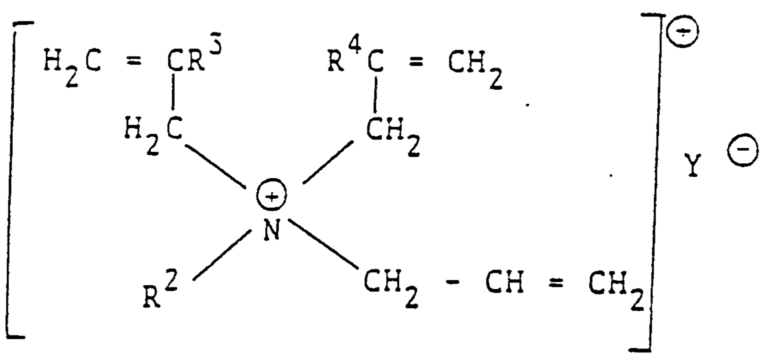

(III)

$R^2$, $R^3$, $R^4$ et $Y^{\ominus}$ ayant les mêmes significations que ci-dessus

ou

- des sels correspondants de térra-allylammonium avec des quantités équimolaires d'un hydrogénosulfite en présence de peroxodisulfates seuls ou en mélange avec d'autres oxydants choisis parmi le chlore, les substances dégageant du chlore, les chlorates, les bromates, le peroxyde d'hydrogène et l'air, dans une plage de pH de 1,5 à 6.

3. Procédé selon la revendication 2 pour la fabrication des 3-sulfinatométhyl-4-sulfonatométhyl-pyrrolidinium-bétaïnes de formule Ia selon la revendication 1,

caractérisé en ce que

a) on fait réagir des sels de diallylammonium de formule II avec des quantités bimolaires d'hydrogénosulfite en présence d'une quantité catalytique de peroxodisulfate dans des conditions de pH de 2 à 4;

b) lorsque $R^1$ est égal à $-CH_2-CH_2-CH_2-SO_3^{\ominus}$, on fait réagir des sels de triallylammonium de formule III avec des quantités trimolaires d'hydrogénosulfite en présence d'une quantité catalytique de peroxodisulfate seul ou en association avec une action simultanée ou immédiatement ultérieure de l'oxygène de l'air, dans des conditions de pH de 2,5 à 6 et de préférence de 4,0 à 5;

c) lorsque R est égal à

$-CH_2-CH-CH_2-SO_3^{\ominus}$,

$\quad\quad\quad|$

$\quad\quad SO_2^{\ominus}$

on fait réagir les sels de triallylamonium de formule III avec des quantités au moins quadrimolaires d'hydrogénosulfite en présence d'une quantité catalytique de peroxodisulfate dans des conditions de pH de 1,5 à 2,5.

4. Procédé selon la revendication 2 pour la fabrication des 3-sulfonatométhyl-4-sulfonatométhyl-pyrrolidiniumbétaïnes de formule Ib selon la revendication 1,

caractérisé en ce que

a) on fait réagir des sels de diallylammonium de formule II avec par mole une quantité bimolaire d'hydrogénosulfite et une quantité équimolaire de peroxodisulfate ou en présence simultanée d'autres oxydants choisis parmi le chlore, les substances dégageant du chlore, les chlorates, les bromates, le peroxyde d'hydrogène et l'air avec au total deux équivalents d'oxydation dans des conditions de pH de 2 à 4;

b) lorsque $R^1$ est égal à $-CH_2-CH_2-CH_2-SO_3^{\ominus}$, on fait réagir par mole des sels de triallylammonium de formule III avec des quantités trimolaires d'hydrogénosulfite et la quantité équimolaire de peroxodisulfate ou en présence simultanée d'autres oxydants choisis parmi le chlore, les substances dégageant du chlore, les chlorates, les bromates, le peroxyde d'hydrogène et l'air avec au total deux équivalents d'oxydation dans des conditions de pH de 2,5 à 6 et de préférence de 4,0 à 5,5;

c) lorsque R est égal à

$-CH_2-CH-CH_2-SO_3^{\ominus}$,

        |

        $SO_3^{\ominus}$

on fait réagir les sels de triallylamonium de formule III par mole avec des quantités quadrimolaires d'hydrogénosulfite et une quantité bimolaire de peroxodisulfate ou en présence simultanée d'autres oxydants choisis parmi le chlore, les substances dégageant du chlore, les chlorates, les bromates, le peroxyde d'hydrogène et l'air avec au total quatre équivalents d'oxydation dans des conditions de pH de 1,5 à 2,5;

5. Compositions tensio-actives, caractérisées en ce qu'elles contiennent au moins une 3-sulfinatométhyl- ou une 3-sulfonatométhyl-4-sulfonatométhyl-pyrrolidiumbétaïne de formule Ia ou Ib selon la revendication 1.

Fig.1

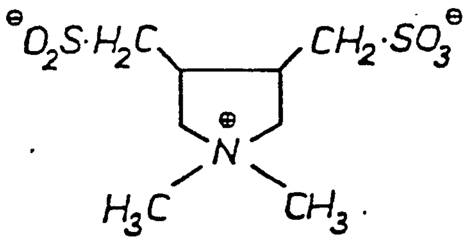

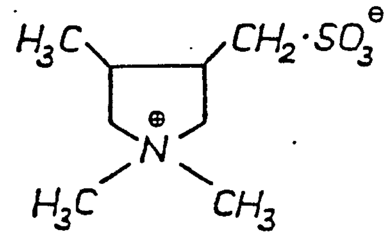

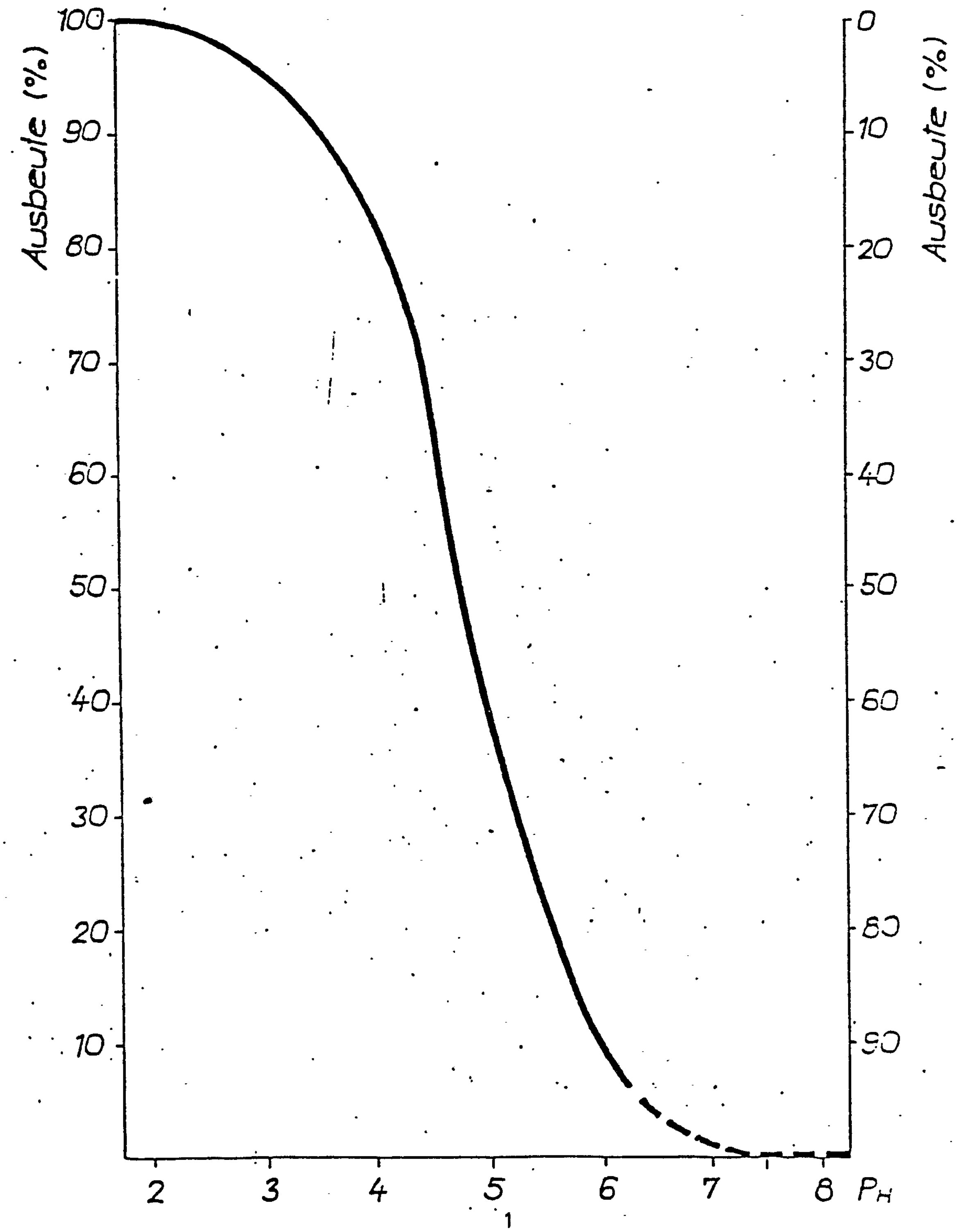
Ausbeute (%)
100
90
80
70
60
50
40
30
20
10
Ausbeute (%)
0
10
20
30
40
50
60
70
80
90
2
3
4
5
6
7
8
PH

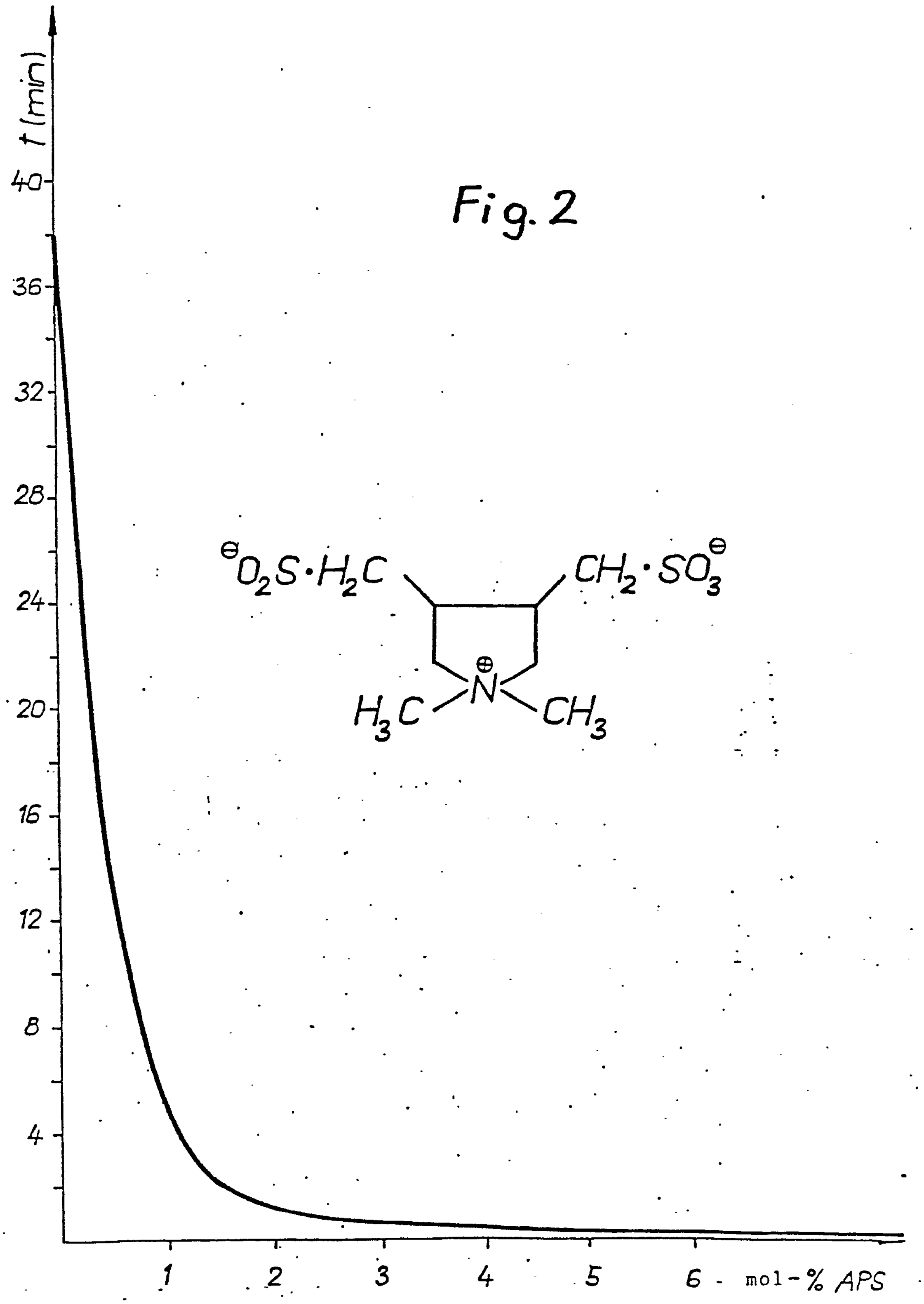


Fig. 2

Fig. 3